# EUROPEAN PATENT SPECIFICATION

(11) **EP 3 941 468 B1**
(45) Date of publication and mention of the grant of the patent: **14.10.2026**
(21) Application number: 20728536.2
(22) Date of filing: 20.03.2020
(51) Int. Cl.: A61K 31/445, A61K 31/40, A61K 31/397, A61P 3/10, A61P 1/16, C07D 207/08, C07D 211/22

(54) **HETEROCYCLYL(PHENYL)METHANOL COMPOUNDS USEFUL IN THE TREATMENT OF HYPERGLYCAEMIA**
HETEROCYCLYL(PHENYL)METHANOLVERBINDUNGEN ZUR BEHANDLUNG VON HYPERGLYKÄMIE
COMPOSÉS HÉTÉROCYCLYL(PHÉNYL) MÉTHANOL UTILES DANS LE TRAITEMENT DE L'HYPERGLYCÉMIE

(30) Priority: 20.03.2019 GB 201903827
(43) Date of publication of application: 26.01.2022
(60) Divisional application: 26202983.8
(73) Proprietor: Atrogi AB, 171 65 Solna (SE)
(72) Inventor: PELCMAN, Benjamin, 113 52 Stockholm (SE); BENGTSSON, Tore, 185 94 Vaxholm (SE)
(74) Representative: Potter Clarkson
(86) International application number: PCT/GB2020/050762
(87) International publication number: WO 2020/188301

(56) References cited:
- WO-A1-2019/053425
- WO-A1-99/65308
- DE-A1- 2 548 053
- US-A- 3 985 887
- ISLER D ET AL: "ANTI-DIABETIC ACTIVITY OF RO-16-8714 A BETA-ADRENERGIC AGONIST IN OBESE HYPERGLYCEMIC OB-OB MICE AND STREPTOZOTOCIN-DIABETIC RATS", DIABETOLOGIA, vol. 27, no. 2, 1984, & 20TH ANNUAL MEETING OF THE EUROPEAN ASSOCIATION FOR THE STUDY OF DIABETES, LONDON, ENGLAND, SEPT. 12, pages 291A, XP093100656, ISSN: 0012-186X
- CHEN-XI YE ET AL: "Dual catalysis for enantioselective convergent synthesis of enantiopure vicinal amino alcohols", NATURE COMMUNICATIONS NATURE PUBLISHING GROUP UK, vol. 9, no. 1, December 2018 (2018-12-01), XP002800110, ISSN: 2041-1723

## Description

### Field of the Invention

The present invention is defined in the appended claims. It relates to novel compounds and compositions, and their use in the treatment of hyperglycaemia and disorders characterised by hyperglycaemia, such as type 2 diabetes. In particular, the invention relates to novel compounds, compositions and methods for the treatment of conditions such as type 2 diabetes through activation of the β₂-adrenergic receptor. Importantly, such compounds are thought to have a beneficial side-effect profile as they do not exert their effect through significant cAMP release.

### Background of the Invention

The listing or discussion of an apparently prior-published document in this specification should not necessarily be taken as an acknowledgement that the document is part of the state of the art or is common general knowledge.

Hyperglycaemia, or high blood sugar, is a condition in which an excessive amount of glucose circulates in the blood plasma. If not treated, hyperglycaemia can be a serious problem, potentially developing into life-threatening conditions such as ketoacidosis. For example, chronic hyperglycemia may cause injury to the heart, and is strongly associated with heart attacks and death in subjects with no coronary heart disease or history of heart failure. There are various causes of hyperglycaemia, including diabetes and severe insulin resistance.

Severe insulin resistance (SIR) is a condition wherein the patient experiences very low levels of (or, in extreme cases, no significant) response to insulin. There are several syndromes characterized by SIR, including Rabson-Mendenhall syndrome, Donohue's syndrome (leprechaunism), Type A and Type B syndromes of insulin resistance, the HAIR-AN (hyperandrogenism, insulin resistance, and acanthosis nigricans) syndrome, pseudoacromegaly, and lipodystrophy. The majority of these conditions have genetic causes, such as mutations in the insulin receptor gene. The prevalence for Donohue's syndrome, Rabson-Mendenhall syndrome and Type A syndrome of insulin resistance, has been reported to vary from about 50 in total reported cases to a prevalence of 1 in 100,000 people. However, since some diseases are severe and extremely rare, it is likely that many patients do not get diagnosed before they die, particularly in less developed areas of the world. Thus, the exact number of patients with these syndromes is difficult to assess.

The current standard for hyperglycaemia treatment in patients having SIR is a controlled diet, supplemented with drugs affecting insulin receptor sensitivity, such as metformin, or insulin supplement. However, particularly for disorders caused by mutations in the insulin receptor gene, this treatment is not sufficiently effective and ultimately proves unsuccessful.

Diabetes comprises two distinct diseases, type 1 (or insulin-dependent diabetes) and type 2 (insulin-independent diabetes), both of which involve the malfunction of glucose homeostasis. Type 2 diabetes affects more than 400 million people in the world and the number is rising rapidly. Complications of type 2 diabetes include severe cardiovascular problems, kidney failure, peripheral neuropathy, blindness and, in the later stages of the disease, even loss of limbs and, ultimately, death. Type 2 diabetes is characterized by insulin resistance in skeletal muscle and adipose tissue, and there is presently no definitive cure. Most treatments used today are focused on remedying dysfunctional insulin signalling or inhibiting glucose output from the liver but many of those treatments have several drawbacks and side effects. There is thus a great interest in identifying novel insulin-independent ways to treat type 2 diabetes.

In particular, it is known that in type 2 diabetes the insulin-signalling pathway is blunted in peripheral tissues, such as adipose tissue and skeletal muscle. Methods for treating type 2 diabetes typically include lifestyle changes, as well as insulin injections or oral medications to regulate glucose homeostasis. People with type 2 diabetes in the later stages of the disease develop 'beta-cell failure' i.e. the inability of the pancreas to release insulin in response to high blood glucose levels. Such patients often require insulin injections in combination with oral medications to manage their diabetes. Further, most common drugs have side effects including downregulation or desensitization of the insulin pathway and/or the promotion of lipid incorporation in adipose tissue, liver and skeletal muscle. There is thus a great interest in identifying novel ways to treat metabolic diseases including type 2 diabetes that do not include these side effects.

Following a meal, increased blood glucose levels stimulate insulin release from the pancreas. Insulin mediates normalization of the blood glucose levels. Important effects of insulin on glucose metabolism include facilitation of glucose uptake into skeletal muscle and adipocytes, and an increase of glycogen storage in the liver. Skeletal muscle and adipocytes are responsible for insulin-mediated glucose uptake and utilization in the fed state, making them very important sites for glucose metabolism.

The signalling pathway downstream from the insulin receptor has been difficult to understand in detail. In brief, control of glucose uptake by insulin involves activation of the insulin receptor (IR), the insulin receptor substrate (IRS), the phosphoinositide 3-kinase (PI3K) and thus stimulation of phosphatidylinositol (3,4,5)-triphosphate (PIP3), the mammalian target of rapamycin (also called the mechanistic target of rapamycin, mTOR), Akt/PKB (Akt) and TBC1D4 (AS160), leading to translocation of the glucose transporter 4 (GLUT4) to the plasma membrane. Akt activation is considered necessary for GLUT4 translocation.

It should be noted that skeletal muscles constitute a major part of the body weight of mammals and have a vital role in the regulation of systemic glucose metabolism, being responsible for up to 85% of whole-body glucose disposal. Glucose uptake in skeletal muscles is regulated by several intra- and extracellular signals. Insulin is the most well studied mediator but others also exist. For example, AMP activated kinase (AMPK) functions as an energy sensor in the cell, which can increase glucose uptake and fatty acid oxidation. Due to the great influence skeletal muscles have on glucose homeostasis it is plausible that additional mechanisms exist. In the light of the increased prevalence of type 2 diabetes, it is of great interest to find and characterize novel insulin-independent mechanisms to increase glucose uptake in muscle cells.

Blood glucose levels may be regulated by both insulin and catecholamines, but they are released in the body in response to different stimuli. Whereas insulin is released in response to the rise in blood sugar levels (e.g. after a meal), epinephrine and norepinephrine are released in response to various internal and external stimuli, such as exercise, emotions and stress, and also for maintaining tissue homeostasis. Insulin is an anabolic hormone that stimulates many processes involved in growth including glucose uptake, glycogen and triglyceride formation, whereas catecholamines are mainly catabolic.

Although insulin and catecholamines normally have opposing effects, it has been shown that they have similar actions on glucose uptake in skeletal muscle (Nevzorova et al., Br. J. Pharmacol, 137, 9, (2002)). In particular, it has been reported that catecholamines stimulate glucose uptake via adrenergic receptors (Nevzorova et al., Br. J. Pharmacol, 147, 446, (2006); Hutchinson, Bengtsson Endocrinology 146, 901, (2005)) to supply muscle cells with an energy-rich substrate. Thus it is likely that in mammals, including humans, the adrenergic and the insulin systems can work independently to regulate the energy needs of skeletal muscle in different situations. Since insulin also stimulates many anabolic processes, including some that promote undesired effects such as stimulation of lipid incorporation into tissues, leading to e.g. obesity, it would be beneficial to be able to stimulate glucose uptake by other means; for example, by stimulation of the adrenergic receptors (ARs).

All ARs are G protein-coupled receptors (GPCRs) located in the cell membrane and characterized by an extracellular N-terminus, followed by seven transmembrane α-helices (TM-1 to TM-7) connected by three intracellular (IL-1 to IL-3) and three extracellular loops (EL-1 to EL-3), and finally an intracellular C-terminus. There are three different classes of ARs, with distinct expression patterns and pharmacological profiles: α₁-, α₂- and β-ARs. The α₁-ARs comprise the α_{1A}, α_{1B} and α_{1D} subtypes while α₂-ARs are divided into α_{2A}, α_{2B} and α_{2C}. The β-ARs are also divided into the subtypes β₁, β₂, and β₃, of which β₂-AR is the major isoform in skeletal muscle cells. ARs are G protein coupled receptors (GPCRs) that signal through classical secondary messengers such as cyclic adenosine monophosphate (cAMP) and phospholipase C (PLC).

Many effects occurring downstream of ARs in skeletal muscles have been attributed to classical secondary messenger signalling, such as increase in cAMP levels, PLC activity and calcium levels. Stimulation involving the classical secondary messengers has many effects in different tissues. For example, it increases heart rate, blood flow, airflow in lungs and release of glucose from the liver, which all can be detrimental or be considered unwanted side effects if stimulation of ARs should be considered as a type 2 diabetes treatment. Adverse effects of classical AR agonists are, for example, tachycardia, palpitation, tremor, sweats, agitation and increased glucose levels in the blood (glucose output from the liver). It would thus be beneficial to be able to activate ARs without activating these classical secondary messengers, such as cAMP, to increase glucose uptake in peripheral tissues without stimulating the unwanted side effects.

Glucose uptake is mainly stimulated via facilitative glucose transporters (GLUT) that mediate glucose uptake into most cells. GLUTs are transporter proteins that mediate transport of glucose and/or fructose over the plasma membrane down the concentration gradient. There are fourteen known members of the GLUT family, named GLUT1-14, divided into three classes (Class I, Class II and Class III) dependent on their substrate specificity and tissue expression. GLUT1 and GLUT4 are the most intensively studied isoforms and, together with GLUT2 and GLUT3, belong to Class I which mainly transports glucose (in contrast to Class II that also transports fructose). GLUT1 is ubiquitously expressed and is responsible for basal glucose transport. GLUT4 is only expressed in peripheral tissues such as skeletal muscle, cardiac muscle and adipose tissues. GLUT4 has also been reported to be expressed in, for example, the brain, kidney, and liver. GLUT4 is the major isoform involved in insulin stimulated glucose uptake. The mechanism whereby insulin signalling increases glucose uptake is mainly via GLUT4 translocation from intracellular storage to the plasma membrane. It is known that GLUT4 translocation is induced by stimulation of the β₂-adrenergic receptor.

Thus, a possible treatment of a condition involving dysregulation of glucose homeostasis or glucose uptake in a mammal, such as type 2 diabetes, would involve the activation of the β₂-adrenergic receptor leading to GLUT4 translocation to the plasma membrane and promotion of glucose uptake into skeletal muscle leading to normalization of whole body glucose homeostasis. In addition, it would be advantageous if the treatment does not involve signalling through cAMP as this would lead to a favourable side-effect profile.

WO 99/65308 describes various 5,5-dimethylpyrrolidines as components of compositions for use in non-therapeutic methods for deterring vermin. Isler D. et al. (Diabetologia (1984), 27(2), 219A) discloses the anti-diabetic activity of the beta-adrenergic receptor agonist Ro 16-8714.

### Description of the Invention

We have now surprisingly found that certain heterocyclyl(phenyl)methanols acting as agonists at the β₂-adrenergic receptor increase glucose uptake in skeletal muscle.

In addition, we have found that this effect is not mediated through significant cAMP release, such that many of the commonly described side effects seen with traditional β₂-adrenergic agonists (e.g. tachycardia, palpitation, tremor, sweats, agitation, and the like) can be reduced.

The use of such compounds in medicine represents a promising strategy for the treatment of conditions characterized by high blood sugar levels (i.e. hyperglycaemia), such as type 2 diabetes.

### Compounds of the invention

In a first aspect of the invention, there is provided a compound of formula I 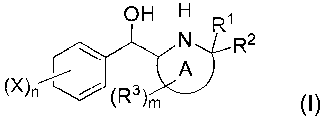 or a pharmaceutically acceptable salt thereof, wherein:
ring A represents a 4- to 8-membered heterocycloalkyl;
each R¹ and R² independently represents C₁₋₆ alkyl optionally substituted by one or more halo;
or alternatively R¹ and R² may be linked together to form together to form a 3- to 6-membered ring, which optionally is substituted by one or more groups independently selected from halo and C₁₋₆ alkyl optionally substituted by one more halo;
each R³ independently represents halo or C₁₋₆ alkyl optionally substituted by one or more halo;
each X independently represents halo, R^{a}, -CN, -N₃, -N(R^{b})R^{c}, -NO₂, -ONO₂, -OR^{d}, -S(O)ₚR^{e} or -S(O)_{q}N(R^{f})R^{g};
R^{a} represents C₁₋₆ alkyl optionally substituted by one or more groups independently selected from G¹;
each R^{b}, R^{c}, R^{d}, R^{e}, R^{f} and R^{g} independently represents H or C₁₋₆ alkyl optionally substituted by one or more groups independently selected from G²;
or alternatively any of R^{b} and R^{c} and/or R^{f} and R^{g} may be linked together to form, together with the nitrogen atom to which they are attached, a 4- to 6-membered ring, which ring optionally contains one further heteroatom and which ring optionally is substituted by one or more groups independently selected from halo, C₁₋₃ alkyl optionally substituted by one or more halo, and =O;
G¹ and G² represents halo, -CN, -N(R^{a1})R^{b1}, -OR^{c1}, -S(O)ₚR^{d1}, -S(O)_{q}N(R^{e1})R^{f1} or =O;
each R^{a1}, R^{b1}, R^{c1}, R^{d1}, R^{e1} and R^{f1} independently represents H or C₁₋₆ alkyl optionally substituted by one or more halo;
or alternatively any of R^{a1} and R^{b1} and/or R^{e1} and R^{f1} may be linked together to form, together with the nitrogen atom to which they are attached, a 4- to 6-membered ring, which ring optionally contains one further heteroatom and which ring optionally is substituted by one or more groups independently selected from halo, C₁₋₃ alkyl optionally substituted by one or more halo, and =O;
n represents 0 to 5;
each p independently represents 0, 1 or 2;
each q independently represents 1 or 2; and
m represents 0 to 11, as appropriate, with the proviso that the compound is not a compound (1) - (36) selected from the list mentioned at pages 13 - 14 of the present description;
which compounds (including pharmaceutically acceptable salts) may be referred to herein as "compounds of the invention".

For the avoidance of doubt, the skilled person will understand that references herein to compounds of particular aspects of the invention (such as the first aspect of the invention, e.g. compounds of formula I) will include references to all embodiments and particular features thereof, which embodiments and particular features may be taken in combination to form further embodiments.

Unless indicated otherwise, all technical and scientific terms used herein have the same meaning as commonly understood by one of ordinary skill in the art to which this invention pertains.

Pharmaceutically acceptable salts include acid addition salts and base addition salts. Such salts may be formed by conventional means, for example by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

Particular acid addition salts that may be mentioned include carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxy-benzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulphonate salts (e.g. benzenesulphonate, methyl-, bromo- or chloro-benzenesulphonate, xylenesulphonate, methanesulphonate, edisylate, ethanesulphonate, propanesulphonate, hydroxy-ethanesulphonate, 1- or 2- naphthalene-sulphonate or 1,5-naphthalenedisulphonate salts) or sulphate, pyrosulphate, bisulphate, sulphite, bisulphite, phosphate, monohydrogenphosphate, dihydrogenphosphate, metaphosphate, pyrophosphate or nitrate salts, and the like.

Particular acid addition salts that may be mentioned include acetate, bisulphate, fumarate, hydrobromide, hydrochloride, maleate and sulphate salts.

More particular acid addition salts that may be mentioned include bisulphate, hydrochloride and maleate salts.

For the avoidance of doubt, the skilled person will understand that acid addition salts may include diacid salts (e.g. dihydrochloride salts).

Particular base addition salts that may be mentioned include salts formed with alkali metals (such as Na and K salts), alkaline earth metals (such as Mg and Ca salts), organic bases (such as ethanolamine, diethanolamine, triethanolamine, tromethamine and lysine) and inorganic bases (such as ammonia and aluminium hydroxide). More particularly, base addition salts that may be mentioned include Mg, Ca and, most particularly, K and Na salts.

For the avoidance of doubt, compounds of the first aspect of the invention may exist as solids, and thus the scope of the invention includes all amorphous, crystalline and part crystalline forms thereof, and may also exist as oils. Where compounds of the first aspect of the invention exist in crystalline and part crystalline forms, such forms may include solvates, which are included in the scope of the invention. Compounds of the first aspect of the invention may also exist in solution.

Compounds of the first aspect of the invention may contain double bonds and may thus exist as *E* (*entgegen*) and *Z* (*zusammen*) geometric isomers about each individual double bond. All such isomers and mixtures thereof are included within the scope of the invention.

Compounds of the first aspect of the invention may also exhibit tautomerism. All tautomeric forms and mixtures thereof are included within the scope of the invention.

Compounds of the first aspect of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers (i.e. enantiomers) may be isolated by separation of a racemic or other mixture of the compounds using conventional, e.g. fractional crystallisation or HPLC, techniques. Alternatively the desired optical isomers may be obtained from appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution); for example, with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

As used herein, the term heterocycloalkyl may refer to non-aromatic, saturated and monocyclic groups wherein at least one atom comprised in the ring is a heteroatom (i.e. saturated heterocyclic groups). In particular, such groups may comprise from 1 to 4 heteroatoms, such as heteroatoms selected from O, S and N, which N may be present in secondary or tertiary degrees of substitution.

For the avoidance of doubt, ring A, as described in compounds of formula I, contains an essential nitrogen atom and two essential carbon atoms, as represented in the 2-position of ring A (i.e. in the position alpha to both the essential nitrogen atom of the A ring and the carbon bearing the essential -OH group) and the carbon atom in the other ring position alpha to the essentially nitrogen atom. Thus, for the avoidance of doubt, in particular embodiments ring A will be understood to contain one heteroatom which is the essential N atom.

For the avoidance of doubt, ring A may be substituted by a number of R³ groups, as defined herein, which number is defined by m, as defined herein. The skilled person will understand that the (maximum) number and position of such substituents will be dictated by the nature of the heterocyclic ring, such as by the size of the ring and the number and type of heteroatoms comprised therein. Thus, where m is defined as 0 to 11, it will be understood that the value 11 represents a theoretical maximum when considering the heterocyclic rings that may be present as ring A, and that for certain heterocyclic groups representing ring A the actual maximum value for m may be lower, as will be readily determined by the skilled person. Moreover, the skilled person will understand that such substituents may be present on suitable moieties comprised within ring A, such as C (carbon) moieties and secondary N (nitrogen) moieties.

In particular, ring A as defined herein may comprise one or two heteroatoms (including the essential NH moiety), which may be selected (in addition to the essential NH moiety) from O, S and N (e.g. O and N, such as N). Thus, in addition to the essential NH moiety, ring A as defined herein may comprise up to one additional heteroatom, which may be selected from O, S and N (e.g. O and N, such as N).

In particular, ring A as defined herein may be 4- to 6-membered. For example, ring A as defined herein may be 4- to 6-membered comprising one or two heteroatoms (i.e. a 4-membered ring may comprise up to one heteroatom and a 5- or 6-membered ring may comprise up to 1 or 2 heteroatoms), which may be selected from O, S and N (e.g. O and N, such as N).

More particularly, ring A as defined herein may be 5- or 6-membered. For example, ring A as defined herein may be 5- or 6-membered comprising one or two heteroatoms (i.e. up to one additional heteroatom), which may be selected from O, S and N (e.g. O and N, such as N).

More particularly, ring A as defined herein may be a 4-membered. For example, ring A as defined herein may be a 4-membered comprising one heteroatom, which is the essential N atom.

Particular heterocycloalkyl groups that may be mentioned (e.g. in relation to ring A as defined for compounds of formula I, including all embodiments thereof) include azetidinyl (e.g. azetidine-2-yl, wherein position 1 is the N atom), pyrrolidinyl (e.g. pyrrolidine-2-yl), piperidinyl (e.g. piperidin-2-yl) and azepanyl (e.g. azepan-2-yl).

More particular heterocycloalkyl groups that may be mentioned (e.g. in relation to ring A) include azetidinyl (e.g. azetidine-2-yl) pyrrolidinyl (e.g. pyrrolidine-2-yl) and piperidinyl (e.g. piperidin-2-yl).

More particular heterocycloalkyl groups may be azetidinyl (e.g. azetidine-2-yl).

More particular heterocycloalkyl groups may be pyrrolidinyl (e.g. pyrrolidine-2-yl).

More particular heterocycloalkyl groups may be piperidinyl (e.g. piperidin-2-yl).

As used herein, references to halo and/or halogen groups will each independently refer to fluoro, chloro, bromo and iodo (for example, fluoro (F) and chloro (CI), such as F).

Unless otherwise specified, C_{1-z} alkyl groups (where z is the upper limit of the range) defined herein may be straight-chain or, when there is a sufficient number (i.e. a minimum of three) of carbon atoms, be branched-chain, and/or cyclic (so forming a C_{3-z}-cycloalkyl group). When there is a sufficient number (i.e. a minimum of four) of carbon atoms, such groups may also be part cyclic. Part cyclic alkyl groups that may be mentioned include cyclopropylmethyl and cyclohexylethyl. When there is a sufficient number of carbon atoms, such groups may also be multicyclic (e.g. bicyclic or tricyclic) or spirocyclic. Such alkyl groups may also be saturated or, when there is a sufficient number (i.e. a minimum of two) of carbon atoms, be unsaturated (forming, for example, a C_{2-z} alkenyl or a C_{2-z} alkynyl group). Particular alkyl groups that may be mentioned include saturated alkyl groups.

For the avoidance of doubt, as used herein, references to heteroatoms will take their normal meaning as understood by one skilled in the art. Particular heteroatoms that may be mentioned include phosphorus, selenium, tellurium, silicon, boron, oxygen, nitrogen and sulphur (e.g. oxygen, nitrogen and sulphur).

For the avoidance of doubt, references to polycyclic (e.g. bicyclic or tricyclic) groups (e.g. when employed in the context of cycloalkyl groups) will refer to ring systems wherein at least two scissions would be required to convert such rings into a straight chain, with the minimum number of such scissions corresponding to the number of rings defined (e.g. the term bicyclic may indicate that a minimum of two scissions would be required to convert the rings into a straight chain). For the avoidance of doubt, the term bicyclic (e.g. when employed in the context of alkyl groups) may refer to groups in which the second ring of a two-ring system is formed between two adjacent atoms of the first ring, and may also refer to groups in which two non-adjacent atoms are linked by an alkylene group, which later groups may be referred to as bridged.

The present invention also embraces isotopically-labelled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, the compounds of the invention also include deuterated compounds, i.e. in which one or more hydrogen atoms are replaced by the hydrogen isotope deuterium.

For the avoidance of doubt, in cases in which the identity of two or more substituents in a compound of the invention may be the same, the actual identities of the respective substituents are not in any way interdependent. For example, in the situation in which two or more X groups are present, those X groups may be the same or different. Similarly, where two or more X groups are present and each represent halo, the halo groups in question may be the same or different. Likewise, when more than one R^{a} is present and each independently represents C₁₋₆ alkyl substituted by one or more G group, the identities of each G are in no way interdependent.

The skilled person will appreciate that compounds of the invention that are the subject of this invention include those that are stable. That is, compounds of the invention include those that are sufficiently robust to survive isolation, e.g. from a reaction mixture, to a useful degree of purity.

All embodiments of the invention and particular features mentioned herein may be taken in isolation or in combination with any other embodiments and/or particular features mentioned herein (hence describing more particular embodiments and particular features as disclosed herein) without departing from the disclosure of the invention.

According to the invention, the compound of formula I is not a compound selected from the list consisting of:
(1) (*S*)-((*S*)-5,5-dimethylpyrrolidin-2-yl)(4-(methylthio)phenyl)methanol
(2) (3,4-dichlorophenyl)(5,5-dimethylpyrrolidin-2-yl)methanol
(3) (5,5-dimethylpyrrolidin-2-yl)(*p*-tolyl)methanol
(4) (4-chlorophenyl)(5,5-dimethylpyrrolidin-2-yl)methanol
(5) 3-((5,5-dimethylpyrrolidin-2-yl)(hydroxy)methyl)benzonitrile
(6) (5,5-dimethylpyrrolidin-2-yl)(*m*-tolyl)methanol
(7) (5,5-dimethylpyrrolidin-2-yl)(3-(trifluoromethyl)phenyl)methanol
(8) (5,5-dimethylpyrrolidin-2-yl)(phenyl)methanol
(9) (2,4-dichlorophenyl)(5,5-dimethylpyrrolidin-2-yl)methanol
(10) (2,6-dichlorophenyl)(5,5-dimethylpyrrolidin-2-yl)methanol
(11) (3,4-dichlorophenyl)(6,6-dimethylpiperidin-2-yl)methanol
(12) (3-chlorophenyl)(6,6-dimethylpiperidin-2-yl)methanol
(13) (2,4-dimethylphenyl)(5,5-dimethylpyrrolidin-2-yl)methanol
(14) (3-chlorophenyl)(5,5-dimethylpyrrolidin-2-yl)methanol
(15) (4-chlorophenyl)(6,6-dimethylpiperidin-2-yl)methanol
(16) (*R**)-(4-chlorophenyl)((*S**)-5,5-dimethylpyrrolidin-2-yl)methanol
(17) (*R**)-(4-chlorophenyl)((*R**)-5,5-dimethylpyrrolidin-2-yl)methanol
(18) (*R**)-(3,4-dichlorophenyl)((*S**)-5,5-dimethylpyrrolidin-2-yl)methanol
(19) (*R**)-(3,4-dichlorophenyl)((*R**)-5,5-dimethylpyrrolidin-2-yl)methanol
(20) (*R**)-(3-chlorophenyl)((*S**)-5,5-dimethylpyrrolidin-2-yl)methanol
(21) (*R**)-(3-chlorophenyl)((*R**)-5,5-dimethylpyrrolidin-2-yl)methanol
(22) (*R**)-(3-chlorophenyl)((*R**)-6,6-dimethylpiperidin-2-yl)methanol
(23) (*R**)-((*S**)-5,5-dimethylpyrrolidin-2-yl)(3-(trifluoromethyl)phenyl)methanol
(24) (*R**)-((*R**)-5,5-dimethylpyrrolidin-2-yl)(3-(trifluoromethyl)phenyl)methanol
(25) (*R**)-((*S**)-5,5-dimethylpyrrolidin-2-yl)(phenyl)methanol
(26) (*R**)-((*R**)-5,5-dimethylpyrrolidin-2-yl)(phenyl)methanol
(27) (*R**)-((*S**)-5,5-dimethylpyrrolidin-2-yl)(m-tolyl)methanol
(28) (*R**)-((*R**)-5,5-dimethylpyrrolidin-2-yl)(m-tolyl)methanol
(29) (*R**)-(2,6-dichlorophenyl)((*S**)-5,5-dimethylpyrrolidin-2-yl)methanol
(30) (*R*)-(2,6-dichlorophenyl)((*R*)-5,5-dimethylpyrrolidin-2-yl)methanol
(31) (*R**)-(3,4-dichlorophenyl)((*S**)-6,6-dimethylpiperidin-2-yl)methanol
(32) (*R**)-(3,4-dichlorophenyl)((*R**)-6,6-dimethylpiperidin-2-yl)methanol
(33) (*R**)-(3-chlorophenyl)((*S**)-6,6-dimethylpiperidin-2-yl)methanol
(34) (*R**)-(2,4-dichlorophenyl)((*S**)-5,5-dimethylpyrrolidin-2-yl)methanol
(35) (*R**)-(2,4-dichlorophenyl)((*R**)-5,5-dimethylpyrrolidin-2-yl)methanol
(36) 3-((*R**)-((*R**)-5,5-dimethylpyrrolidin-2-yl)(hydroxy)methyl)benzonitrile

The skilled person will understand that chiral centres denoted with an * indicates that the stereochemistry is relative.

In a more particular embodiment of the first aspect of the invention, the compound of formula I is not a compound selected from the list consisting of:
(1) (5,5-dimethylpyrrolidin-2-yl)(4-(methylthio)phenyl)methanol
(2) (3,4-dichlorophenyl)(5,5-dimethylpyrrolidin-2-yl)methanol
(3) (5,5-dimethylpyrrolidin-2-yl)(*p*-tolyl)methanol
(4) (4-chlorophenyl)(5,5-dimethylpyrrolidin-2-yl)methanol
(5) 3-(5,5-dimethylpyrrolidin-2-yl)(hydroxy)methyl)benzonitrile
(6) (5,5-dimethylpyrrolidin-2-yl)(*m*-tolyl)methanol
(7) (5,5-dimethylpyrrolidin-2-yl)(3-(trifluoromethyl)phenyl)methanol
(8) (5,5-dimethylpyrrolidin-2-yl)(phenyl)methanol
(9) (2,4-dichlorophenyl)(5,5-dimethylpyrrolidin-2-yl)methanol
(10) (2,6-dichlorophenyl)(5,5-dimethylpyrrolidin-2-yl)methanol
(11) (3,4-dichlorophenyl)(6,6-dimethylpiperidin-2-yl)methanol
(12) (3-chlorophenyl)(6,6-dimethylpiperidin-2-yl)methanol
(13) (2,4-dimethylphenyl)(5,5-dimethylpyrrolidin-2-yl)methanol
(14) (3-chlorophenyl)(5,5-dimethylpyrrolidin-2-yl)methanol
(15) (4-chlorophenyl)(6,6-dimethylpiperidin-2-yl)methanol

In certain embodiments of the first aspect of the invention, there is provided a compound of formula IA (i.e. the compound of formula I may be a compound of formula IA) 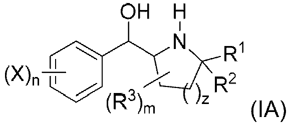
or a pharmaceutically available salts thereof, wherein;
R¹, R², R³, X, n and m are as defined herein (i.e. including all embodiments thereof);
z represents 1 or 2; and
when z represents 1 then m represents 0 to 5, and when z represents 2 then m represents 0 to 7.

Further, in certain embodiments of the first aspects of the invention, there is provided a compound of formula IX (i.e. the compound of formula I may be a compound of formula IX) 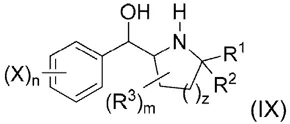
or a pharmaceutically available salt thereof, wherein;
R¹, R², R³, X, n and m are as defined herein (i.e. including all embodiments thereof);
z represents 0; and
m represents 0 to 3.

For avoidance of doubt, the skilled person will understand that:
when z represents 0 (i.e. the ring containing the essential nitrogen atom is an azetidine ring), then m may be 0, 1, 2, 3, 4 or 5 (e.g. 0 or 1), such as 1, 2, 3, 4 or 5 (i.e. 1 to 5);
when z represents 1 (i.e the ring containing the essential nitrogen atom is a pyrrolidin-2-yl ring), then m may be 0, 1, 2, 3, 4 or 5 (e.g. 0 or 1, such as 0); and
when z represents 2 (i.e the ring containing the essential nitrogen atom is a piperidine ring), then m may be 0, 1, 2, 3, 4, 5, 6 or 7 (e.g. 0 or 1, such as 0).

In certain embodiments, z represents 0.

In certain embodiments, z represents 1.

In certain embodiments, z represents 2.

In certain embodiments, z represents 1 or 2.

In certain embodiments, there is provided a compound of the invention wherein each X independently represents halo (e.g. Cl or F, such as F), OH, NH₂, CN, or CF₃.

In certain embodiments, there is provided a compound of the invention wherein each X independently represents halo (e.g. Cl or F) or NH₂.

In particular embodiments, each X independently represents halo (e.g. Cl or F, such as F).

In more particular embodiments, at least one (e.g. one) X represents Cl or F (in particular F).

In more particular embodiments, at least one (e.g. one) X group is present and represents F.

In particular embodiments, n represents 1.

In certain particular embodiment, when n represents 1, X is in the *ortho-* position.

In certain particular embodiment, when n represents 1, X is in the *meta-* position.

In particular embodiments, n represents 2.

In certain particular embodiments, when n represents 2, one X is in the *ortho*-position and one X is in the *meta*-position.

In particular embodiments, n represents 3.

In certain particular embodiments, when n represents 3, two of the X substituents are in the *meta-* position and one X is in the *para-* position.

In particular embodiments, m represents 0.

In certain embodiments, each R¹ and R² independently represents C₁₋₃ alkyl (e.g. methyl, ethyl, *n*-propyl, such as methyl or n-propyl) optionally substituted by one or more halo (e.g. one or more F).

In particular embodiments, each R¹ and R² independently represent C₁ alkyl optionally substituted by one or more F (e.g. methyl).

In further embodiments, each R¹ and R² independently represents C₃ alkyl optionally substituted by one or more F (e.g. *n*-propyl).

In particular embodiments that may be mentioned (particularly where R¹ and R² are not linked), R¹ and R² are identical groups (i.e. R¹ and R² are the same).

In certain embodiments, R¹ and R² may also be linked together to form a 3- to 5- membered ring, which is optionally substituted by one or more groups independently selected from halo and C₁₋₆ alkyl optionally substituted by one or more halo.

In particular embodiments, R¹ and R² are linked together to form a 3- to 5- membered cycloalkyl optionally substituted by one or more F.

In particular embodiments, R¹ and R² are linked together to form a 3- membered cycloalkyl optionally substituted by one or more F.

In particular embodiments, R¹ and R² are linked together to form a 5- membered cycloalkyl optionally substituted by one or more F.

In certain embodiments, each R¹ and R² independently represents C₁₋₃ alkyl (e.g. methyl, ethyl, *n*-propyl, such as methyl or *n*-propyl) optionally substituted by one or more halo (e.g. one or more F), or R¹ and R² may be linked together to form a 3- to 5- membered ring, which is optionally substituted by one or more groups independently selected from halo and C₁₋₆ alkyl optionally substituted by one or more halo .

Particular compounds of the first aspect of the invention that may be mentioned include the compounds of the examples provided herein, and pharmaceutically acceptable salts thereof. For the avoidance of doubt, compounds of the examples that are salts may also be provided as the non-salt form or in the form of any (other) pharmaceutically acceptable salt thereof.

For example, compounds of formula I that may be mentioned include:
(1) (R)-((R)-6,6-Dimethylpiperidin-2-yl)(3-fluorophenyl)methanol;
(2) (S)-((R)-6,6-Dimethylpiperidin-2-yl)(3-fluorophenyl)methanol;
(3) 3-((R)-((R)-6,6-Dimethylpiperidin-2-yl)(hydroxy)methyl)phenol (e.g. 3-((R)-((R)-6,6-Dimethylpiperidin-2-yl)(hydroxy)methyl)phenol acetate);
(4) 3-((S)-((R)-6,6-Dimethylpiperidin-2-yl)(hydroxy)methyl)phenol (e.g. 3-((S)-((R)-6,6-Dimethylpiperidin-2-yl)(hydroxy)methyl)phenol acetate);
(5) (R)-(2-Chlorophenyl)((R)-6,6-dimethylpiperidin-2-yl)methanol (e.g. (R)-(2-Chlorophenyl)((R)-6,6-dimethylpiperidin-2-yl)methanol hydrochloride);
(6) (S)-(2-Chlorophenyl)((R)-6,6-dimethylpiperidin-2-yl)methanol (e.g. (S)-(2-Chlorophenyl)((R)-6,6-dimethylpiperidin-2-yl)methanol hydrochloride);
(7) (R)-(2-Chlorophenyl)((R)-6,6-dimethylpiperidin-2-yl)methanol (e.g. (R)-(2-Chlorophenyl)((R)-6,6-dimethylpiperidin-2-yl)methanol hydrochloride);
(8) (S)-(2-Chlorophenyl)((R)-6,6-dimethylpiperidin-2-yl)methanol (e.g. (S)-(2-Chlorophenyl)((R)-6,6-dimethylpiperidin-2-yl)methanol hydrochloride);
(9) (R)-((R)-6,6-Dimethylpiperidin-2-yl)(2-fluorophenyl)methanol (e.g. (R)-((R)-6,6-Dimethylpiperidin-2-yl)(2-fluorophenyl)methanol hydrochloride);
(10) (S)-((R)-6,6-Dimethylpiperidin-2-yl)(2-fluorophenyl)methanol (e.g. (S)-((R)-6,6-Dimethylpiperidin-2-yl)(2-fluorophenyl)methanol hydrochloride);
(11) (R)-((R)-5,5-Dimethylpyrrolidin-2-yl)(3-fluorophenyl)methanol (e.g. (R)-((R)-5,5-Dimethylpyrrolidin-2-yl)(3-fluorophenyl)methanol hydrochloride);
(12) (S)-((R)-5,5-Dimethylpyrrolidin-2-yl)(3-fluorophenyl)methanol (e.g. (S)-((R)-5,5-Dimethylpyrrolidin-2-yl)(3-fluorophenyl)methanol hydrochloride);
(13) (R)-((S)-5,5-Dimethylpyrrolidin-2-yl)(3-fluorophenyl)methanol (e.g. (R)-((S)-5,5-Dimethylpyrrolidin-2-yl)(3-fluorophenyl)methanol hydrochloride);
(14) (S)-((S)-5,5-Dimethylpyrrolidin-2-yl)(3-fluorophenyl)methanol (e.g. (S)-((S)-5,5-Dimethylpyrrolidin-2-yl)(3-fluorophenyl)methanol hydrochloride);
(15) (R)-((R)-5,5-Dimethylpyrrolidin-2-yl)(hydroxy)methyl)phenol (e.g. (R)-((R)-5,5-Dimethylpyrrolidin-2-yl)(hydroxy)methyl)phenol acetate);
(16) (S)-((R)-5,5-Dimethylpyrrolidin-2-yl)(hydroxy)methyl)phenol (e.g. (S)-((R)-5,5-Dimethylpyrrolidin-2-yl)(hydroxy)methyl)phenol acetate);
(17) (R)-((S)-5,5-Dimethylpyrrolidin-2-yl)(hydroxy)methyl)phenol;
(18) (S)-((S)-5,5-Dimethylpyrrolidin-2-yl)(hydroxy)methyl)phenol;
(19) (R)-((R)-5,5-Dimethylpyrrolidin-2-yl)(2-fluorophenyl)methanol (e.g. (R)-((R)-5,5-Dimethylpyrrolidin-2-yl)(2-fluorophenyl)methanol hydrochloride);
(20) (S)-((R)-5,5-Dimethylpyrrolidin-2-yl)(2-fluorophenyl)methanol (e.g. (S)-((R)-5,5-Dimethylpyrrolidin-2-yl)(2-fluorophenyl)methanol hydrochloride);
(21) (R)-((R)-5,5-Dimethylpyrrolidin-2-yl)(4-fluorophenyl)methanol (e.g. (R)-((R)-5,5-Dimethylpyrrolidin-2-yl)(4-fluorophenyl)methanol hydrochloride);
(22) (S)-((R)-5,5-Dimethylpyrrolidin-2-yl)(4-fluorophenyl)methanol (e.g. (S)-((R)-5,5-Dimethylpyrrolidin-2-yl)(4-fluorophenyl)methanol hydrochloride);
(23) (R)-(2-Chlorophenyl)((R)-5,5-dimethylpyrrolidin-2-yl)methanol (e.g. (R)-(2-Chlorophenyl)((R)-5,5-dimethylpyrrolidin-2-yl)methanol hydrochloride);
(24) (S)-(2-Chlorophenyl)((R)-5,5-dimethylpyrrolidin-2-yl)methanol (e.g. (S)-(2-Chlorophenyl)((R)-5,5-dimethylpyrrolidin-2-yl)methanol hydrochloride);
(25) (R)-(3-Chlorophenyl)((R)-5,5-dimethylpyrrolidin-2-yl)methanol (e.g. (R)-(3-Chlorophenyl)((R)-5,5-dimethylpyrrolidin-2-yl)methanol hydrochloride);
(26) (S)-(2-Chlorophenyl)((R)-5,5-dimethylpyrrolidin-2-yl)methanol (e.g. (S)-(2-Chlorophenyl)((R)-5,5-dimethylpyrrolidin-2-yl)methanol hydrochloride);
(27) (R)-((R)-5,5-Dipropylpyrrolidin-2-yl)(3-fluorophenyl)methanol (e.g. (R)-((R)-5,5-Dipropylpyrrolidin-2-yl)(3-fluorophenyl)methanol hydrochloride);
(28) (S)-((R)-5,5-Dipropylpyrrolidin-2-yl)(3-fluorophenyl)methanol (e.g. (S)-((R)-5,5-Dipropylpyrrolidin-2-yl)(3-fluorophenyl)methanol hydrochloride);
(29) (R)-(3-Fluorophenyl)((R)-4-azaspiro[2.4]heptan-5-yl)methanol (e.g. (R)-(3-Fluorophenyl)((R)-4-azaspiro[2.4]heptan-5-yl)methanol hydrochloride);
(30) (S)-(3-Fluorophenyl)((R)-4-azaspiro[2.4]heptan-5-yl)methanol (e.g. (S)-(3-Fluorophenyl)((R)-4-azaspiro[2.4]heptan-5-yl)methanol hydrochloride);
(31) (R)-(3-fluorophenyl)((R)-1-azaspiro[4.4]nonan-2-yl)methanol (e.g. (R)-(3-fluorophenyl)((R)-1-azaspiro[4.4]nonan-2-yl)methanol maleate); and
(32) (S)-(3-fluorophenyl)((R)-1-azaspiro[4.4]nonan-2-yl)methanol (e.g. (S)-(3-fluorophenyl)((R)-1-azaspiro[4.4]nonan-2-yl)methanol maleate),
and pharmaceutically acceptable salts thereof.

Further compounds of formula I that may be mentioned include:
(33) (R)-((R)-4,4-dimethylazetidin-2-yl)(3-fluorophenyl)methanol (e.g. (R)-((R)-4,4-dimethylazetidin-2-yl)(3-fluorophenyl)methanol hydrochloride);
(34) (S)-((S)-4,4-dimethylazetidin-2-yl)(2-fluorophenyl)methanol (e.g. (S)-((S)-4,4-dimethylazetidin-2-yl)(2-fluorophenyl)methanol hydrochloride);
(35) (R)-((R)-4,4-dimethylazetidin-2-yl)(2-fluorophenyl)methanol (e.g. (R)-((R)-4,4-dimethylazetidin-2-yl)(2-fluorophenyl)methanol hydrochloride);
(36) (S)-((R)-4,4-dimethylazetidin-2-yl)(2-fluorophenyl)methanol (e.g. (S)-((R)-4,4-dimethylazetidin-2-yl)(2-fluorophenyl)methanol hydrochloride);
(37) (R)-((S)-4,4-dimethylazetidin-2-yl)(2-fluorophenyl)methanol (e.g. (R)-((S)-4,4-dimethylazetidin-2-yl)(2-fluorophenyl)methanol hydrochloride);
(38) (S)-((R)-4,4-dimethylazetidin-2-yl)(2-fluorophenyl)methanol (e.g. (S)-((R)-4,4-dimethylazetidin-2-yl)(2-fluorophenyl)methanol hydrochloride);
(39) (R)-((S)-4,4-dimethylazetidin-2-yl)(3-fluorophenyl)methanol (e.g. (R)-((S)-4,4-dimethylazetidin-2-yl)(3-fluorophenyl)methanol hydrochloride);
(40) (S)-((S)-4,4-dimethylazetidin-2-yl)(3-fluorophenyl)methanol (e.g. (S)-((S)-4,4-dimethylazetidin-2-yl)(3-fluorophenyl)methanol hydrochloride);
(41) (R)-((S)-6,6-dimethylpiperidin-2-yl)(2-fluorophenyl)methanol (e.g. (R)-((S)-6,6-dimethylpiperidin-2-yl)(2-fluorophenyl)methanol hydrochloride);
(42) (S)-((S)-6,6-dimethylpiperidin-2-yl)(2-fluorophenyl)methanol (e.g. (S)-((S)-6,6-dimethylpiperidin-2-yl)(2-fluorophenyl)methanol hydrochloride);
(43) (S)-((S)-6,6-dimethylpiperidin-2-yl)(3-fluorophenyl)methanol (e.g. (S)-((S)-6,6-dimethylpiperidin-2-yl)(3-fluorophenyl)methanol hydrochloride);
(44) (R)-((S)-6,6-dimethylpiperidin-2-yl)(3-fluorophenyl)methanol (e.g. (R)-((S)-6,6-dimethylpiperidin-2-yl)(3-fluorophenyl)methanol hydrochloride);
(45) (R)-(3-chlorophenyl)((R)-4,4-dimethylazetidin-2-yl)methanol (e.g. (R)-(3-chlorophenyl)((R)-4,4-dimethylazetidin-2-yl)methanol maleate);
(46) (S)-(3-chlorophenyl)((R)-4,4-dimethylazetidin-2-yl)methanol (e.g. (S)-(3-chlorophenyl)((R)-4,4-dimethylazetidin-2-yl)methanol maleate);
(47) (R)-(3-chlorophenyl)((S)-4,4-dimethylazetidin-2-yl)methanol (e.g. (R)-(3-chlorophenyl)((S)-4,4-dimethylazetidin-2-yl)methanol maleate);
(48) (S)-(3-chlorophenyl)((S)-4,4-dimethylazetidin-2-yl)methanol (e.g. (S)-(3-chlorophenyl)((S)-4,4-dimethylazetidin-2-yl)methanol maleate);
(49) (R)-(3-amino-2-fluorophenyl)((R)-5,5-dimethylpyrrolidin-2-yl)methanol (e.g. (R)-(3-amino-2-fluorophenyl)((R)-5,5-dimethylpyrrolidin-2-yl)methanol hydrochloride);
(50) (S)-(3-amino-2-fluorophenyl)((R)-5,5-dimethylpyrrolidin-2-yl)methanol (e.g. (S)-(3-amino-2-fluorophenyl)((R)-5,5-dimethylpyrrolidin-2-yl)methanol hydrochloride); and
(51) (S)-(4-Amino-3,5-difluorophenyl)((R)-5,5-dimethylpyrrolidin-2-yl)methanol (e.g. (S)-(4-Amino-3,5-difluorophenyl)((R)-5,5-dimethylpyrrolidin-2-yl)methanol hydrochloride),
and pharmaceutically acceptable salts thereof.

In particular, compounds of formula I that may be mentioned include:
(a) (S)-(2-Chlorophenyl)((R)-6,6-dimethylpiperidin-2-yl)methanol (e.g. (S)-(2-Chlorophenyl)((R)-6,6-dimethylpiperidin-2-yl)methanol hydrochloride);
(b) (S)-((R)-6,6-Dimethylpiperidin-2-yl)(2-fluorophenyl)methanol (e.g. (S)-((R)-6,6-Dimethylpiperidin-2-yl)(2-fluorophenyl)methanol hydrochloride);
(c) (R)-((R)-5,5-Dimethylpyrrolidin-2-yl)(3-fluorophenyl)methanol (e.g. (R)-((R)-5,5-Dimethylpyrrolidin-2-yl)(3-fluorophenyl)methanol hydrochloride);
(d) (R)-((R)-5,5-Dimethylpyrrolidin-2-yl)(hydroxy)methyl)phenol (e.g. (R)-((R)-5,5-Dimethylpyrrolidin-2-yl)(hydroxy)methyl)phenol acetate);
(e) (R)-((R)-5,5-Dimethylpyrrolidin-2-yl)(2-fluorophenyl)methanol (e.g. (R)-((R)-5,5-Dimethylpyrrolidin-2-yl)(2-fluorophenyl)methanol hydrochloride);
(f) (R)-(2-Chlorophenyl)((R)-5,5-dimethylpyrrolidin-2-yl)methanol (e.g. (R)-(2-Chlorophenyl)((R)-5,5-dimethylpyrrolidin-2-yl)methanol hydrochloride); and/or
(g) (R)-(3-Chlorophenyl)((R)-5,5-dimethylpyrrolidin-2-yl)methanol (e.g. (R)-(3-Chlorophenyl)((R)-5,5-dimethylpyrrolidin-2-yl)methanol hydrochloride),
and pharmaceutically acceptable salts thereof.

As described herein, compounds of the first aspect of the invention may also contain one or more asymmetric carbon atoms and may therefore exhibit optical and/or diastereoisomerism. Moreover, it has been found that certain such optical and/or diastereoisomers may show increased utility in the treatment of hyperglycaemia or disorders characterized by hyperglycaemia (such as type 2 diabetes), as described herein.

In a certain embodiments of the first aspect of the invention, the right-hand side of the compound may be depicted as follows 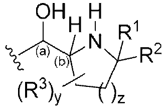 wherein the carbon substituted with the essential -OH group (denoted with (a)) is chiral and may be in either the *(R)* or *(S)* configuration, and the carbon beta to the hydroxy group and adjoined to ring A (is denoted with (b)) is chiral and may be in either the *(R)* or *(S)* configuration.

In a particular embodiment, wherein z represents 1 (in which case the skilled person will understand that ring A is pyrrolidine-2-yl), and carbon (a) is in the *(R)* configuration.

In a particular embodiment, wherein z represents 1, carbon (a) is in the *(R)* configuration and carbon (b) is in the *(R)* configuration.

In a further embodiment, wherein z represents 2 (in which case ring A is piperidin-2-yl), carbon (a) is in the *(S)* configuration.

In a more particular embodiment, wherein z represents 2, carbon (a) is in the *(S)* configuration and carbon (b) is in the *(R)* configuration.

In a particular embodiment, wherein z represents 0 (in which case the skilled person will understand that ring A is azetidin-2-yl), and carbon (a) is in the *(R)* configuration.

In a particular embodiment, wherein z represents 0, carbon (a) is in the *(R)* configuration and carbon (b) is in the *(R)* configuration.

In a particular embodiment, wherein z represents 1, compounds of formula IA may be depicted as IA¹ and IA²

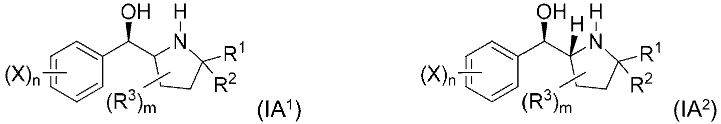

In a particular embodiment, wherein z represents 2, compounds of formula IA may be depicted as IA³ and IA⁴

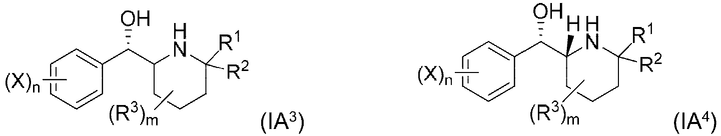

In a particular embodiment, wherein z represents 0, compounds of formula IX may be depicted as IX¹ and IX²

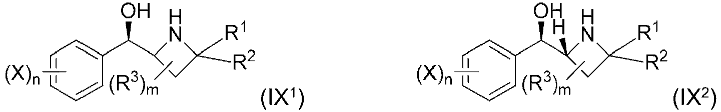

For the avoidance of doubt, compound depicted herein as having a certain stereochemistry may also be depicted with the relevant stereochemistry labelled. For example, compounds of formula IA⁴ may be depicted as:

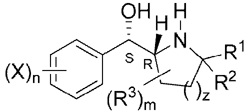

The skilled person will understand that where compounds of the invention are referred to as having specific stereochemistry, that compound is provided in the substantial absence of other stereoisomers.

As used herein, references to the substantial absence of other stereoisomer will refer to the desired stereoisomers (e.g. in the case of compounds of formula IA, when the carbon (a) is in the (R) configuration) being present at a purity of at least 80% (e.g. at least 90%, such as at least 95%) relative to the opposite stereoisomer (e.g. in the case of compounds of formula I, when the carbon (b) is in the (*S*) configuration). Alternatively, in such instances, compounds may be indicated to be present in the substantial absence of the compound in the other configurations (i.e. for example, the (*S*) configuration), which may indicate that the compound in the relevant configuration is present in an enantiomeric excess (e.e.) or diastereomeric excess (d.e.) of at least 90% (such as at least 95%, at least 98% or, particularly, at least 99%, for example at least 99.9%).

For the avoidance of doubt, compounds referred to as having a specific stereochemistry at a defined position (e.g. in the case of compounds of formula I, the carbon (a) in the (*R*) or (*S*) configuration) may also have stereochemistry at one or more other positions, and so may exist as mixtures of enantiomers or diastereoisomers in relation to the stereochemistry at those positions.

The skilled person will understand that compounds of the invention are agonists of the β₂-adrenergic receptor. In particular embodiments, such compounds may be identified using techniques known to those skilled in the art, such as the assay described in Biological example 1 herein below, wherein an agonist may be identified as a compound showing activity of more than 25% (e.g. more than 50%, particularly more than 75%) of that of isoproterenol in the same assay.

The skilled person will also understand that compounds of the invention may act without (or with only a minimal effect in) inducing cAMP production. In particular embodiments, such compounds may be identified using techniques known to those skilled in the art, such as the assay described in Biological example 2 herein below, wherein a compound acting without (or with only a minimal effect in) inducing cAMP production may be identified as a compound showing activity of less than 75% (e.g. less than 50%, particularly less than 25%) of that of isoproterenol in the same assay.

### Medical Uses

As indicated herein, the compounds of the invention, and therefore compositions and kits comprising the same, are useful as pharmaceuticals.

Thus, according to a second aspect of the invention there is provided a compound of the first aspect of the invention, as hereinbefore defined (i.e. a compound as defined in the first aspect of the invention, including all embodiments and particular features thereof), for use as a pharmaceutical (or for use in medicine).

For the avoidance of doubt, references to compounds as defined in the first aspect of the invention will include references to compounds of formula I (including all embodiments thereof) and pharmaceutically acceptable salts thereof.

As indicated herein, the compounds of the invention may be of particular use in treating hyperglycaemia or a disorder characterized by hyperglycaemia.

Thus, in a third aspect of the invention, there is provided a compound of the first aspect of the invention, as hereinbefore defined, for use in the treatment of hyperglycaemia or a disorder characterized by hyperglycaemia.

In an alternative third aspect of the invention, there is provided the use of a compound of formula I, or a pharmaceutically acceptable salt thereof, in the manufacture of a medicament for use in the treatment of hyperglycaemia or a disorder characterized by hyperglycaemia.

In a further alternative third aspect of the invention, there is provided a method of treating hyperglycaemia or a disorder characterized by hyperglycaemia comprising administering to a patient in need thereof a therapeutically effective amount of a compound of formula I, or a pharmaceutically acceptable salt thereof.

For the avoidance of doubt, the term "hyperglycaemia" as used herein will be understood by those skilled in the art to refer to a condition wherein an excessive amount of glucose circulates in blood plasma of the subject experiencing the same. In particular, it may refer to a subject (e.g a human subject) having blood glucose levels higher than about 10.0 mmol/L (such as higher than about 11.1 mmol/L, e.g. higher than about 15 mmol/L), although it may also refer to a subject (e.g a human subject) having blood glucose levels higher than about 7 mmol/L for an extended period of time (e.g. for greater than 24 hours, such as for greater than 48 hours).

The skilled person will understand that references to the treatment of a particular condition (or, similarly, to treating that condition) take their normal meanings in the field of medicine. In particular, the terms may refer to achieving a reduction in the severity of one or more clinical symptom associated with the condition. For example, in the case of type 2 diabetes, the term may refer to achieving a reduction of blood glucose levels. In particular embodiments, in the case of treating hyperglycaemia or conditions characterised by hyperglycaemia, the term may refer to achieving a reduction of blood glucose levels (for example, to or below about 10.0 mmol/mL (e.g. to levels in the range of from about 4.0 mmol/L to about 10.0 mmol/L), such as to or below about 7.5 mmol/mL (e.g. to levels in the range of from about 4.0 mmol/L to about 7.5 mmol/L) or to or below about 6 mmol/mL (e.g. to levels in the range of from about 4.0 mmol/L to about 6.0 mmol/L)).

As used herein, references to patients will refer to a living subject being treated, including mammalian (e.g. human) patients. Thus, in particular embodiments of the first aspect of the invention, the treatment is in a mammal (e.g. a human).

As used herein, the term therapeutically effective amount will refer to an amount of a compound that confers a therapeutic effect on the treated patient. The effect may be objective (i.e. measurable by some test or marker) or subjective (i.e. the subject gives an indication of and/or feels an effect).

Although compounds of the first aspect of the invention may possess pharmacological activity as such, certain pharmaceutically-acceptable (e.g. "protected") derivatives of compounds of the invention may exist or be prepared which may not possess such activity, but may be administered parenterally or orally and thereafter be metabolised in the body to form compounds of the invention. Such compounds (which may possess some pharmacological activity, provided that such activity is appreciably lower than that of the active compounds to which they are metabolised) may therefore be described as "prodrugs" of compounds of the invention.

As used herein, references to prodrugs will include compounds that form a compound of the invention, in an experimentally-detectable amount, within a predetermined time, following enteral or parenteral administration (e.g. oral or parenteral administration). All prodrugs of the compounds of the first aspect of the invention are included within the scope of the invention.

For the avoidance of doubt, the compounds of the first aspect of the invention are useful because they possess pharmacological activity, and/or are metabolised in the body following oral or parenteral administration to form compounds that possess pharmacological activity. In particular, as described herein, compounds of the first aspect of the invention are useful in the treatment of hyperglycaemia or disorders characterized by hyperglycaemia (such as type 2 diabetes), which terms will be readily understood by one of skill in the art (as described herein).

In a particular embodiment, the treatment is of a disorder (which may also be referred to as a condition or disease) characterised by hyperglycaemia.

In particular embodiments, compounds of the invention (i.e. compounds of formula I, including all embodiments thereof) are for use in the treatment of type 2 diabetes (or useful in the manufacture of a medicament for such treatment, or useful in a method for such treatment, as described herein).

In particular embodiments of the first aspect of the invention, the disorder is type 2 diabetes, such as type 2 diabetes of a sub-type selected from the list consisting of maturity-onset diabetes in the young (MODY), ketosis-prone diabetes in adults, latent autoimmune diabetes of adults (LADA), and gestational diabetes.

In further particular embodiments, the treatment of type 2 diabetes is in a non-obese patient.

For the avoidance of doubt, the skilled person will understand that patients with a Body Mass Index (BMI) of greater than 30 are considered to be obese.

In particular embodiments, the treatment may be of hyperglycaemia in a patient who is at risk of developing type 2 diabetes, which condition may be defined as pre-diabetes. Thus, compounds of the invention may be useful in the prevention of type 2 diabetes (e.g. in a patient having pre-diabetes).

As used herein, the term prevention (and, similarly, preventing) includes references to the prophylaxis of the disease or disorder (and vice-versa). As such, references to prevention may also be references to prophylaxis, and vice versa. In particular, the term may refer to achieving a reduction in the likelihood of the patient (or healthy subject) developing the condition (for example, at least a 10% reduction, such as at least a 20%, 30% or 40% reduction, e.g. at least a 50% reduction).

In more particular embodiments, the type 2 diabetes is characterised by the patient displaying severe insulin resistance (SIR).

In further embodiments, the treatment may be of hyperglycaemia in a patient having type 1 diabetes. Thus, compounds of the invention may be useful in the treatment of hyperglycaemia in type 1 diabetes.

The skilled person will understand that compounds of the invention may be useful in treating hyperglycaemia in patients having impaired insulin production, such as in patients having cystic fibrosis. Thus, in further embodiments, the disorder characterized by hyperglycaemia is cystic fibrosis-related diabetes.

In particular embodiments that may be mentioned, the disorder characterised by hyperglycaemia is (or is characterized by) severe insulin resistance (SIR), which may be understood by those in the art to refer to disorders wherein typically the subject has normal, or in some cases increased, insulin production but significantly reduced insulin sensitivity. In particular instances, such patients may be non-obese (e.g. being of a healthy weight). Thus, in particular embodiments, such treatments are performed in patients who are not defined as being obese (e.g. in patients who are defined as being of a healthy weight).

For example, SIR may be identified in a patient based in said patient having fasting insulin >150 pmol/L and/or a peak insulin on glucose tolerance testing of >1,500 pmol/L, particularly in individuals with a BMI < 30kg/m² (which patient may otherwise have normal glucose tolerance).

More particularly, SIR may be characterised by the patient having no significant response to the presence of insulin, which may result from a defect (e.g. a genetic defect) in the function of the insulin receptor.

Particular disorders that may be characterised by SIR include: Rabson-Mendenhall syndrome, Donohue's syndrome (leprechaunism), Type A and Type B syndromes of insulin resistance, the HAIR-AN (hyperandrogenism, insulin resistance, and acanthosis nigricans) syndromes, pseudoacromegaly, and lipodystrophy.

More particular disorders that may be characterised by SIR include Donohue's syndrome and Type A syndrome of insulin resistance and, yet more particularly, Rabson-Mendenhall syndrome.

The skilled person will understand that treatment with compounds of the first aspect of the invention may further comprise (i.e. be combined with) further (i.e. additional/other) treatment(s) for the same condition. In particular, treatment with compounds of the invention may be combined with other means for the treatment of type 2 diabetes, such as treatment with one or more other therapeutic agent that is useful in the treatment of type 2 diabetes as known to those skilled in the art, such as therapies comprising requiring the patient to undergo a change of diet and/or undertake exercise regiments, and/or surgical procedures designed to promote weight loss (such as gastric band surgery).

In particular, treatment with compounds of the invention may be performed in combination with (e.g. in a patient who is also being treated with) one or more (e.g. one) additional compounds (i.e. therapeutic agents) that:
(i) are capable of reducing blood sugar levels; and/or
(ii) are insulin sensitizers; and/or
(iii) enhance insulin release,
all of which are described herein below.

In alternative embodiments, compounds of the first aspect of the invention (i.e. compounds of the invention) may be useful in the treatment of a non-alcoholic fatty liver disease (NAFLD).

Non-alcoholic fatty liver disease (NAFLD) is defined by excessive fat accumulation in the form of triglycerides (steatosis) in the liver (designated as an accumulation of greater than 5% of hepatocytes histologically). It is the most common liver disorder in developed countries (for example, affecting around 30% of US adults) and most patients are asymptomatic. If left untreated, the condition may progressively worsen and may ultimately lead to cirrhosis of the liver. NAFLD is particularly prevalent in obese patients, with around 80% thought to have the disease.

A sub-group of NAFLD patients (for example, between 2 and 5% of US adults) exhibit liver cell injury and inflammation in addition to excessive fat accumulation. This condition, designated as non-alcoholic steatohepatitis (NASH), is virtually indistinguishable histologically from alcoholic steatohepatitis. While the simple steatosis seen in NAFLD does not directly correlate with increased short-term morbidity or mortality, progression of this condition to NASH dramatically increases the risks of cirrhosis, liver failure and hepatocellular carcinoma. Indeed, NASH is now considered to be one of the main causes of cirrhosis (including cryptogenic cirrhosis) in the developed world.

The exact cause of NASH has yet to be elucidated, and it is almost certainly not the same in every patient. It is most closely related to insulin resistance, obesity, and the metabolic syndrome (which includes diseases related to diabetes mellitus type 2, insulin resistance, central (truncal) obesity, hyperlipidaemia, low high-density lipoprotein (HDL) cholesterol, hypertriglyceridemia, and hypertension). However, not all patients with these conditions have NASH, and not all patients with NASH suffer from one of these conditions. Nevertheless, given that NASH is a potentially fatal condition, leading to cirrhosis, liver failure and hepatocellular carcinoma, there exists a clear need for an effective treatment.

In particular embodiments, compounds of the invention (i.e. compounds of formula I, including all embodiments thereof) are for use in the treatment of a non-alcoholic fatty liver disease (or useful in the manufacture of a medicament for such treatment, or useful in a method for such treatment, as described herein).

The process by which the triglyceride fat accumulates in liver cells is called steatosis (i.e. hepatic steatosis). The skilled person will understand that the term "steatosis" encompasses the abnormal retention of fat (i.e. lipids) within a cell. Thus, in particular embodiments of the first aspect of the invention, the treatment or prevention is of a fatty liver disease which is characterized by steatosis.

During steatosis, excess lipids accumulate in vesicles that displace the cytoplasm of the cell. Over time, the vesicles can grow large enough to distort the nucleus, and the condition is known as macrovesicular steatosis. Otherwise, the condition may be referred to as microvesicular steatosis. Steatosis is largely harmless in mild cases; however, large accumulations of fat in the liver can cause significant health issues. Risk factors associated with steatosis include diabetes mellitus, protein malnutrition, hypertension, obesity, anoxia, sleep apnea and the presence of toxins within the cell.

As described herein, fatty liver disease is most commonly associated with alcohol or a metabolic syndrome (for example, diabetes, hypertension, obesity or dyslipidemia). Therefore, depending on the underlying cause, fatty liver disease may be diagnosed as alcohol-related fatty liver disease or non-alcoholic fatty liver disease (NAFLD).

Particular diseases or conditions that are associated with fatty liver disease that are not related to alcohol include metabolic conditions such as diabetes, hypertension, obesity, dyslipidemia, abetalipoproteinemia, glycogen storage diseases, Weber-Christian disease, acute fatty liver of pregnancy, and lipodystrophy. Other non-alcohol related factors related to fatty liver diseases include malnutrition, total parenteral nutrition, severe weight loss, refeeding syndrome, jejunoileal bypass, gastric bypass, polycystic ovary syndrome and diverticulosis.

The compounds of the invention have been found to be particularly useful in the treatment or prevention of NAFLD, which may be referred to as a fatty liver disease which is not alcohol related. A fatty liver disease which is "not alcohol related" may be diagnosed wherein alcohol consumption of the patient is not considered to be a main causative factor. A typical threshold for diagnosing a fatty liver disease as "not alcohol related" is a daily consumption of less than 20 g for female subjects and less than 30 g for male subjects.

If left untreated, subjects suffering from fatty liver disease may begin to experience inflammation of the the liver (hepatitis). It has been postulated that one of the possible causes of this inflammation may be lipid peroxidative damage to the membranes of the liver cells. Inflammation of a fatty liver can lead to a number of serious conditions and it is therefore desirable to treat or prevent fatty liver disease before inflammation occurs. Thus, in particular embodiments of the first aspect of the invention, the treatment or prevention is of a NAFLD which is associated with inflammation.

Non-alcoholic steatohepatitis (NASH) is the most aggressive form of NAFLD, and is a condition in which excessive fat accumulation (steatosis) is accompanied by inflammation of the liver. If advanced, NASH can lead to the development of scar tissue in the liver (fibrosis) and, eventiually, cirrhosis. As described above, the compounds of the invention have been found to be useful in the treatment or prevention of NAFLD, particularly when accompanied by inflamation of the liver. It follows that the compounds of the invention are also useful in the treatment or prevention of NASH. Therefore, in a further embodiment of the first aspect of the invention, the treatment or prevention is of non-alcoholic steatohepatitis (NASH).

The skilled person will understand that treatment with compounds of the first aspect of the invention may further comprise (i.e. be combined with) further (i.e. additional/other) treatment(s) for the same condition. In particular, treatment with compounds of the invention may be combined with other means for the treatment of a fatty liver disease, as described herein, such as treatment with one or more other therapeutic agent that is useful in the treatment of a fatty liver disease as known to those skilled in the art; for example, therapies comprising requiring the patient to undergo a change of diet and/or undertake exercise regiments, and/or surgical procedures designed to promote weight loss (such as gastric band surgery).

In particular, treatment with compounds of the invention may be performed in combination with (e.g. in a patient who is also being treated with) one or more (e.g. one) additional compounds (i.e. therapeutic agents) that are capable of reducing the level of fat (e.g. triglycerides) in the liver.

References to treatment of a fatty liver disease may refer to achieving a therapeutically significant reduction of fat (e.g. triglycerides levels) in liver cells (such as a reduction of at least 5% by weight, e.g. a reduction of at least 10%, or at least 20% or even 25%).

### Pharmaceutical compositions

As described herein, compounds of the first aspect of the invention are useful as pharmaceuticals. Such compounds may be administered alone or may be administered by way of known pharmaceutical compositions/formulations.

In a fourth aspect of the invention, there is provided a pharmaceutical composition comprising a compound as defined in the first aspect of the invention (i.e. a compound of the invention), and optionally one or more pharmaceutically acceptable adjuvant, diluent and/or carrier, with the proviso that the compound is not a compound (1) - (36) selected from the list mentioned at pages 13 - 14 of the present description.

The skilled person will understand that references herein to compounds of the first aspect of the invention being for particular uses (and, similarly, to uses and methods of use relating to compounds of the invention) may also apply to pharmaceutical compositions comprising compounds of the invention as described herein.

In a fifth aspect of the invention, there is provided a pharmaceutical composition for use in the treatment of hyperglycaemia or a disoder characterized by hyperglycaemia (as defined herein, such as type 2 diabetes) comprising a compound as defined in the first aspect of the invention, and optionally one or more pharmaceutically acceptable adjuvant, diluent and/or carrier.

In an alternative fifth aspect of the invention, there is provided a pharmaceutical composition for use in the treatment or prevention of a non-alcoholic fatty liver disease, as defined herein.

The skilled person will understand that compounds of the first (and, therefore, second and third) aspect of the invention may act systemically and/or locally (i.e. at a particular site). The skilled person will understand that compounds and compositions as described in the first to fifth aspects of the invention will normally be administered orally, intravenously, subcutaneously, buccally, rectally, dermally, nasally, tracheally, bronchially, sublingually, intranasally, topically, by any other parenteral route or *via* inhalation, in a pharmaceutically acceptable dosage form. Pharmaceutical compositions as described herein will include compositions in the form of tablets, capsules or elixirs for oral administration, suppositories for rectal administration, sterile solutions or suspensions for parenteral or intramuscular administration, and the like. Alternatively, particularly where such compounds of the invention act locally, pharmaceutical compositions may be formulated for topical administration.

Thus, in particular embodiments of the fourth and fifth aspects of the invention, the pharmaceutical formulation is provided in a pharmaceutically acceptable dosage form, including tablets or capsules, liquid forms to be taken orally or by injection, suppositories, creams, gels, foams, inhalants (e.g. to be applied intranasally), or forms suitable for topical administration. For the avoidance of doubt, in such embodiments, compounds of the invention may be present as a solid (e.g. a solid dispersion), liquid (e.g. in solution) or in other forms, such as in the form of micelles.

For example, in the preparation of pharmaceutical formulations for oral administration, the compound may be mixed with solid, powdered ingredients such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or compressed into tablets.

Soft gelatin capsules may be prepared with capsules containing one or more active compounds (e.g. compounds of the first and, therefore, second and third aspects of the invention, and optionally additional therapeutic agents), together with, for example, vegetable oil, fat, or other suitable vehicle for soft gelatin capsules. Similarly, hard gelatine capsules may contain such compound(s) in combination with solid powdered ingredients such as lactose, saccharose, sorbitol, mannitol, potato starch, corn starch, amylopectin, cellulose derivatives or gelatin.

Dosage units for rectal administration may be prepared (i) in the form of suppositories which contain the compound(s) mixed with a neutral fat base; (ii) in the form of a gelatin rectal capsule which contains the active substance in a mixture with a vegetable oil, paraffin oil, or other suitable vehicle for gelatin rectal capsules; (iii) in the form of a ready-made micro enema; or (iv) in the form of a dry micro enema formulation to be reconstituted in a suitable solvent just prior to administration.

Liquid preparations for oral administration may be prepared in the form of syrups or suspensions, e.g. solutions or suspensions, containing the compound(s) and the remainder of the formulation consisting of sugar or sugar alcohols, and a mixture of ethanol, water, glycerol, propylene glycol and polyethylene glycol. If desired, such liquid preparations may contain colouring agents, flavouring agents, saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of the compound(s) in a pharmaceutically acceptable solvent. These solutions may also contain stabilizing ingredients and/or buffering ingredients and are dispensed into unit doses in the form of ampoules or vials. Solutions for parenteral administration may also be prepared as a dry preparation to be reconstituted with a suitable solvent extemporaneously before use.

The skilled person will understand that compounds of the invention, and pharmaceutically-acceptable salts thereof, may be administered (for example, as formulations as described hereinabove) at varying doses, with suitable doses being readily determined by one of skill in the art. Oral, pulmonary and topical dosages (and subcutaneous dosages, although these dosages may be relatively lower) may range from between about 0.01 µg/kg of body weight per day (µg/kg/day) to about 200 µg/kg/day, preferably about 0.01 to about 10 µg/kg/day, and more preferably about 0.1 to about 5.0 µg/kg/day. For example, when administered orally, treatment with such compounds may comprise administration of a formulations typically containing between about 0.01 µg to about 2000 mg, for example between about 0.1 µg to about 500 mg, or between 1 µg to about 100 mg (e.g. about 20 µg to about 80 mg), of the active ingredient(s). When administered intravenously, the most preferred doses will range from about 0.001 to about 10 µg/kg/hour during constant rate infusion. Advantageously, treatment may comprise administration of such compounds and compositions in a single daily dose, or the total daily dosage may be administered in divided doses of two, three or four times daily (e.g. twice daily with reference to the doses described herein, such as a dose of 10 mg, 20 mg, 30 mg or 40 mg twice daily, or 10 µg, 20 µg, 30 µg or 40 µg twice daily).

In any event, the skilled person (e.g. the physician) will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

As described herein above, the skilled person will understand that treatment with compounds of the first aspect of the invention may further comprise (i.e. be combined with) further (i.e. additional/other) treatment(s) for the same condition. In particular, treatment with compounds of the invention may be combined with other means for the treatment of hyperglycaemia or a disoder characterized by hyperglycaemia(as defined herein, such as type 2 diabetes), such as treatment with one or more other therapeutic agent that is useful in the treatment of hyperglycaemia or a disoder characterized by hyperglycaemia(as defined herein, such as type 2 diabetes).

In particular embodiments of the fourth and fifth aspects of the invention, the pharmaceutical composition may further comprise one or more additional (i.e. other) therapeutic agent.

In more particular embodiments, the one or more additional therapeutic agent is an agent for the treatment of type 2 diabetes as known to those skilled in the art, such as metformin, sulfonylureas (e.g. carbutamide, acetohexamide, chlorpropamide, tolbutamide. glipizide (glucotrol), gliclazide, glibenclamide, glyburide (Micronase), glibornuride, gliquidone, glisoxepide, glyclopyramide, glimepiride (Amaryl), glimiprime, JB253 or JB558), thiazolidinediones (e.g. pioglitazone, rosiglitazone (Avandia), lobeglitazone (Duvie) and troglitazone (Rezulin)), dipeptidyl peptidase-4 inhibitors (e.g. sitagliptin, vildagliptin, saxagliptin, linagliptin, anagliptin, teneligliptin, alogliptin, trelagliptin, gemigliptin, dutogliptin and omarigliptin), SGLT2 inhibitors (e.g. dapagliflozin, empagliflozin, canagliflozin, ipragliflozin, tofogliflozin, sergliflozin etabonate, remogliflozin etabonate, and ertugliflozin), and glucagon-like peptide-1 (GLP-1) analogues (e.g. exenatide, liraglutide, lixisenatide, albiglutide, dulaglutide and semaglutide).

The skilled person will understand that combinations of therapeutic agents may also described as a combination product and/or provided as a kit-of-parts.

In a sixth aspect of the invention, there is provided a combination product comprising:
(A) a compound as defined in the first aspect of the invention; and
(B) one or more additional therapeutic agent,
wherein each of components (A) and (B) is formulated in admixture, optionally with one or more a pharmaceutically-acceptable adjuvant, diluent or carrier.

In a seventh aspect of the invention, there is provided a kit-of-parts comprising:
(a) a compound as defined in the first (or second and/or third) aspect of the invention, (or a pharmaceutical composition comprising the same) or a pharmaceutical composition as defined in the fourth or fifth aspect of the invention; and
(b) one or more other therapeutic agent, optionally in admixture with one or more pharmaceutically-acceptable adjuvant, diluent or carrier,
which components (a) and (b) are each provided in a form that is suitable for administration in conjunction with the other.

In particular embodiments (e.g. of the sixth and seventh aspects of the invention), the additional therapeutic agent is a therapeutic agent that is useful for the treatment of hyperglycaemia or a disorder characterized by hyperglycaemia (e.g. type 2 diabetes), as known to those skilled in the art (such as those described herein).

For example, in particular embodiments of the fourth to fifth aspects of the invention, the additional therapeutic agent is an agent that:
(i) is capable of reducing blood sugar levels; and/or
(ii) is an insulin sensitizer; and/or
(iii) is able to enhance insulin release,
which agents will be readily identified by those skilled in the art and include, in particular, such therapeutic agents that are commercially available (e.g. agents that the subject of a marketing authorization in one or more territory, such as a European or US marketing authorization).

The skilled person will understand that references to therapeutic agents capable of reducing blood glucose levels may refer to compounds capable of reducing levels of blood by at least 10% (such as at least 20%, at least 30% or at least 40%, for example at least 50%, at least 60%, at least 70% or at least 80%, e.g. at least 90%) when compared to the blood glucose levels prior to treatment with the relevant compound.

In alternative embodiments of the sixth and seventh aspects of the invention, the additional therapeutic agent is an agent for the treatment or prevention of a non-alcoholic fatty liver disease (such as NASH), which agents will be readily identified by those skilled in the art and include, in particular, such therapeutic agents that are commercially available (e.g. agents that the subject of a marketing authorization in one or more territory, such as a European or US marketing authorization).

### Preparation of compounds/compositions

Pharmaceutical compositions/formulations, combination products and kits as described herein may be prepared in accordance with standard and/or accepted pharmaceutical practice.

Thus, in a further aspect of the invention there is provided a process for the preparation of a pharmaceutical composition/formulation, as hereinbefore defined, which process comprises bringing into association a compound of the invention, as hereinbefore defined, with one or more pharmaceutically-acceptable adjuvant, diluent or carrier.

In further aspects of the invention, there is provided a process for the preparation of a combination product or kit-of-parts as hereinbefore defined, which process comprises bringing into association a compound of the invention, as hereinbefore defined, or a pharmaceutically acceptable salt thereof with the other therapeutic agent that is useful in the treatment of hyperglycaemia or a disorder characterized by hyperglycaemia (e.g. type 2 diabetes), and at least one pharmaceutically- acceptable adjuvant, diluent or carrier.

As used herein, references to bringing into association will mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Compounds as defined in the first (and, therefore, second and third) aspect of the invention (i.e. compounds of the invention) may be prepared in accordance with techniques that are well known to those skilled in the art, such as those described in the examples provided hereinafter.

For example, there is provided a process for the preparation of a compound of formula I, or a pharmaceutically acceptable salt thereof, as defined in the first aspect of the invention (which may be utilised in the preparation of, for example, a compound as defined in the second aspect of the invention), which process comprises:
(i) reaction of a compound of formula II 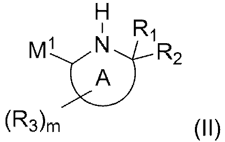
   wherein ring A, R¹, R², R³ and m are as defined herein, and wherein M¹ represents a suitable metal or metal halide, with a compound of formula III 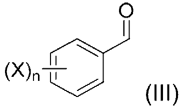
   wherein n and X are as defined herein, under conditions known to those skilled in the art;
(ii) reaction of a compound of formula IV 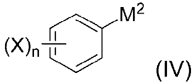
   wherein n and X are as defined herein, and wherein M² represents a suitable metal or metal halide, with a compound of formula V 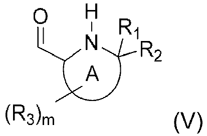
   wherein ring A, R¹, R², R³ and m are as defined herein, under conditions known to those skilled in the art;
(iii) for compounds wherein at least one X is present and represents -OH, deprotection of a compound of formula VI 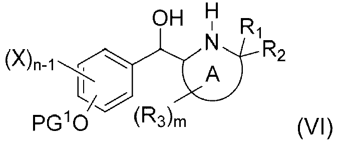 wherein ring A, R¹, R², R³, n and m are as herein, and PG¹ represents a suitable protecting group as known to those skilled in the art, under conditions known to those skilled in the art;
(iv) for compounds wherein at least one X is present and represents NH₂, deprotection of a compound of formula VII 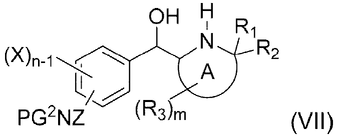 wherein ring A, R¹, R², R³, n and m are as defined herein, and Z represents H or PG³, wherein PG² and PG³ each represents a suitable protecting group as known to those skilled in the art, under conditions known to those skilled in the art;
(v) for compounds wherein at least one X is present and represents NH₂, reduction of a compound of formula VIII 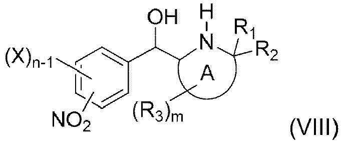 wherein ring A, R¹, R², R³, n, and m are as defined herein, under conditions known to those skilled in the art;
(vi) deprotection of a compound of formula IX 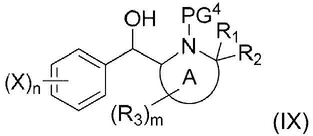 wherein ring A, X, R¹, R², R³, n and m are as defined herein, and PG⁴ represents a suitable protecting group as known to those skilled in the art, under conditions known to those skilled in the art; or
(vii) reduction of a compound of formula X 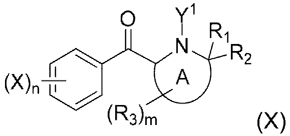 wherein ring A, X, R¹, R²,R³, n and m are as defined herein and Y¹ represents H or PG⁵ wherein PG⁵ is a suitable protecting group as known to those skilled in the art, in the presence of a suitable catalyst (such as for a compounds having a stereocentre at the carbon bearing the essential OH group, e.g. compounds of formulas IA¹⁻⁴ , a suitable catalyst may be a complex between (1*S*, 2*S*)-(+)-*N*-(4-toluenesulphonyl)-1,2-diphenylethylene diamine and [Ru(cymene)Cl₂]₂)) in the presence of hydrogen or a suitable hydrogen donor (such as formic acid) and optionally in the presence of a base (e.g. Et₃N) and in the presence of a suitable solvent (such as CH₂Cl₂).

Compounds of formulae II, III, IV, V, VI, VII, VIII, IX and X are either commercially available, are known in the literature, or may be obtained either by analogy with the processes described herein, or by conventional synthetic procedures, in accordance with standard techniques, from available starting materials (e.g. appropriately substituted benzaldehydes, styrenes or phenacyl bromides (or phenacylchloride, and the like) using appropriate reagents and reaction conditions. In this respect, the skilled person may refer to *inter alia "*Comprehensive Organic Synthesis" by B. M. Trost and I. Fleming, Pergamon Press, 1991. Further references that may be employed include *"*Science of Synthesis", Volumes 9-17 (Hetarenes and Related Ring Systems), Georg Thieme Verlag, 2006.

The substituents X and R¹, as hereinbefore defined, may be modified one or more times, after or during the processes described above for preparation of compounds of formula I by way of methods that are well known to those skilled in the art. Examples of such methods include substitutions, reductions, oxidations, dehydrogenations, alkylations, dealkylations, acylations, hydrolyses, esterifications, etherifications, halogenations and nitrations. The precursor groups can be changed to a different such group, or to the groups defined in formula I, at any time during the reaction sequence. The skilled person may also refer to *"*Comprehensive Organic Functional Group Transformations" by A. R. Katritzky, O. Meth-Cohn and C. W. Rees, Pergamon Press, 1995 and/or *"*Comprehensive Organic Transformations" by R. C. Larock, Wiley-VCH, 1999.

Such compounds may be isolated from their reaction mixtures and, if necessary, purified using conventional techniques as known to those skilled in the art. Thus, processes for preparation of compounds of the invention as described herein may include, as a final step, isolation and optionally purification of the compound of the invention (e.g. isolation and optionally purification of the compound of formula I).

The skilled person will understand that compounds of formula I having specific stereochemistry may be provided by reacting suitable starting materials having the required stereochemistry in processes as described herein. Further, the skilled person will understand that suitable starting materials having the required stereochemistry may be prepared by analogy with the processes described herein.

It will be appreciated by those skilled in the art that, in the processes described above and hereinafter, the functional groups of intermediate compounds may need to be protected by protecting groups. The protection and deprotection of functional groups may take place before or after a reaction in the above-mentioned schemes.

Protecting groups may be applied and removed in accordance with techniques that are well known to those skilled in the art and as described hereinafter. For example, protected compounds/intermediates described herein may be converted chemically to unprotected compounds using standard deprotection techniques. The type of chemistry involved will dictate the need, and type, of protecting groups as well as the sequence for accomplishing the synthesis. The use of protecting groups is fully described in *"*Protective Groups in Organic Synthesis", 3rd edition, T.W. Greene & P.G.M. Wutz, Wiley-Interscience (1999).

Compounds as described herein (in particular, compounds as defined in the first and, therefore, second and third aspects of the invention) may have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise. In particular, such compounds may have the advantage that they are more efficacious and/or exhibit advantageous properties *in vivo.*

Without wishing to be bound by theory, compounds as described herein are thought to be potent agonists of the β₂-adrenergic receptor, which allows for increased glucose uptake in skeletal muscle cells.

In addition, compounds as described herein are thought to be agonists of the β₂-adrenergic receptor without (or with only a minimal effect in) inducing cAMP production. It is thought that this allows for the increased glucose uptake in skeletal muscle cells with lower levels of side effects than would result from other treatments. Further, combining compounds as described herein with therapeutic agents that are able to decrease blood glucose levels is thought to provide an effective combination therapy.

### Examples

The present invention is illustrated by way of the following examples.

Chemicals and reagents were obtained from commercial suppliers and were used as received unless otherwise stated. All reactions involving moisture sensitive reagents were performed in oven or flame dried glassware under a positive pressure of nitrogen or argon.

### Abbreviations

Abbreviations as used herein will be known to those skilled in the art. In particular, the following abbreviations may be used herein.
- AcOH: acetic acid
- aq: aqueous
- Boc: *tert*-butoxycarbonyl
- Boc₂O: di-*tert*-butyldicarbonate
- DMF: dimethylformamide
- DMSO: dimethylsulfoxide
- EtOAc: ethyl acetate
- iPrOH: isopropanol
- MeCN: acetonitrile
- MeOH: methanol
- Pd-C: palladium on carbon
- rt: room temperature
- sat: saturated
- TBDMS: *tert*-butyldimethylsilyl
- THF: tetrahydrofuran

### Example compounds

In the event that there is a discrepancy between nomenclature and the structure of compounds as depicted graphically, it is the latter that presides (unless contradicted by any experimental details that may be given and/or unless it is clear from the context).

### Example 1: (R)-((R)-6,6-Dimethylpiperidin-2-yl)(3-fluorophenyl)methanol

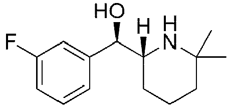

### (a) Methyl (R)-1-benzyl-6-oxopiperidine-2-carboxylate

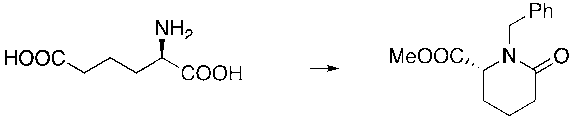

Chlorotrimethylsilane (8.3 mL, 65 mmol) was added dropwise to an ice-cooled solution of (R)-2-aminoadipic acid (3.0 g, 18.6 mmol) in MeOH (35 mL). The mixture was stirred at rt for 18 h and concentrated. The residue was dissolved in CH₂Cl₂ (30 mL). Benzaldehyde (2.08 mL, 20.5 mmol), triethyl amine (3.37 mL, 24.2 mmol) and MgSO₄ (1.12g, 9.3 mmol) were added at 0 °C and the mixture was stirred at rt for 4 h and concentrated. Et₂O (50 mL) was added and the mixture was filtered. The filtrate was concentrated and dissolved in MeOH (40 mL). NaBH₄ (1.27 g, 33.5 mmol) was added in portions at 0 °C and the mixture was stirred at rt for 3 h and concentrated. H₂O was added and the mixture was extracted with CH₂Cl₂. The combined extracts were washed with brine, dried (Na₂SO₄) and concentrated. EtOH (7 mL) and AcOH (3 drops) were added and the mixture was heated at 80 °C for 24 h and concentrated. The residue was purified by chromatography to give the sub-title product (2.9 g, 63%).

### (b) (R)-1-Benzyl-6-(hydroxymethyl)piperidin-2-one

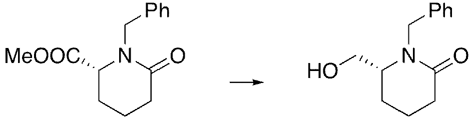

Lithium triethylborohydride (1.7 M in THF, 6.3 mL, 10.7 mmol) was added to methyl (R)-1-benzyl-6-oxopiperidine-2-carboxylate (1.2 g, 4.85 mmol) in THF (40 mL) at 0 °C and the mixture was stirred at 0 °C for 1 h. The reaction was quenched with ice-water, and the aqueous phase was extracted with CH₂Cl₂. The combined organic phases were dried (Na₂SO₄) and concentrated. The residue was purified by chromatography to give the sub-title product (1.0 g, 94%).

### (c) (R)-1-Benzyl-6-(((tert-butyldimethylsilyl)oxy)methyl)piperidin-2-one

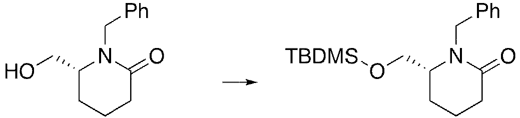

Imidazole (456 mg, 6.70 mmol) and tert-butyldimethylsilylchloride (808 mg, 5.36 mmol) were added to a mixture of (*R*)-1-benzyl-6-(hydroxymethyl)piperidin-2-one (980 mg, 4.47 mmol) and DMF (8 mL) at rt. The mixture was stirred at rt overnight and Et₂O and H₂O were added. The layers were separated and the aq phase was extracted with Et₂O. The combined extracts were washed with brine, dried (MgSO₄) and concentrated. The residue was purified by chromatography to give the sub-title product (1.20 g, 81 %).

### (d) (R)-1-benzyl-6-(((tert-butyldimethylsilyl)oxy)methyl)-2,2-dimethylpiperidine

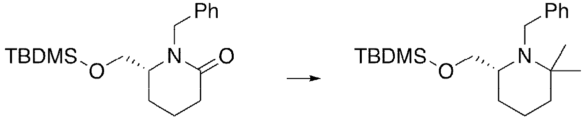

Freshly distilled triflic anhydride (531 µl, 3.24 mmol) was added dropwise to a mixture of (*R*)-1-benzyl-6-(((*tert*-butyldimethylsilyl)oxy)methyl)piperidin-2-one (900 mg, 2.7 mmol), 2,6-di-tert-butyl-4-methylpyridine (665 mg, 3.24 mmol) and CH₂Cl₂ (20 mL) at -78 °C. The mixture was stirred at -78 °C for 1 h and methylmagnesium bromide (3 M in Et₂O, 2.7 mL, 8.1 mmol) was added dropwise. The stirred mixture was slowly allowed to reach rt over 3 h, quenched with NH₄Cl (aq, sat, 10 mL) and extracted with CH₂Cl₂. The combined organic phases were dried (Na₂SO₄) and concentrated. The residue was purified by chromatography to give the sub-title product (731 mg, 78 %).

### (e) (R)-(1-Benzyl-6,6-dimethylpiperidin-2-yl)methanol

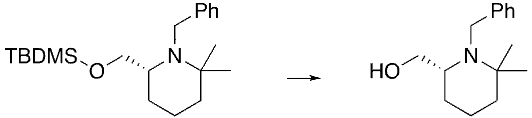

Tetrabutylammonium fluoride (1 M in THF, 1.15 mL, 1.15 mmol) was added to a solution of (*R*)-1-benzyl-6-(((*tert*-butyldimethylsilyl)oxy)methyl)-2,2-dimethylpiperidine (200 mg, 0.58 mmol) in THF (8 mL) at rt. The mixture was stirred at rt overnight, diluted with H₂O and extracted with EtOAc. The combined extracts were washed with H₂O, brine, dried (Na₂SO₄) and concentrated. The residue was purified by chromatography to give the sub-title product (105 mg, 78%).

### (f) (R)-1-Benzyl-6,6-dimethylpiperidine-2-carbaldehyde

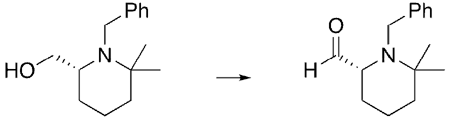

A solution of DMSO (95 µL, 1.14 mmol) in CH₂Cl₂ (1 mL) was added dropwise to oxalyl chloride (56 µL, 0.64 mmol) in CH₂Cl₂ (1 mL) at -78 °C. After 30 min at -78 °C a solution of (*R*)-(1-benzyl-6,6-dimethylpiperidin-2-yl)methanol (125 mg, 0.54 mmol) in CH₂Cl₂ (1.2 mL) was added. After 30 min at -78 °C, triethylamine (373 µL, 2.68 mmol) was added dropwise. After 10 min at -78 °C, the flask was placed in an ice-water bath and stirred for 1 h, allowed to warm-up to rt and stirred at rt for 30 min. H₂O was added and the phases separated. The aq phase was washed with CH₂Cl₂ and the combined extracts were dried (MgSO₄) and concentrated to give the sub-title product (120 mg, 97%), which was used in the next step without purification.

### (g) (R)-((R)-1-Benzyl-6,6-dimethylpiperidin-2-yl)(3-fluorophenyl)methanol and (S)-((R)-1-benzyl-6,6-dimethylpiperidin-2-yl)(3-fluorophenyl)methanol

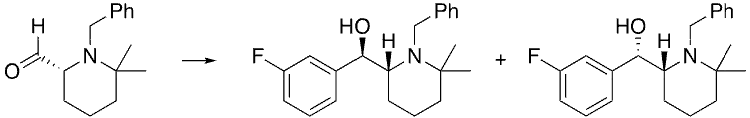

3-Fluorophenylmagnesium bromide (0.9 M in THF, 1.02 mL, 0.92 mmol), prepared from 1-bromo-3-fluorobenzene and Mg by microwave irradiation in THF for 20 min 80 °C, was added dropwise to a suspension of CeCl₃ (226 mg, 0.92 mmol) in THF (1 mL) at -78 °C. After 1 h at -78 °C, a solution of the (*R*)-1-benzyl-6,6-dimethylpiperidine-2-carbaldehyde (85 mg, 0.37 mmol) in THF (2 ml) was added dropwise. The temperature was allowed to reach rt over 4 h. NH₄Cl (aq, sat) was added and the mixture was extracted with CH₂Cl₂. The combined extracts were washed with brine, dried (Na₂SO₄) and concentrated. The residue was purified by chromatography to give (*R*)-((*R*)-1-benzyl-6,6-dimethylpiperidin-2-yl)(3-fluorophenyl)methanol (34 mg, 28%) and (*S*)-((*R*)-1-benzyl-6,6-dimethylpiperidin-2-yl)(3-fluorophenyl)methanol (67 mg, 56%).

### (h) (R)-((R)-6,6-Dimethylpiperidin-2-yl)(3-fluorophenyl)methanol

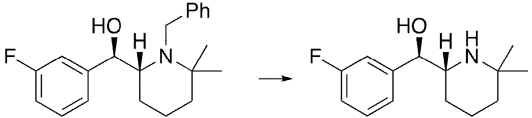

Pd-C (10%, 8.1 mg, 0.008 mmol) and ammonium formate (48 mg, 0.76 mmol) were added to a mixture of (*R*)-((*R*)-1-benzyl-6,6-dimethylpiperidin-2-yl)(3-fluorophenyl)methanol (25 mg, 0.076 mmol) in iPrOH (1 mL) and the mixture was stirred at 70 °C for 1 h. The mixture was filtered through Celite and the filtrate was concentrated. The residue was crystallized from Et₂O/pentane to give the title compound (10 mg, 55%).

¹H NMR (400 MHz, CDCl₃): δ 7.33-7.27 (m, 1H), 7.13 - 7.05 (m, 2H), 7.00 - 6.93 (m, 1H), 4.29 (d, J = 6.8 Hz, 1H), 2.84 (ddd, J = 11.6, 6.7, 2.8 Hz, 1H), 1.66 - 1.57 (m, 1H), 1.51 - 1.35 (m, 3H), 1.22 (td, J = 13.3, 4.2 Hz, 1H), 1.12 (s, 3H), 1.11 - 1.00 (m, 1H), 1.07 (s, 3H).

### Example 2: (S)-((R)-6,6-Dimethylpiperidin-2-yl)(3-fluorophenyl)methanol

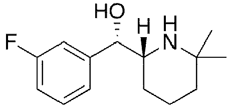

The title compound was isolated in accordance with the procedure in Example 1, Step (h) from (S)-((R)-1-benzyl-6,6-dimethylpiperidin-2-yl)(3-fluorophenyl)methanol, see Example 1, Step (g).

¹H NMR (400 MHz, CDCl₃): δ 7.36 - 7.23 (m, 1H), 7.11 - 7.06 (m, 2H), 6.98 - 6.91 (m, 1H), 4.59 (d, J = 4.0 Hz, 1H), 3.04 (ddd, J = 11.6, 4.0, 3.0 Hz, 1H), 1.62 - 1.52 (m, 1H), 1.49 - 1.35 (m, 2H), 1.29 - 1.15 (m, 2H), 1.13 (s, 3H), 1.12 (s, 3H), 1.11-1.00 (m, 1H)

### Example 3: 3-((R)-((R)-6,6-Dimethylpiperidin-2-yl)(hydroxy)methyl)phenol acetate

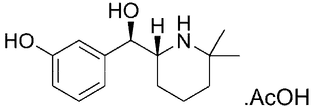

The title compound was prepared from (*R*)-1-benzyl-6,6-dimethylpiperidine-2-carbaldehyde and 3-benzyloxyphenylmagnesium bromide in accordance with the procedures in Example 1, Steps (g) and (h) and purification by reverse phase chromatography eluating with a gradient of 2% AcOH in MeCN to 100% MeCN.

¹H NMR (400 MHz, D₂O): δ 7.38 - 7.32 (m, 1H), 6.99 - 6.95 (m, 1H), 6.94 - 6.88 (m, 2H), 4.92 (d, J = 4.0 Hz, 1H), 3.60 (ddd, J = 12.4, 3.7, 2.7 Hz, 1H), 1.92 (s, 3H), 1.79 - 1.68 (m, 3H), 1.68 - 1.54 (m, 2H), 1.54 - 1.47 (m, 1H), 1.43 (s, 3H), 1.40 (s, 3H).

### Example 4: 3-((S)-((R)-6,6-Dimethylpiperidin-2-yl)(hydroxy)methyl)phenol acetate

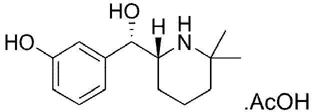

The title compound was prepared from (*S*)-1-benzyl-6,6-dimethylpiperidine-2-carbaldehyde and 3-benzyloxyphenylmagnesium bromide in accordance with the procedures in Example 1, Steps (g) and (h) and purification by reverse phase chromatography eluating with a gradient of 2% AcOH in MeCN to 100% MeCN.

¹H NMR (400 MHz, D₂O): δ 7.38 - 7.32 (m, 1H), 6.99 - 6.95 (m, 1H), 6.94 - 6.88 (m, 2H), 4.92 (d, J = 4.0 Hz, 1H), 3.60 (ddd, J = 12.4, 3.7, 2.7 Hz, 1H), 1.92 (s, 3H), 1.79 - 1.68 (m, 3H), 1.68 - 1.54 (m, 2H), 1.54 - 1.47 (m, 1H), 1.43 (s, 3H), 1.40 (s, 3H).

### Example 5: (R)-(2-Chlorophenyl)((R)-6,6-dimethylpiperidin-2-yl)methanol hydrochloride

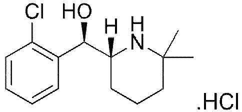

### (a) (R)-6-(((tert-Butyldimethylsilyl)oxy)methyl)-2,2-dimethylpiperidine

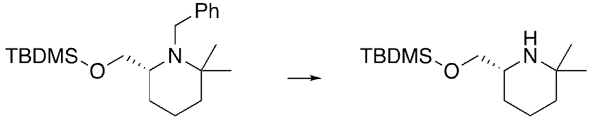

A mixture of (*R*)-1-benzyl-6-(((*tert*-butyldimethylsilyl)oxy)methyl)-2,2-dimethylpiperidine (1 g, 2.9 mmol) see Example 1, Step (d), Pd(OH)₂ on C (20 % wt, 2.0 g, 1.4 mmol) and freshly distilled EtOAc (25 mL) was hydrogenated at ambient temperature and pressure for 1 h and filtered through Celite. The solids were washed with EtOAc and the filtrates dried over Na₂SO₄ and concentrated to give the sub-title product (0.66 g, 89%) which was used in next step without further purification.

### (b) tert-Butyl (R)-6-(((tert-butyldimethylsilyl)oxy)methyl)-2,2-dimethylpiperidine-1-carboxylate

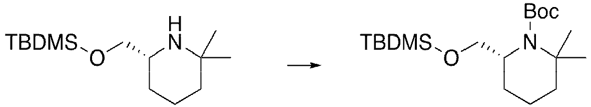

A mixture of (*R*)-6-((*tert*-butyldimethylsilyl)oxy)methyl)-2,2-dimethylpiperidine (0.66 g, 2.6 mmol) and Boc₂O (0.67 g, 3.1 mmol) was heated at 60 °C for 72 h, cooled and purified by chromatography to give the sub-title product (0.77 g, 84%).

### (c) tert-Butyl (R)-6-formyl-2,2-dimethylpiperidine-1-carboxylate

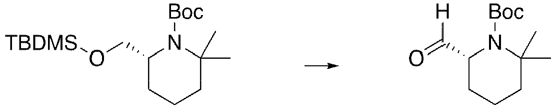

A mixture of *tert*-butyl (*R*)-6-(((*tert*-butyldimethylsilyl)oxy)methyl)-2,2-dimethylpiperidine-1-carboxylate (0.77 g, 2.2 mmol), NH₄F (1.6 g, 44 mmol) and anhydrous MeOH (50 mL) was stirred at 40 °C for 16 h, diluted with water and extracted with EtOAc. The combined extracts were washed with water and brine, dried over Na₂SO₄ and concentrated. The residue was dissolved in CH₂Cl₂ and Dess-Martin periodinane (0.96 g, 2.3 mmol) in CH₂Cl₂ (30 mL) was added via syringe. The mixture was stirred at rt for 1 h and NaOH (aq, 1 M, 30 mL) was added. The mixture was stirred vigorously for 10 min and the layers were separated. The aq phase was washed with CH₂Cl₂ and the combined organic phases were dried over Na₂SO₄ and concentrated. The residue was purified by chromatography to give the sub-title product (0.24 g, 49%).

### (d) tert-Butyl (R)-6-((R)-(2-chlorophenyl)(hydroxy)methyl)-2,2-dimethylpiperidine-1-carboxylate and tert-butyl (R)-6-((S)-(2-chlorophenyl)(hydroxy)methyl)-2,2-dimethyl-piperidine-1-carboxylate

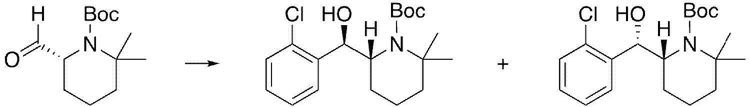

2-Chlorophenylmagnesium bromide (1 M in THF, 0.49 mL, 0.49 mmol, freshly prepared from 1-bromo-2-chlorobenzene and iPrMgCl.LiCl complex at 0 °C) was added dropwise to a solution of *tert*-butyl (*R*)-6-formyl-2,2-dimethylpiperidine-1-carboxylate (96 mg, 0.40 mmol) in THF (5 ml) at -20 °C. After 20 min, NH₄Cl (aq, sat) was added and the mixture was extracted with CH₂Cl₂. The combined extracts were washed with brine, dried (Na₂SO₄) and concentrated. The residue was purified by chromatography to give the sub-title product (65 mg, 46%). During chromatography the (*R*,*S*)-isomer was also isolated (60 mg, 43 %).

### (e) (R)-(2-Chlorophenyl)((R)-6,6-dimethylpiperidin-2-yl)methanol hydrochloride

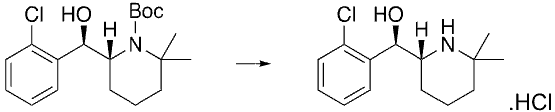

A mixture of *tert*-butyl (*R*)-6-((*R*)-(2-chlorophenyl)(hydroxy)methyl)-2,2-dimethylpiperidine-1-carboxylate (50 mg, 0.14 mmol), CeCl₃.7H₂O (79 mg, 0.21 mmol), NaI (28 mg, 0.18 mmol) and MeCN (1.5 mL) was stirred at 100 °C for 1 h and cooled to rt. EtOAc and NaOH (aq, 1 M) were added and the mixture was shaken until it became colorless. The layers were separated and the organic phase was washed with water, dried over Na₂SO₄ and concentrated. The residue was dissolved in dry Et₂O (5 mL) and HCl (2 M in Et₂O, 78 µL, 0.16 mmol) was added dropwise. The mixture was stirred at rt for 15 min and the solid was collected, washed with Et₂O followed by MeCN and dried to give the title compound (30 mg, 73%).

¹H NMR (400 MHz, CD₃OD): δ 7.69 - 7.64 (m, 1H), 7.46 - 7.39 (m, 2H), 7.38 - 7.31 (m, 1H), 5.23 (d, J = 9.3 Hz, 1H), 3.45 - 3.37 (m, 1H), 1.80 - 1.71 (m, 1H), 1.71 - 1.56 (m, 3H), 1.56 - 1.47 (m, 1H), 1.50 (s, 3H), 1.44 - 1.37 (m, 1H, overlapping), 1.43 (s, 3H)

### Example 6: (S)-(2-Chlorophenyl)((R)-6,6-dimethylpiperidin-2-yl)methanol hydrochloride

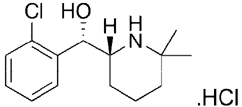

The title compound was prepared in accordance with the procedure in Example 5, Step (e) from *tert*-butyl (*R*)-6-((*R*)-(2-chlorophenyl)(hydroxy)methyl)-2,2-dimethylpiperidine-1-carboxylate isolated in Example 5, Step (d).

¹H NMR (400 MHz, CD₃OD): δ 7.69 (dd, *J* = 7.7, 1.8 Hz, 1H), 7.46 - 7.38 (m, 2H), 7.34 (td, *J* = 7.6, 1.8 Hz, 1H), 5.35 (d, *J* = 2.6 Hz, 1H), 3.63 (dt, *J* = 12.5, 2.7 Hz, 1H), 1.81 - 1.55 (m, 5H), 1.50 (s, 3H), 1.43 (s, 3H), 1.40 - 1.32 (m, 1H).

### Example 7: 0(R)-(2-Chlorophenyl)((R)-6,6-dimethylpiperidin-2-yl)methanol hydrochloride

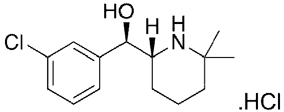

### (a) tert-Butyl (R)-6-((S)-(3-chlorophenyl)(hydroxy)methyl)-2,2-dimethylpiperidine-1-carboxylate and tert-butyl ((R)-(3-chlorophenyl)((R)-6,6-dimethylpiperidin-2-yl)methyl) carbonate

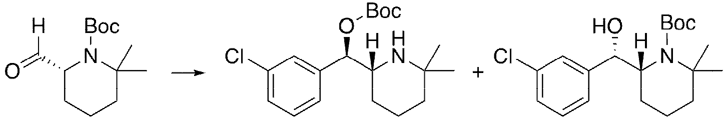

A mixture of the subtitle compounds was obtained in accordance with Example 5, Step (d) from *tert*-butyl (*R*)-6-formyl-2,2-dimethylpiperidine-1-carboxylate and 3-chlorophenylmagnesium bromide and were separated by chromatography.

### (b) (R)-(3-Chlorophenyl)((R)-6,6-dimethylpiperidin-2-yl)methanol hydrochloride

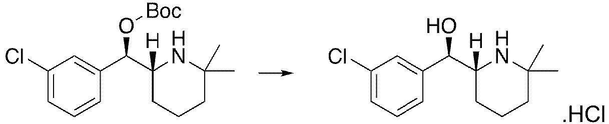

The title compound was prepared in accordance with Example 5, Step (e) from *tert*-butyl ((*R*)-(3-chlorophenyl)((*R*)-6,6-dimethylpiperidin-2-yl)methyl) carbonate.

¹H NMR (400 MHz, CDCl₃): δ 9.59 (br s, 1H), 8.09 (br s, 1H), 7.41 - 7.36 (m, 1H), 7.30 - 7.19 (m, 3H), 6.13 (dd, *J* = 8.8, 6.1 Hz, 1H), 5.20 - 5.05 (m, 1H), 3.40 - 3.23 (m, 1H), 1.97 (td, *J* = 13.8, 3.9 Hz, 1H), 1.79 - 1.61 (m, 5H), 1.61 - 1.44 (m, 2H), 1.41 (d, *J* = 4.2 Hz, 3H), 1.30 - 1.18 (m, 1H).

### Example 8: (S)-(2-Chlorophenyl)((R)-6,6-dimethylpiperidin-2-yl)methanol hydrochloride

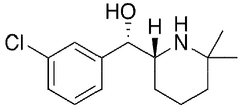

The title compound was prepared in accordance with Example 5, Step (e) from, *tert*-butyl (*R*)-6-((*S*)-(3-chlorophenyl)(hydroxy)methyl)-2,2-dimethylpiperidine-1-carboxylate see Example 7, Step (a).

¹H NMR (400 MHz, CDCl₃): δ 9.96 (br s, 1H), 7.90 (br s, 1H), 7.46 - 7.43 (m, 1H), 7.35 - 7.30 (m, 1H), 7.25 - 7.15 (m, 2H), 5.64 (br s, 1H), 5.49 (d, *J* = 2.2 Hz, 1H), 3.30 (t, *J* = 11.3 Hz, 1H), 1.91 - 1.77 (m, 1H), 1.77 - 1.66 (m, 2H), 1.62 (s, 3H), 1.62 - 1.55 (m, 1H), 1.54 - 1.40 (m, 5H).

### Example 9: (R)-((R)-6,6-Dimethylpiperidin-2-yl)(2-fluorophenyl)methanol hydrochloride

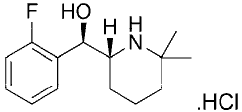

The title compound was prepared in accordance with Example 5, using 2-fluororophenyl-magnesium bromide in Step (d).

¹H NMR (400 MHz, CD₃OD): δ 7.3 - 7.57 (m, 1H), 7.44 - 7.36 (m, 1H), 7.31 - 7.25 (m, 1H), 7.17 - 7.09 (m, 1H), 4.98 (d, *J* = 9.4 Hz, 1H), 3.47 - 7.37 (m, 1H), 1.80 - 1.72 (m, 1H), 1.71 - 1.59 (m, 3H), 1.50 (s, 3H), 1.43 (s, 3H), 1.48 - 1.38 (m, 2H).

### Example 10: (S)-((R)-6,6-Dimethylpiperidin-2-yl)(2-fluorophenyl)methanol hydrochloride

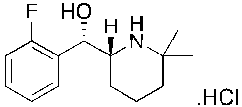

The title compound was prepared in accordance with Example 5, using 2-fluororophenyl-magnesium bromide in Step (d).

¹H NMR (400 MHz, CD₃OD): δ 7.66 - 7.59 (m, 1H), 7.42 - 7.34 (m, 1H), 7.30 - 7.23 (m, 1H), 7.17 - 7.09 (m, 1H), 5.32 (d, J = 2.8 Hz, 1H), 3.59 - 3.47 (m, 1H), 1.77 - 1.61 (m, 5H), 1.49 (s, 3H), 1.47 - 1.44 (m, 1H), 1.43 (s, 3H).

### Example 11: (R)-((R)-5,5-Dimethylpyrrolidin-2-yl)(3-fluorophenyl)methanol hydrochloride

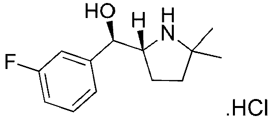

### (a) (R)-5-(Hydroxymethyl)pyrrolidin-2-one

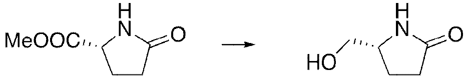

NaBH₄ (132 mg, 3.49 mmol) was added in portions to a mixture of methyl (*R*)-5-oxopyrrolidine-2-carboxylate (250 mg, 1.75 mmol) and MeOH (2 mL) at 0 °C. The mixture was stirred at 0 °C for 90 min and concentrated. The residue was purified by chromatography to give the sub-title compound (190 mg, 95 %).

### (b) (R)-5-(((tert-Butyldimethylsilyl)oxy)methyl)pyrrolidin-2-one

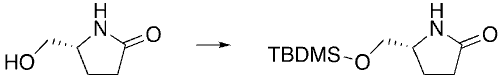

The sub-title compound was prepared in accordance with the procedure in Example 1, Step (c) but without the cromatographic purification and was used as such in the next step.

### (c) (R)-1-Benzyl-5-(((tert-butyldimethylsilyl)oxy)methyl)pyrrolidin-2-one

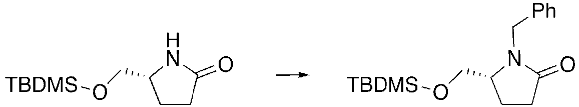

(*R*)-5-(((*tert*-Butyldimethylsilyl)oxy)methyl)pyrrolidin-2-one (1.24 g, 5.42 mmol) in THF (18.5 mL) was added dropwise to a mixture of NaH (60% dispersion in mineral oil, 325 mg, 8.13 mmol, washed with pentane) and THF (7 ml) at 0 °C. The mixture was stirred at 0 °C for 10 min and benzyl bromide (0.97 mL, 8.13 mmol) was added. The ice-bath was removed and the mixture was stirred at rt for 10 min and at reflux for 90 min. The reaction was carefully quenched with H₂O. EtOAc was added and the aq phase was extracted with EtOAc. The combined organic phases were washed with brine, dried (Na₂SO₄) and concentrated. The residue was purified by chromatography to give the sub-title compound (1.35 g, 78%).

### (d) ((R)-1-Benzyl-5,5-dimethylpyrrolidine-2-carbaldehyde

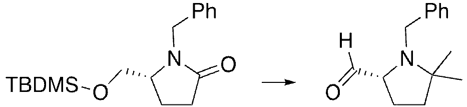

The sub-title compound was prepared in accordance with the procedures in Example 1, Steps (d) to (f).

### (e) (R)-((R)-1-Benzyl-5,5-dimethylpyrrolidin-2-yl)(3-fluorophenyl)methanol and (S)-((R)-1-benzyl-5,5-dimethylpyrrolidin-2-yl)(3-fluorophenyl)methanol

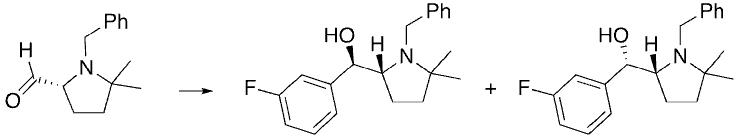

The sub-title compounds were prepared in accordance with the procedure in Example 1, Step (g) and were separated by chromatography.

### (f) (R)-((R)-5,5-Dimethylpyrrolidin-2-yl)(3-fluorophenyl)methanol hydrochloride

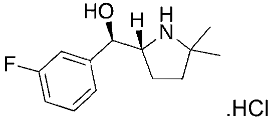

The title compound was prepared from (*R*)-((*R*)-1-benzyl-5,5-dimethylpyrrolidin-2-yl)(3-fluorophenyl)methanol in accordance with the procedures in Example 1, Step (h), followed by addition of HCl in Et₂O as described in Example 5, Step (e).

¹H NMR (400 MHz, D₂O): δ 7.54 - 7.44 (m, 1H), 7.33 - 7.23 (m, 2H), 7.23 - 7.15 (m, 1H), 4.88 (d, *J* = 8.5 Hz, 1H), 4.10 - 3.99 (m, 1H), 2.05 - 1.81 (m, 4H), 1.56 (s, 3H), 1.47 (s, 3H).

### Example 12: (S)-((R)-5,5-Dimethylpyrrolidin-2-yl)(3-fluorophenyl)methanol hydrochloride

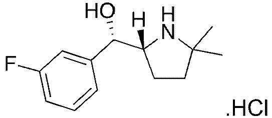

The title compound was prepared from (*S*)-((*R*)-1-benzyl-5,5-dimethylpyrrolidin-2-yl)(3-fluorophenyl)methanol in accordance with the procedures in Example 1, Step (h), followed by addition of HCl in Et₂O as described in Example 5, Step (e).

¹H NMR (400 MHz, CDCl₃): δ 7.32 - 7.22 (m, 1H), 7.16 - 7.06 (m, 2H), 6.97 - 6.86 (m, 1H), 4.72 (d, *J* = 3.5 Hz, 1H), 3.39 - 2.79 (br s, 2H), 1.80 - 1.65 (m, 1H), 1.65 - 1.45 (m, 2H), 1.45 - 1.30 (m, 2H), 1.26 (s, 3H), 1.23 (s, 3H).

### Example 13: (R)-((S)-5,5-Dimethylpyrrolidin-2-yl)(3-fluorophenyl)methanol hydrochloride

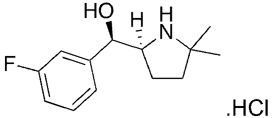

The title compound was prepared from (*S*)-1-benzyl-5,5-dimethylpyrrolidine-2-carbaldehyde (prepared from methyl (*S*)-5-oxopyrrolidine-2-carboxylate in accordance with the procedures in Example 11, Steps (a) to (d)) and 3-fluoromagnesium bromide in accordance with the procedures in Example 11, Steps (e) and (f).

¹H NMR (400 MHz, CDCl₃): δ 7.52 - 7.43 (m, 1H), 7.30 - 7.20 (m, 2H), 7.20 - 7.11 (m, 1H), 5.06 (d, *J* = 5.0 Hz, 1H), 4.15 - 4.07 (m, 1H), 2.18 - 2.06 (m, 1H), 2.06 - 1.88 (m, 3H), 1.49 (s, 3H), 1.43 (s, 3H).

### Example 14: (S)-((S)-5,5-Dimethylpyrrolidin-2-yl)(3-fluorophenyl)methanol hydrochloride

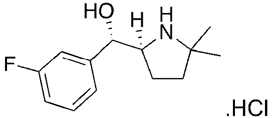

The title compound was prepared from (*S*)-1-benzyl-5,5-dimethylpyrrolidine-2-carbaldehyde (prepared from methyl (*S*)-5-oxopyrrolidine-2-carboxylate in accordance with the procedures in Example 11, Steps (a) to (d)) and 3-fluoromagnesium bromide in accordance with the procedures in Example 11, Steps (e) and (f).

¹H NMR (400 MHz, D₂O): δ 7.51 - 7.43 (m, 1H), 7.31 - 7.21 (m, 2H), 7.21 - 7.14 (m, 1H), 4.86 (d, *J* = 8.5 Hz, 1H), 4.10 - 3.99 (m, 1H), 2.05 - 1.81 (m, 4H), 1.53 (s, 3H), 1.45 (s, 3H).

### Example 15: (R)-((R)-5,5-Dimethylpyrrolidin-2-yl)(hydroxy)methyl)phenol acetate

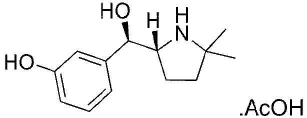

The title compound was prepared from (*R*)-1-benzyl-5-oxopyrrolidine-2-carbaldehyde (see Example 11, Step (d)) and 3-benzyloxymagnesium bromide in accordance with the procedures in Example 11, Steps (e) and (f) and purification by reverse phase chromatography eluating with a gradient of 2% AcOH in MeCN to 100% MeCN.

¹H NMR (400 MHz, D₂O): δ 7.41 - 7.30 (m, 1H), 7.07 - 6.99 (m, 1H), 6.99 - 6.88 (m, 2H), 4.86 - 4.73 (m, 1H, overlapping), 2.07 - 1.77 (m, 7H), 1.53 (s, 3H), 1.45 (s, 3H).

### Example 16: (S)-((R)-5,5-Dimethylpyrrolidin-2-yl)(hydroxy)methyl)phenol acetate

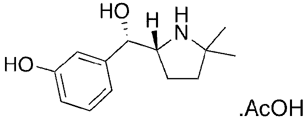

The title compound was prepared from (*R*)-1-benzyl-5-oxopyrrolidine-2-carbaldehyde (see Example 11, Step (d)) and 3-benzyloxymagnesium bromide in accordance with the procedures in Example 11, Steps (e) and (f) and purification by reverse phase chromatography eluating with a gradient of 2% AcOH in MeCN to 100% MeCN.

¹H NMR (400 MHz, D₂O): δ 7.22 - 7.15 (m, 1H), 6.87 - 6.81 (m, 1H), 6.81 - 6.76 (m, 1H), 6.76 - 6.71 (m, 1H), 4.81 (d, *J* = 5.1 Hz, 1H), 3.96 - 3.87 (m, 1H), 2.00 - 1.80 (m, 2H), 1.80 - 1.73 (m, 5H), 1.31 (s, 3H), 1.26 (s, 3H).

### Example 17: (R)-((S)-5,5-Dimethylpyrrolidin-2-yl)(hydroxy)methyl)phenol

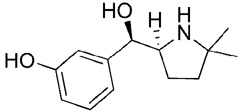

The title compound was prepared from (*S*)-1-benzyl-5,5-dimethylpyrrolidine-2-carbaldehyde (prepared from methyl (*S*)-5-oxopyrrolidine-2-carboxylate in accordance with the procedures in Example 11, Steps (a) to (d)) and 3-benzyloxymagnesium bromide in accordance with the procedures in Example 11, Steps (e) and (f).

¹H NMR (400 MHz, CD₃OD): δ 7.18 - 7.11 (m, 1H), 6.87 - 6.80 (m, 2H), 6.70 - 6.64 (m, 1H), 4.65 (d, *J* = 4.6 Hz, 1H), 3.44 - 3.37 (m, 1H), 1.93 - 1.81 (m, 1H), 1.70 - 1.51 (m, 3H), 1.25 (s, 3H).

### Example 18: (S)-((S)-5,5-Dimethylpyrrolidin-2-yl)(hydroxy)methyl)phenol

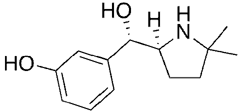

The title compound was prepared from (*S*)-1-benzyl-5,5-dimethylpyrrolidine-2-carbaldehyde (prepared from methyl (*S*)-5-oxopyrrolidine-2-carboxylate in accordance with the procedures in Example 11, Steps (a) to (d)) and 3-benzyloxymagnesium bromide in accordance with the procedures in Example 11, Steps (e) and (f).

¹H NMR (400 MHz, CDCl₃): δ 7.17 - 7.09 (m, 1H), 6.97 - 6.92 (m, 1H), 6.78 - 6.69 (m, 2H), 4.77 - 4.40 (br. s, 3H), 4.33 (d, *J* = 6.8 Hz, 1H), 3.52 - 3.42 (m, 1H), 1.86 - 1.52 (m, 4H), 1.24 (s, 3H), 1.18 (s, 3H).

### Example 19: (R)-((R)-5,5-Dimethylpyrrolidin-2-yl)(2-fluorophenyl)methanol hydrochloride

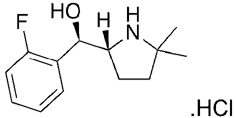

The title compound was prepared in accordance with the procedures in Example 11 using 2-fluorophenylmagnesium bromide.

¹H NMR (400 MHz, D₂O): δ 7.59 - 7.40 (m, 2H), 7.34 - 7.14 (m, 2H), 5.15 (d, *J* = 8.8 Hz, 1H), 4.21 - 4.14 (m, 1H), 2.07 - 1.79 (m, 4H), 1.54 (s, 3H), 1.46 (s, 3H).

### Example 20: (S)-((R)-5,5-Dimethylpyrrolidin-2-yl)(2-fluorophenyl)methanol hydrochloride

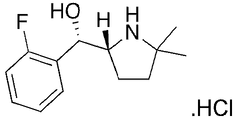

The title compound was prepared in accordance with the procedures in Example 11 using 2-fluorophenylmagnesium bromide.

¹H NMR (400 MHz, D₂O): δ 7.62 - 7.51 (m, 1H), 7.49 - 7.40 (m, 1H), 7.35 - 7.26 (m, 1H), 7.25 - 7.14 (m, 1H), 5.33 (d, *J* = 4.4 Hz, 1H), 4.26 - 4.11 (m, 1H), 2.22 - 1.88 (m, 4H), 1.51 (s, 3H), 1.45 (s, 3H).

### Example 21: (R)-((R)-5,5-Dimethylpyrrolidin-2-yl)(4-fluorophenyl)methanol hydrochloride

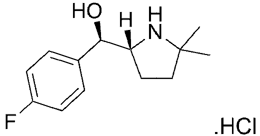

The title compound was prepared in accordance with the procedures in Example 11 using 4-fluorophenylmagnesium bromide.

¹H NMR (400 MHz, D₂O): δ 7.49 - 7.46 (m, 2H), 7.23 - 7.17 (m, 2H), 4.84 (d, *J* = 8.8Hz, 1H), 4.05 (q, *J* = 8.4 Hz, 1H), 2.13 - 1.74 (m, 4H), 1.53 (s, 3H), 1.45 (s, 3H).

### Example 22: (S)-((R)-5,5-Dimethylpyrrolidin-2-yl)(4-fluorophenyl)methanol hydrochloride

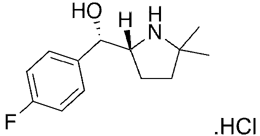

The title compound was prepared in accordance with the procedures in Example 11 using 4-fluorophenylmagnesium bromide.

¹H NMR (400 MHz, D₂O): δ 7.55 - 7.42 (m, 2H), 7.33 - 7.09 (m, 2H), 5.03 (d, J = 5.6 Hz, 1H), 4.13 - 4.07 (m, 1H), 2.22 - 1.86 (m, 4H), 1.48 (s, 3H), 1.43 (s, 3H).

### Example 23: (R)-(2-Chlorophenyl)((R)-5,5-dimethylpyrrolidin-2-yl)methanol hydrochloride

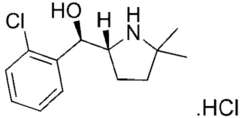

### (a) (R)-1-Benzyl-5-(((tert-butyldimethylsilyl)oxy)methyl)-2,2-dimethylpyrrolidine

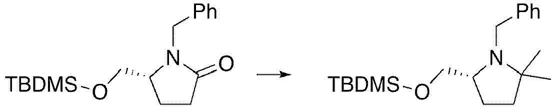

The sub-title compound was prepared from (*R*)-1-benzyl-5-(((*tert-*butyldimethylsilyl)oxy)-methyl)pyrrolidin-2-one (see Example 11, Step (c)), in accordance with the procedure in Example 1, Step (d).

### (b) tert-Butyl (R)-5-(((tert-butyldimethylsilyl)oxy)methyl)-2,2-dimethylpyrrolidine-1-carboxylate

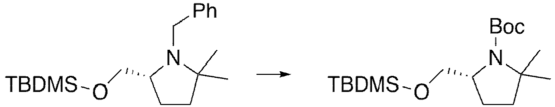

A solution of (*R*)-1-benzyl-5-(((*tert*-butyldimethylsilyl)oxy)methyl)-2,2-dimethylpyrrolidine (0.74 g, 2.22 mmol) in EtOAc (24.4 mL) was added to a mixture of Pd(OH)₂ on carbon (20 %, 1.56 g, 1.11 mmol), Boc₂O (0.58 g, 2.66 mmol) and EtOAc (9.6 mL). The mixture was hydrogenated at ambient temperature and pressure for 20 h, filtered through Celite and concentrated. The residue was purified chromatography to give the sub-title product (0.61 g, 81%).

### (c) tert-Butyl (R)-5-(hydroxymethyl)-2,2-dimethylpyrrolidine-1-carboxylate

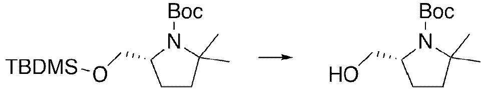

A solution of tetrabutylammonium fluoride in THF (1 M in THF, 3.58 mL, 3.58 mmol) was added to a solution of *tert*-butyl (*R*)-5-(((*tert*-butyldimethylsilyl)oxy)methyl)-2,2-dimethyl-pyrrolidine-1-carboxylate (0.61 g, 1.79 mmol) in THF (4.5 mL) at rt. The mixture was stirred at rt for 16 h, diluted with water and extracted with EtOAc. The combined extracts were washed with water, brine, dried over Na₂SO₄ and concentrated. The residue was purified by chromatography to give the sub-title compound (0.40 g, 99 %).

### (d) tert-Butyl (R)-5-formyl-2,2-dimethylpyrrolidine-1-carboxylate

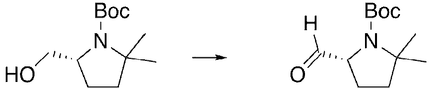

A solution of dimethylsulfoxide (0.31 mL, 4.41 mmol) in CH₂Cl₂ (1.9 mL) was added to a stirred mixture of oxalyl chloride (0.18 mL, 2.12 mmol) and CH₂Cl₂ (1.9 mL) at -78 °C. After 30 min -78 °C, a solution of *tert*-butyl (R)-5-(hydroxymethyl)-2,2-dimethylpyrrolidine-1-carboxylate (0.40 g, 1.76 mmol) in CH₂Cl₂ (3.6 mL) was added dropwise at -78 °C. After 30 minutes at -78 °C, triethylamine (1.23 mL, 8.83 mmol) was added and mixture was allowed to warm to 0 °C, stirred at 0 °C for 1 h, allowed to warm to rt and stirred at rt for 30 min. Water was added and the organic phase collected. The aq phase was extracted with CH₂Cl₂ and the combined organic phases were dried over MgSO₄ and concentrated. The residue was purified by chromatography to give the sub-title product (0.35 g, 87 %)

### (e) tert-Butyl (R)-5-((R)-(2-chlorophenyl)(hydroxy)methyl)-2,2-dimethylpyrrolidine-1-carboxylate and tert-butyl (R)-5-((S)-(2-chlorophenyl)(hydroxy)methyl)-2,2-dimethyl-pyrrolidine-1-carboxylate

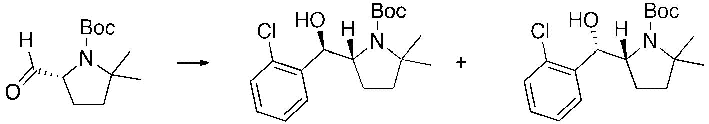

The sub-title compounds was prepared from *tert*-butyl (*R*)-5-formyl-2,2-dimethyl-pyrrolidine-1-carboxylate and 2-chlorophenylmagnesium bromide in accordance with the procedure in Example 1, Step (g) followed by chromatographic separation.

### (f) (R)-(2-Chlorophenyl)((R)-5,5-dimethylpyrrolidin-2-yl)methanol hydrochloride

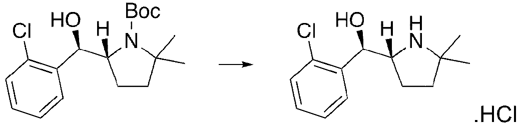

The title compound was prepared from *tert*-butyl (*R*)-5-((*R*)-(2-chlorophenyl)(hydroxy)-methyl)-2,2-dimethylpyrrolidine-1-carboxylate in accordance with the procedure in Example 5, Step (e).

¹H NMR (300 MHz, D₂O): δ 7.69 - 7.36 (m, 4H), 5.41 (d, *J* = 8.1 Hz, 1H), 4.23 - 4.12 (m, 1H), 2.11 - 1.84 (m, 4H), 1.56 (s, 3H), 1.46 (s, 3H).

### Example 24: (S)-(2-Chlorophenyl)((R)-5,5-dimethylpyrrolidin-2-yl)methanol hydrochloride

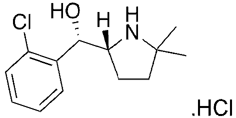

The title compound was prepared from *tert*-butyl (*S*)-5-((*R*)-(2-chlorophenyl)(hydroxy)-methyl)-2,2-dimethylpyrrolidine-1-carboxylate, see Example 23, Step (e) in accordance with the procedure in Example 5.

¹H NMR (300 MHz, D₂O): δ 7.67 (dd, *J* = 7.5, 1.8 Hz, 1H), 7.54 - 7.35 (m, 3H), 5.44 (d, *J* = 3.6 Hz, 1H), 4.28 - 4.21 (m, 1H), 2.31 - 2.10 (m, 1H), 2.06 - 1.70 (m, 3H), 1.54 (s, 3H), 1.47 (s, 3H).

### Example 25: (R)-(3-Chlorophenyl)((R)-5,5-dimethylpyrrolidin-2-yl)methanol hydrochloride

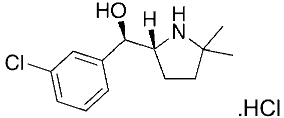

The title compound was prepared in accordance with Example 23, using 3-chlorophenylmagnesium bromide in Step (e).

¹H NMR (400 MHz, D₂O): δ 7.58 - 7.30 (m, 4H), 4.82 (overlapping with D₂O, 1H), 4.05 - 3.97 (m, 1H), 2.00 - 1.77 (m, 4H), 1.51 (s, 3H), 1.43 (s, 3H).

### Example 26: (S)-(2-Chlorophenyl)((R)-5,5-dimethylpyrrolidin-2-yl)methanol hydrochloride

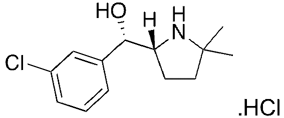

The title compound was prepared in accordance with Example 23, using 3-chlorophenylmagnesium bromide in Step (e).

¹H NMR (400 MHz, D₂O): δ 7.49 - 7.48 (m, 1H), 7.46 - 7.32 (m, 3H), 5.04 (d, *J* = 4.8 Hz, 1H), 4.11 - 4.06 (m, 1H), 2.17 - 1.86 (m, 4H), 1.47 (s, 3H), 1.42 (s, 3H).

### Example 27: (R)-((R)-5,5-Dipropylpyrrolidin-2-yl)(3-fluorophenyl)methanol hydrochloride

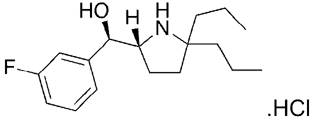

### (a) (R)-1-Benzyl-5-(((tert-butyldimethylsilyl)oxy)methyl)-2,2-dipropylpyrrolidine

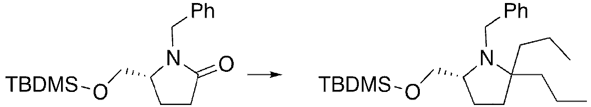

The sub-title compound was prepared from (*R*)-1-benzyl-5-(((*tert*-butyldimethylsilyl)oxy)-methyl)pyrrolidin-2-one (see Example 11, Step (c)) and propylmagnesium bromide accordance with the procedure in Example 1, Step (d).

### (b) (R)-((R')-5,5-Dipropylpyrrolidin-2-yl)(3-fluorophenyl)methanol hydrochloride

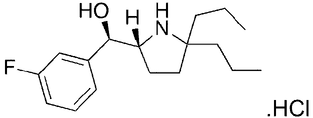

The title compound was prepared from (*R*)-1-benzyl-5-(((tert-butyldimethylsilyl)oxy)-methyl)-2,2-dipropylpyrrolidine in accordance with the procedures in Example 1, Steps (e) to (h), followed by addition of HCl in Et₂O as described in Example 5, Step (e).

¹H NMR (300 MHz, D₂O): δ 7.55- 7.41 (m, 1H), 7.33 - 7.12 (m, 3H), 4.85 (d, *J* = 8.8 Hz, 1H), 4.04 - 3.90 (m, 1H), 2.08 - 1.61 (m, 8H), 1.53 - 1.25 (m, 4H), 1.07 - 0.85(m, 6H).

### Example 28: (S)-((R)-5,5-Dipropylpyrrolidin-2-yl)(3-fluorophenyl)methanol hydrochloride

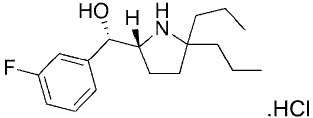

The title compound was prepared from (*R*)-1-benzyl-5-(((*tert*-butyldimethylsilyl)oxy)-methyl)-2,2-dipropylpyrrolidine, see Example 27, Step (a), in accordance with the procedures in Example 1, Steps (e) to (h), followed by addition of HCl in Et₂O as described in Example 5, Step (e).

¹H NMR (300 MHz, D₂O): δ 7.55 7.41 (m, 1H), 7.32 - 7.09 (m, 3H), 5.12 (d, *J* = 4.1 Hz, 1H), 4.12 - 3.98 (m, 1H), 2.18 - 1.58 (m, 8H), 1.52 - 1.23 (m, 4H), 1.06 - 0.83 (m, 6H).

### Example 29: (R)-(3-Fluorophenyl)((R)-4-azaspiro[2.4]heptan-5-yl)methanol hydrochloride

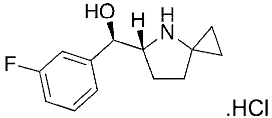

### (a) (R)-4-Benzyl-5-(((tert-butyldimethylsilyl)oxy)methyl)-4-azaspiro[2.4]heptane

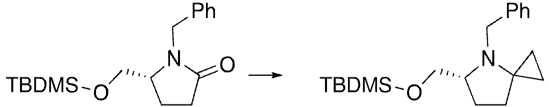

TiCl₄ (1 M in toluene, 6.46 mL, 6.46 mmol) was added dropwise to Ti(O*i*Pr)₄ (5.76 mL, 19.37 mmol) under ice-cooling. The mixture was stirred at rt for 2 h and cooled in an ice-bath. MeLi (1.6M in Et₂O, 16.1 mL, 25.82 mmol) was added dropwise and the mixture was stirred at room temperature for 1 h. A solution of ((*R*)-1-benzyl-5-(((*tert-*butyldimethylsilyl)oxy)methyl)pyrrolidin-2-one (see Example 11, Step (c)) (2.75 g, 8.61 mmol) THF (27 ml) was added and after stirring for 5 min, EtMgBr (0.9M in THF, 28,69 mL, 25.82 mmol) was added dropwise with syringe pump (0.8 ml/min) and the mixture was stirred at rt for 16 h. Water was added and the mixture was vigorously stirred for 2 h and filtered through Celite. The solids were washed with EtOAc and the filtrate was extracted with EtOAc. The combined organic phases were washed with brine, dried over NaSO₄, and concentrated. The residue was purified by chromatography to give the sub-title compound (0.74 g, 26%).

### (b) (R)-(3-Fluorophenyl)((R)-4-azaspiro[2.4]heptan-5-yl)methanol hydrochloride

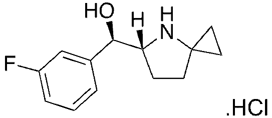

The title compound was prepared from (*R*)-4-benzyl-5-(((*tert*-butyldimethylsilyl)oxy)-methyl)-4-azaspiro[2.4]heptane in accordance with the procedures in Example 1, Steps (e) to (h), followed by addition of HCl in Et₂O as described in Example 5, Step (e).

¹H NMR (400 MHz, CD₃OD): δ 7.49 - 7.40 (m, 1H), 7.35 - 7.25 (m, 2H), 7.15 - 7.06 (m, 1H), 4.82 (d, *J* = 7.9 Hz, 1H), 4.02 - 3.90 (m, 1H), 2.20 - 1.94 (m, 4H), 1.31 - 1.14 (m, 2H), 0.99 (ddd, *J* = 9.7, 6.1, 5.3 Hz, 1H), 0.89 (ddd, *J* = 10.6, 6.5, 5.3 Hz, 1H).

### Example 30: (S)-(3-Fluorophenyl)((R)-4-azaspiro[2.4]heptan-5-yl)methanol hydrochloride

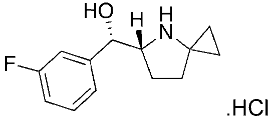

The title compound was prepared from (*R*)-4-benzyl-5-(((*tert*-butyldimethylsilyl)oxy)-methyl)-4-azaspiro[2.4]heptane, see Example 29, Step (a)) in accordance with the procedures in Example 1, Steps (e) to (h), followed by addition of HCl in Et₂O as described in Example 5, Step (e).

¹H NMR (400 MHz, CD₃OD): δ 7.47 - 7.37 (m, 1H), 7.31 - 7.19 (m, 2H), 7.12 - 7.00 (m, 1H), 5.08 (d, *J* = 3.7 Hz, 1H), 4.06 (ddd, *J* = 9.7, 7.6, 3.7 Hz, 1H), 2.32 - 2.16 (m, 1H), 2.11 - 1.95 (m, 2H), 1.87 - 1.74 (m, 1H), 1.28 - 1.13 (m, 2H), 1.00 - 0.84 (m, 2H).

### Example 31: (R)-(3-Fluorophenyl)((R)-1-azaspiro[4.4]nonan-2-yl)methanol maleate

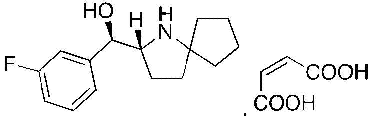

### (a) (R)-2,2-Diallyl-1-benzyl-5-(((tert-butyldimethylsilyl)oxy)methyl)pyrrolidine

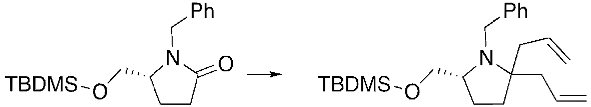

The sub-title compound was prepared from (*R*)-1-benzyl-5-(((*tert*-butyldimethylsilyl)oxy)-methyl)pyrrolidin-2-one (see Example 11, Step (c)) and allylmagnesium bromide accordance with the procedure in Example 1, Step (d).

### (b) (R)-(1-Benzyl-1-azaspiro[4.4]non-7-en-2-yl)methanol

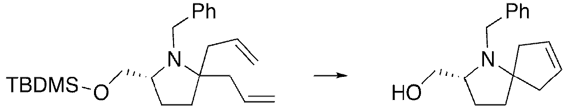

A mixture of (*R*)-2,2-diallyl-1-benzyl-5-(((tert-butyldimethylsilyl)oxy)methyl)pyrrolidine (1.00 g, 2.59 mmol), Grubbs 2^{nd} generation catalyst (0.11 g, 0.13 mmol) and CH₂Cl₂ (20 ml) was heated at reflux for 2 h. The mixture was allowed to cool, concentrated and purified by chromatography. The material was dissolved in THF (6 mL) and tetrabutylammonium fluoride (1 M in THF, 3.91 mL, 3.91 mmol) was added and the mixture was stirred at rt overnight. Water was added and the mixture was extracted with EtOAc. The combined extracts were washed with water, brine, dried over Na₂SO₄, and concentrated. The residue was purified by reverse phase chromatography to give the sub-title compound (0.31 g, 58%).

### (c) (R)-1-Benzyl-1-azaspiro[4.4]non-7-ene-2-carbaldehyde

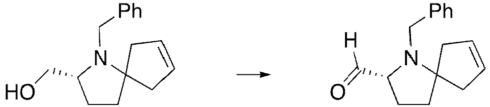

The sub-title compound was prepared in accordance with the procedure in Example 1, Step (f).

### (d) (R)-((R)-1-Benzyl-1-azaspiro[4.4]non-7-en-2-yl)(3-fluorophenyl)methanol and (S)-((R)-1-Benzyl-1-azaspiro[4.4]non-7-en-2-yl)(3-fluorophenyl)methanol

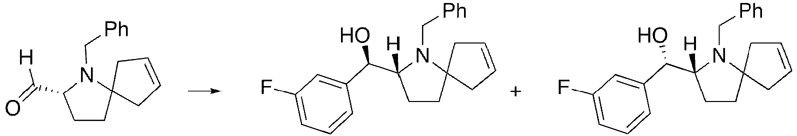

The sub-title compounds was prepared from (R)-1-benzyl-1-azaspiro[4.4]non-7-ene-2-carbaldehyde and 3-flurophenylmagnesium bromide in accordance with the procedure in Example 1, Step (g) followed by chromatographic separation.

### (e) (R)-(3-Fluorophenyl)((R)-1-azaspiro[4.4]nonan-2-yl)methanol maleate

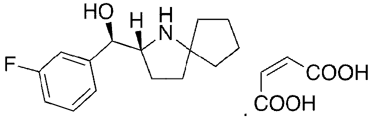

A mixture of (*R*)-((*R*)-1-benzyl-1-azaspiro[4.4]non-7-en-2-yl)(3-fluorophenyl)methanol (40 mg, 0.12 mmol), Pd-C (10%, 25 mg, 0.024 mmol) and *i*PrOH (2 mL) was hydrogenated at 8 bar and rt for 2 h 30 min, filtered through Celite and concentrated. The residue was dissolved in MeCN (3 mL) and maleic acid (13 mg, 0.11 mmol) was added. The mixture was concentrated and the residue purified by reverse phase chromatography to give the title compound (13 mg, 32%).

¹H NMR (400 MHz, CD₃OD) δ 7.47- 7.36 (m, 1H), 7.32 - 7.22 (m, 2H), 7.12 - 7.03 (m, 1H), 6.26 (s, 2H), 4.75 (d, *J* = 7.9 Hz, 1H), 3.90 - 3.77 (m, 1H), 2.19 - 1.63 (m, 12H).

### Example 32: (S)-(3-Fluorophenyl)((R)-1-azaspiro[4.4]nonan-2-yl)methanol maleate

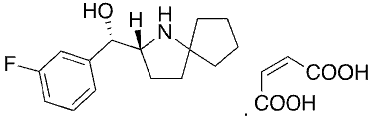

The title compound was prepared from (*S*)-((*R*)-1-benzyl-1-azaspiro[4.4]non-7-en-2-yl)(3-fluorophenyl)methanol, see Example 31, Step (d)) in accordance with the procedures in Example 31, Step (e).

¹H NMR (400 MHz, CD₃OD) δ 7.47- 7.36 (m, 1H), 7.29 - 7.16 (m, 2H), 7.09 - 6.98 (m, 1H), 6.26 (s, 2H), 5.06 (d, *J* = 3.1 Hz, 1H), 4.02 - 3.89 (m, 1H), 2.28 - 2.08 (m, 2H), 2.08 - 1.61 (m, 10H).

### Example 33: (R)-((R)-4,4-dimethylazetidin-2-yl)(3-fluorophenyl)methanol hydrochloride

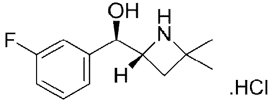

### (a) 2-(Benzylamino)-2-methylpropan-1-ol

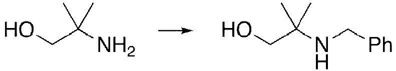

Benzaldehyde (3.4 mL, 33.7 mmol) was added dropwise to a stirred mixture of 2-amino-2-methylpropan-1-ol (3.0 g, 33.7 mmol), 5 Å molecular sieves (5 g) and CH₂Cl₂ (30 mL) at rt. The mixture was stirred at rt for 3 h, filtered through a pad of cotton and concentrated. MeOH (20 mL) followed by NaBH₄ (1.5 g, 40.4 mmol) was added and the mixture was stirred at rt for 1 h. NH₄Cl (aq, sat, 10 mL) was added and the mixture was concentrated, treated with NaOH (1 M, 20 mL) and extracted with EtOAc. The combined extracts were dried (Na₂SO₄) and concentrated to give the sub-title compound (5.8 g, 33.3 mmol, 96 %), which was used in the next step without further purification.

### (b) (2-(Benzyl(1-hydroxy-2-methylpropan-2-yl)amino)acetonitrile

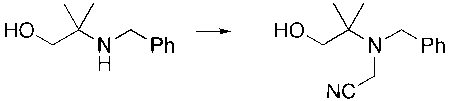

Bromoacetonitrile (5.3 mL, 78.1 mmol) and K₂CO₃ (5.8 g, 41.8 mmol) were added to a solution of 2-(benzylamino)-2-methylpropan-1-ol (5.0 g, 27.9 mmol) in MeCN (40 mL) at rt. The mixture was heated in a sealed vial for 16 h at 100 °C and concentrated. The residue was treated with H₂O and extracted with Et₂O. The combined extracts were dried (Na₂SO₄), concentrated and the residue purified by chromatography to give the sub-title compound (5.0 g, 22.8 mmol, 82 %).

### (c) 1-Benzyl-4,4-dimethylazetidine-2-carbonitrile

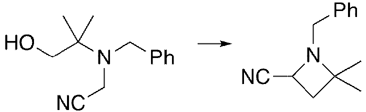

Diethyl chlorophosphate (2.1 mL, 14.4 mmol) was added drop-wise to a solution of 2-(benzyl(1-hydroxy-2-methylpropan-2-yl)amino)acetonitrile (3.0 g, 13.7 mmol) in THF (30 mL) at -20 °C. Potassium bis(trimethylsilyl)amide (1 M in THF, 28.9 mL, 28.9 mmol) was added dropwise keeping the temperature below -15 °C and the mixture was stirred at -20 °C for 1 h. H₂O was added and the mixture was extracted with EtOAc. The combined extracts were dried (Na₂SO₄), concentrated and the residue purified by chromatography to give the sub-title compound (2.1 g, 10.5 mmol, 76 %).

### (d) 1-Benzyl-4,4-dimethylazetidine-2-carboxylic acid

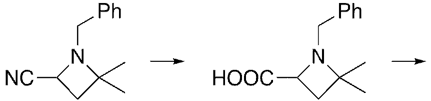

H₂O (5 mL) followed by NaOH (0.8 g, 20 mmol) were added to a solution of 1-benzyl-4,4-dimethylazetidine-2-carbonitrile (2.0 g, 9.1 mmol) in EtOH (10 mL) at rt. The mixture was heated in a sealed vial at 80 °C for 24 h and allowed to cool. The pH was adjusted to 7 with HCl (aq, 1 M) and the mixture was concentrated. The residue was extracted with CH₂Cl₂. Filtration and concentrated gave the sub-title compound (2.1 g, 9.6 mmol, 96 %), which was used in the next step without any further purification.

### (e) (1-Benzyl-4,4-dimethylazetidin-2-yl)methanol

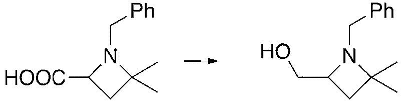

LiAlH₄ (2.4 M in THF, 7.6 mL, 18.2 mmol) was added dropwise to a mixture of 1-benzyl-4,4-dimethylazetidine-2-carboxylic acid (2.1 g, 9.6 mmol) and THF (40 mL) at 0 °C. The mixture was stirred at 0 °C for 10 min, the cooling bath was removed and the stirring continued for 15 min. The mixture was carefully quenched by addition of NH₄Cl (aq, sat, 10 mL) and extracted with CH₂Cl₂. The combined extracts were dried (Na₂SO₄) and concentrated to give the sub-title compound (1.4 g, 6.8 mmol, 75 %), which was used in the next step without further purification.

### (f) tert-Butyl 4-(hydroxymethyl)-2,2-dimethylazetidine-1-carboxylate

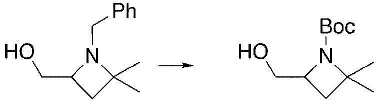

A mixture of (1-benzyl-4,4-dimethylazetidin-2-yl)methanol (1.4 g, 6.8 mmol), Boc₂O (2.4 mL, 10.2 mmol), Pd-C (10 %, 0.72 g, 0.7 mmol) and EtOH (15 mL) was hydrogenated at normal pressure and temperature for 16 h and filtered through Celite. The solids were washed with EtOH and the combined liquids concentrated and purified by chromatography to give the sub-title compound (1.1 g, 5.1 mmol, 75 %).

### (g) tert-Butyl 4-formyl-2,2-dimethylazetidine-1-carboxylate

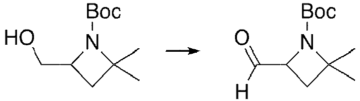

A suspension of Dess-Martin periodinane (1.18 g, 2.79 mmol) in CH₂Cl₂ (10 mL) was slowly added via a syringe to a solution of *tert*-butyl 4-(hydroxymethyl)-2,2-dimethyl-azetidine-1-carboxylate 500 mg, 2.34 mmol) in CH₂Cl₂ (20 mL) at rt. The mixture was stirred at rt for 1 h and quenched with Na₂S₂O₃ (aq, 10 %) and NaHCO₃ (aq, sat), stirred for 10 min and extracted with CH₂Cl₂. The combined extracts were washed with NaHCO₃ (aq, sat),2dried (Na₂SO₄) and concentrated to give the sub-title compound (495 mg, 2.32 mmol, 99 %), which was used in the next step without further purification.

### (h) tert-Butyl (R)-4-((R)-(3-fluorophenyl)(hydroxy)methyl)-2,2-dimethylazetidine-1-carboxylate

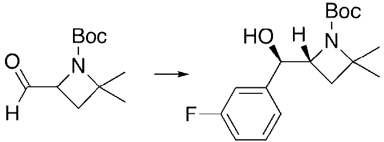

3-Fluorophenylmagnesium bromide, freshly prepared from 1-bromo-2-fluorobenzene and iPrMgCl.LiCl, (1 M in THF, 3.17 mL, 3.17 mmol) was added dropwise to a solution of *tert-*butyl 4-formyl-2,2-dimethylazetidine-1-carboxylate (450 mg, 2.11 mmol) in THF (12 mL) at -20 °C. The mixture was stirred at -20 °C for 30 min and then at rt for 1 h. NH₄Cl (aq, sat, 20 mL) was added and the mixture was extracted with Et₂O. The combined extracts were dried (Na₂SO₄) and concentrated and the residue purified chromatography to give a mixture of stereoisomers that were separated by preparative chiral chromatography to give the sub-title compound (100 mg, 0.32 mmol, 15 %) along with the (*S,S*)-isomer (102 mg, 0.33 mmol, 16 %) (*S,R*)-isomer (49 mg, 0.16 mmol, 8 %) and the (*R,S*)-isomer (49 mg, 0.16 mmol, 8 %).

### (i) (R)-((R)-4,4-Dimethylazetidin-2-yl)(3-fluorophenyl)methanol hydrochloride

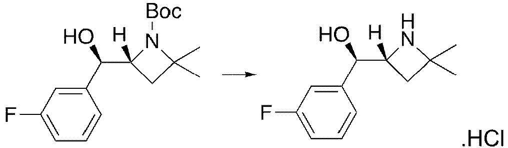

H₂O (1 mL) followed by NaOH (414 mg, 10.3 mmol) was added to a solution of *tert*-butyl (*R*)-4-((*R*)-(3-fluorophenyl)(hydroxy)methyl)-2,2-dimethylazetidine-1-carboxylate (80 mg, 0.26 mmol) in EtOH (2 mL) at rt. The mixture was heated in a sealed vial at 130 °C for 48 h, cooled to rt and concentrated. The residue was extracted with EtOAc. The extract was concentrated and purified by chromatography. The product was dissolved in Et₂O (3 mL). HCl (2 M in Et₂O, 0.11 mL, 0.22 mmol) was added dropwise at rt and the mixture was stirred at rt for 15 min and filtered to give the title compound (42 mg, 0.20 mmol, 66 %).

¹H NMR (300 MHz, CD₃OD) δ 7.47 - 7.38 (m, 1H), 7.35 - 7.25 (m, 2H), 7.12 - 7.02 (m, 1H), 4.93 (d, *J* = 4.4 Hz, 1H), 4.50 (td, *J* = 8.9, 4.4 Hz, 1H), 2.58 (dd, *J* = 11.9, 8.9 Hz, 1H), 2.27 (dd, *J* = 11.9, 8.9 Hz, 1H), 1.63 (s, 3H), 1.62 (s, 3H).

### Example 34: (S)-((S)-4,4-dimethylazetidin-2-yl)(2-fluorophenyl)methanol hydrochloride

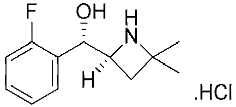

The title compound was prepared from tert-butyl (*S*)-4-((*S*)-(2-fluorophenyl)(hydroxy)-methyl)-2,2-dimethylazetidine-1-carboxylate, see Example 33, Step (h) in accordance with the procedure in Example 33, Step (i).

¹H NMR (400 MHz, CD₃OD) δ 7.73 - 7.64 (m, 1H), 7.43 - 7.35 (m, 1H), 7.30 - 7.24 (m, 1H), 7.15 (ddd, *J* = 10.8, 8.2, 1.2 Hz, 1H), 5.20 (d, *J* = 4.5 Hz, 1H), 4.51 (tdd, *J* = 8.9, 4.6, 0.7 Hz, 1H), 2.55 (dd, *J* = 11.9, 8.9 Hz, 1H), 2.29 (dd, *J* = 11.9, 8.9 Hz, 1H), 1.63 (s, 3H), 1.62 (s, 3H).

### Example 35: (R)-((R)-4,4-dimethylazetidin-2-yl)(2-fluorophenyl)methanol hydrochloride

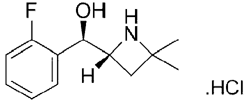

The title compound was prepared from 2-fluorophenylmagnesium bromide and *tert*-butyl 4-formyl-2,2-dimethylazetidine-1-carboxylate in accordance with the procedures in Example 33, Steps (h) and (i).

¹H NMR (400 MHz, CD₃OD) δ 7.73 - 7.64 (m, 1H), 7.43 - 7.35 (m, 1H), 7.30 - 7.24 (m, 1H), 7.15 (ddd, *J* = 10.8, 8.2, 1.2 Hz, 1H), 5.20 (d, *J* = 4.5 Hz, 1H), 4.51 (tdd, *J* = 8.9, 4.6, 0.7 Hz, 1H), 2.55 (dd, *J* = 11.9, 8.9 Hz, 1H), 2.29 (dd, *J* = 11.9, 8.9 Hz, 1H), 1.63 (s, 3H), 1.62 (s, 3H).

### Example 36: (S)-((R)-4,4-dimethylazetidin-2-yl)(2-fluorophenyl)methanol hydrochloride

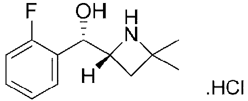

The title compound was prepared from *tert*-butyl (*S*)-4-((*R*)-(2-fluorophenyl)(hydroxy)-methyl)-2,2-dimethylazetidine-1-carboxylate, see Example 33, Step (h) in accordance with the procedure in Example 33, Step (i).

¹H NMR (400 MHz, CD₃OD) δ 7.62 (td, *J* = 7.6, 1.8 Hz, 1H), 7.59 - 7.52 (m, 1H), 7.35 (td, *J* = 7.6, 1.2 Hz, 1H), 7.33 - 7.26 (m, 1H), 4.93 - 4.88 (m, overlapping with CD₃OD, 1H), 4.72 (d, *J* = 6.9 Hz, 1H), 2.48 (dd, *J* = 13.6, 7.0 Hz, 1H), 2.11 (dd, *J* = 13.6, 6.1 Hz, 1H), 1.65 (s, 3H), 1.54 (s, 3H).

### Example 37: (R)-((S)-4,4-dimethylazetidin-2-yl)(2-fluorophenyl)methanol hydrochloride

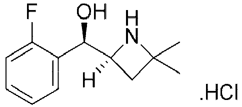

The title compound was prepared from *tert*-butyl (*R*)-4-((*S*)-(2-fluorophenyl)(hydroxy)-methyl)-2,2-dimethylazetidine-1-carboxylate, see Example 33, Step (h) in accordance with the procedure in Example 33, Step (i).

¹H NMR (400 MHz, CD₃OD) δ 7.62 (td, *J* = 7.6, 1.8 Hz, 1H), 7.59 - 7.52 (m, 1H), 7.35 (td, *J* = 7.6, 1.2 Hz, 1H), 7.33 - 7.26 (m, 1H), 4.93 - 4.88 (m, overlapping with CD₃OD, 1H), 4.72 (d, *J* = 6.9 Hz, 1H), 2.48 (dd, *J* = 13.6, 7.0 Hz, 1H), 2.11 (dd, *J* = 13.6, 6.1 Hz, 1H), 1.65 (s, 3H), 1.55 (s, 3H).

### Example 38: (S)-((R)-4,4-dimethylazetidin-2-yl)(2-fluorophenyl)methanol hydrochloride

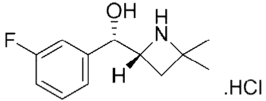

The title compound was prepared from 3-fluorophenylmagnesium bromide and tert-butyl 4-formyl-2,2-dimethylazetidine-1-carboxylate in accordance with the procedures in Example 33, Steps (h) and (i).

¹H NMR (300 MHz, CD₃OD) δ 7.59 - 7.54 (m, 1H), 7.44 - 7.33 (m, 2H), 7.28 - 7.19 (m, 1H), 4.78 (td, *J* = 7.6, 6.4 Hz, 1H), 4.61 (d, *J* = 7.7 Hz, 1H), 2.46 (dd, *J* = 13.6, 7.5 Hz, 1H), 2.08 (dd, *J* = 13.6, 6.4 Hz, 1H), 1.66 (s, 3H), 1.56 (s, 3H).

### Example 39: (R)-((S)-4,4-dimethylazetidin-2-yl)(3-fluorophenyl)methanol hydrochloride

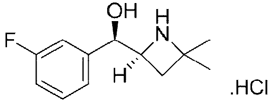

The title compound was prepared from 3-fluorophenylmagnesium bromide and *tert*-butyl 4-formyl-2,2-dimethylazetidine-1-carboxylate in accordance with the procedures in Example 33, Steps (h) and (i).

¹H NMR (300 MHz, CD₃OD) δ 7.59 - 7.50 (m, 1H), 7.43 - 7.33 (m, 2H), 7.27 - 7.19 (m, 1H), 4.77 (td, *J* = 7.6, 6.4 Hz, 1H), 4.61 (d, *J* = 7.7 Hz, 1H), 2.46 (dd, *J* = 13.6, 7.5 Hz, 1H), 2.08 (dd, *J* = 13.6, 6.4 Hz, 1H), 1.66 (s, 3H), 1.56 (s, 3H).

### Example 40: (S)-((S)-4,4-dimethylazetidin-2-yl)(3-fluorophenyl)methanol hydrochloride

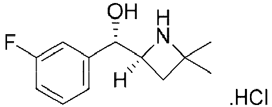

The title compound was prepared from 3-fluorophenylmagnesium bromide and *tert*-butyl 4-formyl-2,2-dimethylazetidine-1-carboxylate in accordance with the procedures in Example 33, Steps (h) and (i).

¹H NMR (300 MHz, CD₃OD) δ 7.47 - 7.38 (m, 1H), 7.36 - 7.24 (m, 2H), 7.12 - 6.99 (m, 1H), 4.93 (d, *J* = 4.4 Hz, 1H), 4.50 (td, *J* = 8.9, 4.4 Hz, 1H), 2.58 (dd, *J* = 11.9, 8.9 Hz, 1H), 2.26 (dd, *J* = 11.9, 9.0 Hz, 1H), 1.63 (s, 3H), 1.62 (s, 3H).

### Example 41: (R)-((S)-6,6-dimethylpiperidin-2-yl)(2-fluorophenyl)methanol hydrochloride

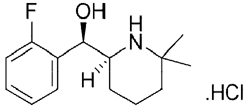

### (a) (S)-6-(((tert-Butyldimethylsilyl)oxy)methyl)-2,2-dimethylpiperidine

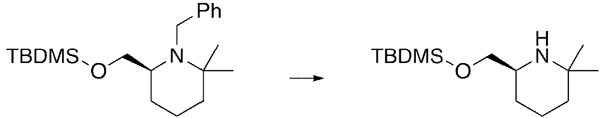

A mixture of (*S*)-1-benzyl-6-(((*tert*-butyldimethylsilyl)oxy)methyl)-2,2-dimethylpiperidine (1.64 g, 4.72 mmol), prepared in accordance with Example 1, Steps (a) to (d) from (S)-2-aminoadipic acid, Pd(OH)₂ on carbon (20 %, 3.31 g, 2.36 mmol) and EtOAc (30 mL) was hydrogenated at normal pressure and temperature for 1 h and filtered through Celite. The solids were washed with EtOAc and the combined liquids dried (Na₂SO₄) and concentrated to give the sub-title compound (1.07 g, 4.16 mmol, 88 %), which was used in the following step without further purification.

### (b) tert-Butyl (S)-6-(((tert-butyldimethylsilyl)oxy)methyl)-2,2-dimethylpiperidine-1-carboxylate

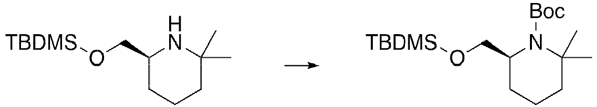

A mixture of (*S*)-6-(((*tert*-butyldimethylsilyl)oxy)methyl)-2,2-dimethylpiperidine (1.07 g, 4.16 mmol) and Boc₂O (1.09 g, 4.99 mmol) was heated at 60 °C for 120 h. The mixture was dissolved in EtOH and imidazole (0.51 g, 7.48 mmol) was added. The mixture was stirred at rt for 15 min, concentrated and dissolved in CHCl₃. The mixture was washed with ice-cold HCl (aq., 1 M), brine and dried (Na₂SO₄), and concentrated. The residue was purified by chromatography to give the sub-title compound (1.06 g, 2.95 mmol, 71 %).

### (c) tert-Butyl (S)-6-((R)-(2fluorophenyl)(hydroxy)methyl)-2,2-dimethylpiperidine-1-carboxylate

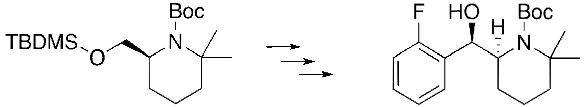

The sub-title compound was prepared in accordance with Example 5, Steps (c) and (d) from *tert*-butyl (*S*)-6-(((*tert*-butyldimethylsilyl)oxy)methyl)-2,2-dimethylpiperidine-1-carboxylate and 2-fluorophenylmagnesium bromide.

### (d) (R)-((S)-6,6-Dimethylpiperidin-2-yl)(2-fluorophenyl)methanol hydrochloride

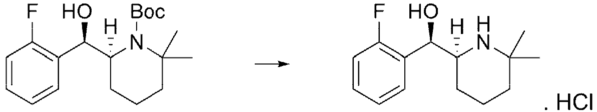

The title compound was prepared in accordance with Example 5, Step (e)) from tert-butyl (*S*)-6-((*R*)-(2fluorophenyl)(hydroxy)methyl)-2,2-dimethylpiperidine-1-carboxylate.

¹H NMR (400 MHz, CD₃OD) δ 7.64 - 7.58 (m, 1H), 7.41 - 7.33 (m, 1H), 7.25 (td, *J* = 7.6, 1.2 Hz, 1H), 7.12 (ddd, *J* = 10.7, 8.2, 1.2 Hz, 1H), 5.29 (d, *J* = 2.8 Hz, 1H), 3.57 - 3.46 (m, 1H), 1.77 - 1.56 (m, 5H), 1.47 (s, 3H), 1.44 (d, *J* = 2.2 Hz, 1H), 1.41 (s, 3H).

### Example 42: (S)-((S)-6,6-dimethylpiperidin-2-yl)(2-fluorophenyl)methanol hydrochloride

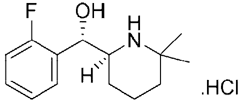

### (a) tert-Butyl (S)-6-((S)-(2fluorophenyl)(hydroxy)methyl)-2,2-dimethylpiperidine-1-carboxylate

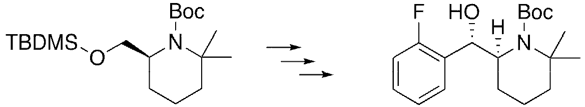

The sub-title compound was prepared in accordance with Example 5, Steps (c) and (d) from *tert*-butyl (*S*)-6-(((*tert*-butyldimethylsilyl)oxy)methyl)-2,2-dimethylpiperidine-1-carboxylate and 2-fluorophenylmagnesium bromide.

### (b) (S)-((S)-6,6-Dimethylpiperidin-2-yl)(2-fluorophenyl)methanol hydrochloride

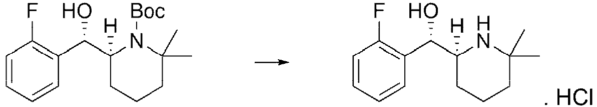

The title compound was prepared in accordance with Example 5, Step (e)) from *tert-*butyl (*S*)-6-((*R*)-(2fluorophenyl)(hydroxy)methyl)-2,2-dimethylpiperidine-1-carboxylate.

¹H NMR (400 MHz, CD₃OD) δ 7.59 (td, J = 7.5, 1.8 Hz, 1H), 7.43 - 7.35 (m, 1H), 7.27 (td, *J* = 7.5, 1.2 Hz, 1H), 7.12 (ddd, *J* = 10.6, 8.3, 1.2 Hz, 1H), 4.96 (d, *J* = 9.4 Hz, 1H), 3.46 - 3.35 (m, 1H), 1.82 - 1.55 (m, 4H), 1.49 (s, 3H), 1.45 - 1.27 (m, 5H).

### Example 43: (S)-((S)-6,6-dimethylpiperidin-2-yl)(3-fluorophenyl)methanol hydrochloride

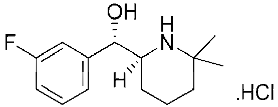

The title compound was prepared from *tert*-butyl (S)-6-(((*tert*-butyldimethylsilyl)oxy)-methyl)-2,2-dimethylpiperidine-1-carboxylate in accordance with Example 5, Steps (c), (d) and (e) using 3-fluorophenylmagnesium bromide in the appropriate step.

¹H NMR (400 MHz, CD₃OD) δ 7.45 - 7.38 (m, 1H), 7.25 - 7.17 (m, 2H), 7.14 - 7.05 (m, 1H), 4.53 (d, *J* = 9.2 Hz, 1H), 3.41 - 3.32 (m, 1H), 1.83 - 1.53 (m, 4H), 1.48 (s, 3H), 1.44 - 1.20 (m, 5H).

### Example 44: (R)-((S)-6,6-dimethylpiperidin-2-yl)(3-fluorophenyl)methanol hydrochloride

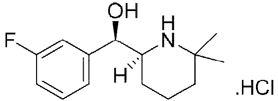

The title compound was prepared in accordance with Example 41, using 3-fluorophenylmagnesium bromide in the appropriate step.

¹H NMR (400 MHz, CD₃OD) δ 7.47 - 7.34 (m, 1H), 7.27 - 7.17 (m, 2H), 7.08 - 7.00 (m, 1H), 5.06- 5.00 (m, 1H), 3.57 - 3.47 (m, 1H), 1.75 - 1.54(m, 5H), 1.47 (s, 3H), 1.46 - 1.36 (m, 4H).

### Example 45: (R)-(3-chlorophenyl)((R)-4,4-dimethylazetidin-2-yl)methanol maleate

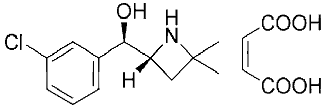

*tert*-Butyl (*R*)-4-((*R*)-(3-chlorophenyl)(hydroxy)methyl)-2,2-dimethylazetidine-1-carboxylate, along with its (*S,S*), (*R,S*) and (*S,R*) isomers were prepared from *tert-*butyl 5-formyl-2,2-dimethylpyrrolidine-1-carboxylate and 3-chlorophenylmagnesium bromide in accordance with the procedure in Example 35, Step (h). The (*R,R*) isomer (78 mg, 0.24 mmol) was dissolved in CH₂Cl₂ (1 mL) and lutidine (0.17 mL, 1.44 mmol) and trimethylsilyl trifluoromethanesulfonate (0.22 mL, 1.20 mmol) were added at rt. The mixture was stirred at rt for 20 h and NaHCO₃ (aq, sat) was added. The mixture was extracted with CH₂Cl₂ and the combined extracts were dried (Na₂SO₄) and concentrated. The residue was dissolved in iPrOH (1 mL). Maleic acid (26.4 mg, 0.23 mmol) was added and the mixture was stirred at 60 °C overnight and allowed to cool. The precipitate was collected to give the title compound (60 mg, 0.18 mmol, 73 %).

¹H NMR (300 MHz, CD₃OD) δ 7.59 -7.54 (m, 1H), 7.43 - 7.28 (m, 3H), 6.25 (s, 2H), 4.89 (d, *J* = 4.3 Hz, 1H), 4.48 (td, *J* = 8.9, 4.3 Hz, 1H), 2.56 (dd, *J* = 11.9, 8.9 Hz, 1H), 2.24 (dd, *J* = 11.8, 8.9 Hz, 1H), 1.60 (s, 3H), 1.59 (s, 3H).

### Example 46: (S)-(3-chlorophenyl)((R)-4,4-dimethylazetidin-2-yl)methanol maleate

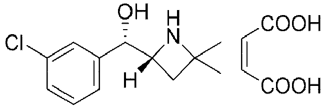

The title compound was prepared in accordance with the procedure in Example 45 from *tert*-butyl (*S*)-4-((*R*)-(3-chlorophenyl)(hydroxy)methyl)-2,2-dimethylazetidine-1-carboxylate.

¹H NMR (300 MHz, CD₃OD) 7.48 - 7.44 (m, 1H), 7.41 - 7.29 (m, 3H), 6.26 (s, 2H), 4.98 (d, *J* = 3.4 Hz, 1H), 4.54 (td, *J* = 8.9, 3.4 Hz, 1H), 2.63 (dd, *J* = 11.8, 8.8 Hz, 1H), 1.95 (dd, *J* = 11.8, 9.0 Hz, 1H), 1.63 (s, 3H), 1.60 (s, 3H).

### Example 47: (R)-(3-chlorophenyl)((S)-4,4-dimethylazetidin-2-yl)methanol maleate

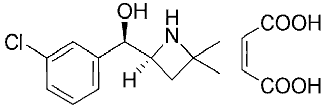

The title compound was prepared in accordance with the procedure in Example 45 from *tert*-butyl (*R*)-4-((*S*)-(3-chlorophenyl)(hydroxy)methyl)-2,2-dimethylazetidine-1-carboxylate.

¹H NMR (300 MHz, CD₃OD) 7.48 - 7.44 (m, 1H), 7.42 - 7.27 (m, 3H), 6.26 (s, 2H), 4.98 (d, *J* = 3.4 Hz, 1H), 4.55 (td, *J* = 8.9, 3.4 Hz, 1H), 2.62 (dd, *J* = 11.8, 8.8 Hz, 1H), 1.95 (dd, *J* = 11.8, 9.0 Hz, 1H), 1.63 (s, 3H), 1.60 (s, 3H).

### Example 48: (S)-(3-chlorophenyl)((S)-4,4-dimethylazetidin-2-yl)methanol maleate

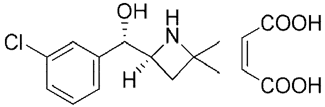

The title compound was prepared in accordance with the procedure in Example 45 from *tert*-butyl (*S*)-4-((*S*)-(3-chlorophenyl)(hydroxy)methyl)-2,2-dimethylazetidine-1-carboxylate.

¹H NMR (300 MHz, CD₃OD) δ 7.59 -7.54 (m, 1H), 7.43 - 7.27 (m, 3H), 6.25 (s, 2H), 4.89 (d, *J* = 4.4 Hz, 1H), 4.48 (td, *J* = 8.9, 4.3 Hz, 1H), 2.56 (dd, *J* = 11.9, 8.9 Hz, 1H), 2.24 (dd, *J* = 11.8, 8.9 Hz, 1H), 1.60 (s, 3H), 1.59 (s, 3H).

### Example 49: (R)-(3-amino-2-fluorophenyl)((R)-5,5-dimethylpyrrolidin-2-yl)methanol hydrochloride

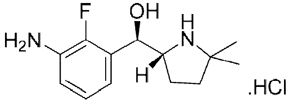

### (a) (E)-1-((3-Bromo-2-fluorophenyl)diazenyl)pyrrolidine

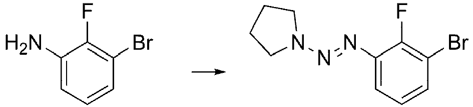

A mixture of 3-bromo-2-fluoroaniline (1.00 g, 5.26 mmol) and HCl (aq, conc, 4.0 mL, 132 mmol) was heated for 20 s with a heat-gun and allowed to cool to rt, and then cooled in an ice-bath. An ice-cold solution of NaNO₂ (508 mg, 7.37 mmol) in H₂O (2.5 mL) was quickly added and the ice-cooled mixture was stirred for 15 min. An ice-cold solution of pyrrolidine (2.19 mL, 26.3 mmol) in KOH (aq, 2 M, 16 mL) was rapidly added and the ice-cooled mixture was stirred for 40 min. The solid was collected, dried and recrystallized from EtOH to give the sub-title compound (1.20 g, 4.36 mmol, 83 %).

### (b) (E)-1-((2-Fluoro-3-iodophenyl)diazenyl)pyrrolidine

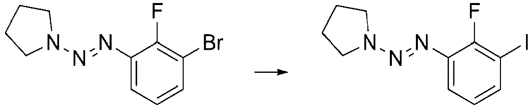

A mixture of (E)-1-((3-bromo-2-fluorophenyl)diazenyl)pyrrolidine (1.20 g, 4.36 mmol), Cul (125 mg, 0.65 mmol), NaI (1.31 g, 8.72 mmol), *N,N'*-dimethylethylenediamine (94 µL, 0.87 mmol) and dioxane (4 mL) was stirred at 140 °C for 3 h. Cul (60 mg, 0.32 mmol), NaI (0.60 g, 4.0 mmol), *N,N*'-dimethylethylenediamine (50 µL, 0.46 mmol) and dioxane (4 mL) were added and the mixture was stirred at 140 °C for 1 h, allowed to cool, diluted with CH₂Cl₂ and washed with H₂O. The aq phase was extracted with CH₂Cl₂ and the combined organic phases were washed with brine, dried (Na₂SO₄) and concentrated. The residue was purified by chromatography to give the sub-title compound (1.07 g, 3.43 mmol, 77 %).

### (c) tert-Butyl (R)-5-((R)-(2-fluoro-3-((E)-pyrrolidin-1-yldiazenyl)phenyl)(hydroxy)-methyl)-2,2-dimethylpyrrolidine-1-carboxylate

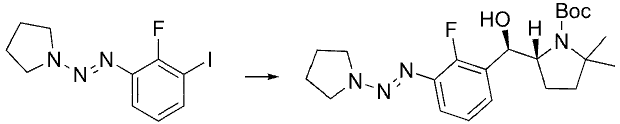

iPrMgCl (2 M in THF, 0.59 mL, 1.19 mmol) was added to a mixture of (*E*)-1-((2-fluoro-3-iodophenyl)diazenyl)pyrrolidine (380 mg, 1.19 mmol) and the mixture was stirred at -60 °C for 2.5 h. A solution of *tert*-butyl (*R*)-5-formyl-2,2-dimethylpyrrolidine-1-carboxylate, see Example 23, Step (d), (180 mg, 0.79 mmol) in THF (5.4 mL) was added dropwise at -60 °C. The mixture was allowed to come to rt, stirred at rt for 1 h, quenched with NH₄Cl (aq, sat, 5 mL) and diluted with H₂O and EtOAc. The phases were separated and the aq phase was extracted with EtOAc. The combined organic phases were washed with brine and dried (Na₂SO₄) and concentrated. The residue was purified by chromatography to give the sub-title compound (70 mg, 0.17 mmol, 21 %) along with the corresponding (*R,S*) isomer (218 mg, 0.52 mmol, 66 %).

### (d) tert-Butyl (R)-5-((R)-(3-azido-2-fluorophenyl)(hydroxy)methyl)-2,2-dimethyl-pyrrolidine-1-carboxylate

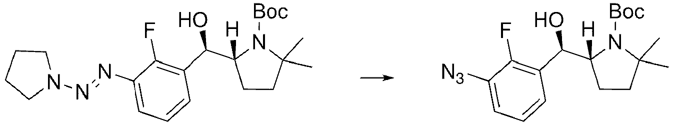

Trimethylsilyl azide (0.10 mL, 0.77 mmol) followed by trifluoroacetic acid (0.12 mL, 1.54 mmol) were added to an ice-cooled solution of *tert*-butyl (*R*)-5-((*R*)-(2-fluoro-3-((*E*)-pyrrolidin-1-yldiazenyl)phenyl)(hydroxy)methyl)-2,2-dimethylpyrrolidine-1-carboxylate (65 mg, 0.15 mmol) in CH₂Cl\₂ (2.4 mL). The ice-bath was removed and the mixture was stirred at rt for 1 h, quenched with NaHCO₃ (aq, sat, 3 mL) and stirred at rt for 10 min. The phases were separated and the aq phase was extracted with CH₂Cl₂. The combined organic phases were dried (MgSO₄) and concentrated to give sub-title compound (44 mg, 0.12 mmol, 78 %), which was used in the following step without further purification.

### (e) tert-Butyl (R)-5-((R)-(3-amino-2-fluorophenyl)(hydroxy)methyl)-2,2-dimethyl-pyrrolidine-1-carboxylate

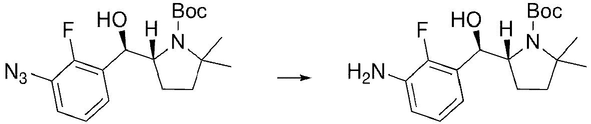

SmI₂ (43 mM in THF, 8.4 mL, 0.36 mmol), was added dropwise to a solution of *tert*-butyl (*R*)-5-((*R*)-(3-azido-2-fluorophenyl)(hydroxy)methyl)-2,2-dimethylpyrrolidine-1-carboxylate (44 mg, 0.12 mmol) in THF (1.2 mL) at rt. The mixture was stirred at rt for 30 min. H₂O followed by Na₂CO₃ (aq, sat) were added. The layers were separated and the aq phase was extracted with EtOAc. The combined organic phases were washed with brine and dried (Na₂SO₄) and concentrated. The residue was purified by chromatography to give the sub-title compound (35 mg, 0.10 mmol, 86 %).

### (f) (R)-(3-Amino-2-fluorophenyl)((R)-5,5-dimethylpyrrolidin-2-yl)methanol hydrochloride

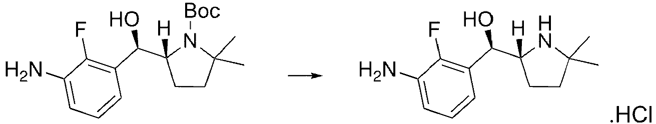

Trifluoroacetic acid (0.11 mL, 81 µmol) was added to an ice-cooled solution of *tert*-butyl (*R*)-5-((*R*)-(3-amino-2-fluorophenyl)(hydroxy)methyl)-2,2-dimethylpyrrolidine-1-carboxylate) (25 mg, 74 µmol) in CH₂Cl₂ (2 mL). The ice-bath was removed and the mixture was stirred at rt for 2 h. Another portion of trifluoroacetic acid (0.05 mL, 40 µmol) was added and the mixture was stirred at rt for 18 h and concentrated. The residue was taken up in EtOAc and the mixture was washed with NaOH (1 M, 1 mL) and brine, dried (Na₂SO₄) and concentrated. The residue was dissolved in Et₂O (2.5 mL) and HCl (2 M in Et₂O, 41 µL, 81 µmol) was added. The solids were collected and dried to give the title compound (15 mg, 55 µmol, 74 %).

¹H NMR (400 MHz, D₂O) δ 7.25 - 7.12 (m, 3H), 5.15 (d, *J* = 8.9 Hz, 1H), 4.21 - 4.09 (m, 1H), 2.08 - 1.77 (m, 4H), 1.54 (s, 3H), 1.46 (s, 3H).

### Example 50: (S)-(3-amino-2-fluorophenyl)((R)-5,5-dimethylpyrrolidin-2-yl)methanol hydrochloride

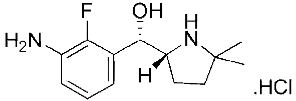

The title compound was prepared from *tert*-butyl (*R*)-5-((*S*)-(2-fluoro-3-((*E*)-pyrrolidin-1-yldiazenyl)phenyl)(hydroxymethyl)-2,2-dimethylpyrrolidine-1-carboxylate, see Example 49, step (c) , in accordance with the procedure described in Example 49, Steps (d) to (f). H NMR (400 MHz, D₂O) δ 7.18 - 7.10 (m, 1H), 7.10 - 7.01 (m, 2H), 5.30 (d, *J* = 4.8 Hz, 1H), 4.24 - 4.11 (m, 1H), 2.22 - 2.07 (m, 1H), 2.07 - 1.89 (m, 3H), 1.50 (s, 3H), 1.45 (s, 3H).

### Example 51: (S)-(4-Amino-3,5-difluorophenyl)((R)-5,5-dimethylpyrrolidin-2-yl)methanol hydrochloride

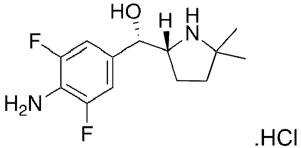

### (a) (E)-1-((2,6-difluoro-4-iodophenyl)diazenyl)pyrrolidine

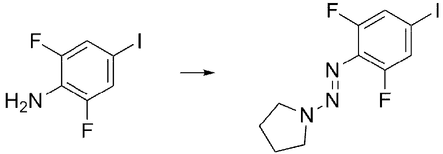

The sub-title compound was prepared in accordance with the procedure in Example 49, Step (a) from 2,6-difluoro-4-iodoaniline.

### (b) (R)-(4-amino-3,5-difluorophenyl)((R)-5,5-dimethylpyrrolidin-2-yl)methanol hydrochloride

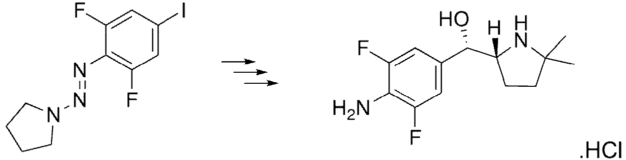

The title compound was prepared in accordance with the procedure in Example 49, Steps (c) to (f).

¹H NMR (400 MHz, CD₃OD) δ 7.04 - 6.86 (m, 2H), 4.97 - 4.78 (1H, overlapping with H₂O), 3.91 (ddd, *J* = 8.8, 7.8, 4.0 Hz, 1H), 2.23 - 2.10 (m, 1H), 1.99 - 1.74 (m, 3H), 1.49 (s, 3H), 1.45 (s, 3H).

### Biological examples

L6- myoblasts were grown in Dulbecco's Modified Eagle's Medium (DMEM) containing 4,5 g/l glucose supplemented with 10% fetal bovine serum, 2 mM L-Glutamine, 50 U/ml penicillin, 50 µg/ml streptomycin and 10 mM HEPES. Cells were plated at 1x 10⁵ cells per ml in 24- well plates. After reaching 90 % confluence the cells were grown in medium containing 2% FBS for 7 days where upon cells differentiated into myotubes.

### Biological example 1: Glucose uptake

Differentiated L6- myotubes were serum-starved overnight in medium containing 0,5 % fatty- acid free BSA and stimulated with agonist, final concentration 1x10⁻⁵. After 1 h 40 min cells were washed with warm, glucose free medium or PBS and another portion of agonist was added to glucose free medium. After 20 min the cells were exposed to 50 nM ³H-2- deoxy- glucose for another 10 min before washed in ice cold glucose free medium or PBS and lysed in 0,2 M NaOH for 1 h in 60° C. Cell lysate was mixed with scintillation buffer (Emulsifier Safe, Perkin Elmer and radioactivity detected in a β-counter (Tri- Carb 2800TR, Perkin Elmer). The activity for each compound is compared to that of isoproterenol. If a compound shows activity of more than 75 % of that of isoproterenol, the activity is denoted with +++, if it is between 75 and 50 % it is denoted with ++; if it is between 50 and 25 % it is denoted with +; if it less than 25 % it is denoted with -.

### Biological example 2: Measurement of intracellular cAMP levels

Differentiated cells were serum-starved overnight and stimulated with agonist, final concentration 1x10-⁵, for 15 min in stimulation buffer (HBSS supplemented with 1% BSA, 5 mM HEPES and 1 mM IBMX, pH 7,4) The medium was then aspirated and to end the reaction 100 µL of 95 % EtOH was added to each well of a 24- well plate and cells were kept in -20° C over night. The EtOH was let to evaporate and 500 µL of lysis buffer (1 % BSA, 5 mM HEPES and 0,3 % Tween- 20, pH 7,4) was added to each well before put in -80° C for 30 min and then kept in -20° C. Intracellular cAMP levels were detected using an alpha screen cAMP kit (6760635D from Perkin Elmer). The activity for each compound is compared to that of isoproterenol. If a compound shows activity of more than 75 % of that of isoproterenol, the activity is denoted with +++, if it is between 75 and 50 % it is denoted with ++; if it is between 50 and 25 % it is denoted with +; if it less than 25 % it is denoted with -.

Using the assays described in Biological Examples 1 and 2 the results shown in Tables 1 and 2 below were obtained (na = not available).

**Table 1**

| **Compound example no.** | **Biological example 1** | **Biological example 2** |
|---|---|---|
| **1** | **++** | **-** |
| **2** | **-** | **-** |
| **3** | **+** | **-** |
| **4** | **+** | **-** |
| **5** | **+** | **-** |
| **6** | **+++** | **-** |
| **7** | **+** | **-** |
| **8** | **++** | **-** |
| **9** | **++** | **-** |
| **10** | **+++** | **+** |
| **11** | **+++** | **-** |
| **12** | **+** | **-** |
| **13** | **-** | **-** |
| **14** | **+** | **-** |
| **15** | **+++** | **-** |
| **16** | **++** | **-** |
| **17** | **+** | **-** |
| **18** | **-** | **-** |
| **19** | **+++** | **-** |
| **20** | **+** | **-** |
| **21** | **++** | **-** |
| **22** | **-** | **-** |
| **23** | **+++** | **-** |
| **24** | **-** | **-** |
| **25** | **+++** | **-** |
| **26** | **+** | **-** |
| **27** | **++** | **-** |
| **28** | **-** | **-** |
| **29** | **++** | **-** |
| **30** | **+** | **-** |
| **31** | **++** | **+** |
| **32** | **-** | **-** |

**Table 2**

| **Compound example no.** | **Biological example 3** | **Biological example 4** |
|---|---|---|
| **33** | **+++** | **++** |
| **34** | **+** | **-** |
| **35** | **+++** | **+** |
| **36** | **+** | **-** |
| **37** | **-** | **-** |
| **38** | **-** | **-** |
| **39** | **-** | **-** |
| **40** | **+** | **-** |
| **41** | **+++** | **+** |
| **42** | **+** | **-** |
| **43** | **+** | **-** |
| **44** | **++** | **-** |
| **45** | **+++** | **-** |
| **46** | **-** | **-** |
| **47** | **++** | **-** |
| **48** | **+** | **-** |
| **49** | **+++** | **-** |
| **50** | **+** | **-** |
| **51** | **+++** | **na** |

## Claims

1. A compound of formula I 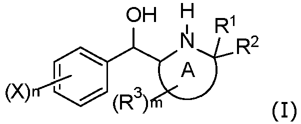 or a pharmaceutically acceptable salt thereof, wherein:
ring A represents a 4- to 8-membered heterocycloalkyl;
each R¹ and R² independently represents C₁₋₆ alkyl, C₂₋₆ alkenyl or C₂₋₆ alkynyl each optionally substituted by one or more halo;
or alternatively R¹ and R² may be linked together to form a 3-membered ring, which optionally is substituted by one or more groups independently selected from halo and C₁₋₆ alkyl, C₂₋₆ alkenyl or C₂₋₆ alkynyl each optionally substituted by one or more halo;
each R³ independently represents halo or C₁₋₆ alkyl, C₂₋₆ alkenyl or C₂₋₆ alkynyl each optionally substituted by one or more halo;
each X independently represents halo, R^{a}, -CN, -N₃, -N(R^{b})R^{c}, -NO₂, -ONO₂, -OR^{d}, -S(O)ₚR^{e} or -S(O)_{q}N(R^{f})R^{g};
R^{a} represents C₁₋₆ alkyl, C₂₋₆ alkenyl or C₂₋₆ alkynyl each optionally substituted by one or more groups independently selected from G¹;
each R^{b}, R^{c}, R^{d}, R^{e}, R^{f} and R^{g} independently represents H or C₁₋₆ alkyl, C₂₋₆ alkenyl or C₂₋₆ alkynyl each optionally substituted by one or more groups independently selected from G²;
or alternatively any of R^{b} and R^{c} and/or R^{f} and R^{g} may be linked together to form, together with the nitrogen atom to which they are attached, a 4- to 6-membered ring, which ring optionally contains one further heteroatom and which ring optionally is substituted by one or more groups independently selected from halo, C₁₋₃ alkyl, C₂₋₃ alkenyl or C₂₋₃ alkynyl each optionally substituted by one or more halo, and =O;
G¹ and G² represents halo, -CN, -N(R^{a1})R^{b1}, -OR^{c1}, -S(O)ₚR^{d1}, -S(O)_{q}N(R^{e1})R^{f1} or =O;
each R^{a1}, R^{b1}, R^{c1}, R^{d1}, R^{e1} and R^{f1} independently represents H or C₁₋₆ alkyl, C₂₋₆ alkenyl or C₂₋₆ alkynyl each optionally substituted by one or more halo;
or alternatively any of R^{a1} and R^{b1} and/or R^{e1} and R^{f1} may be linked together to form, together with the nitrogen atom to which they are attached, a 4- to 6-membered ring, which ring optionally contains one further heteroatom and which ring optionally is substituted by one or more groups independently selected from halo, C₁₋₃ alkyl, C₂₋₃ alkenyl or C₂₋₃ alkynyl each optionally substituted by one or more halo, and =O;
n represents 0 to 5;
each p independently represents 0, 1 or 2;
each q independently represents 1 or 2;
m represents 0 to 11, as appropriate,
wherein alkyl, alkenyl and alkynyl groups may be straight-chain or, when there is a sufficient number of carbon atoms, be branched-chain and/or cyclic or part cyclic,
with the proviso that the compound of formula I is not a compound selected from the list consisting of:
(*S*)-((*S*)-5,5-dimethylpyrrolidin-2-yl)(4-(methylthio)phenyl)methanol;
(3,4-dichlorophenyl)(5,5-dimethylpyrrolidin-2-yl)methanol;
(5,5-dimethylpyrrolidin-2-yl)(p-tolyl)methanol;
(4-chlorophenyl)(5,5-dimethylpyrrolidin-2-yl)methanol;
3-((5,5-dimethylpyrrolidin-2-yl)(hydroxy)methyl)benzonitrile;
(5,5-dimethylpyrrolidin-2-yl)(m-tolyl)methanol;
(5,5-dimethylpyrrolidin-2-yl)(3-(trifluoromethyl)phenyl)methanol;
(5,5-dimethylpyrrolidin-2-yl)(phenyl)methanol;
(2,4-dichlorophenyl)(5,5-dimethylpyrrolidin-2-yl)methanol;
(2,6-dichlorophenyl)(5,5-dimethylpyrrolidin-2-yl)methanol;
(3,4-dichlorophenyl)(6,6-dimethylpiperidin-2-yl)methanol;
(3-chlorophenyl)(6,6-dimethylpiperidin-2-yl)methanol;
(2,4-dimethylphenyl)(5,5-dimethylpyrrolidin-2-yl)methanol;
(3-chlorophenyl)(5,5-dimethylpyrrolidin-2-yl)methanol;
(4-chlorophenyl)(6,6-dimethylpiperidin-2-yl)methanol;
(*R**)-(4-chlorophenyl)((*S**)-5,5-dimethylpyrrolidin-2-yl)methanol;
(*R**)-(4-chlorophenyl)((*R**)-5,5-dimethylpyrrolidin-2-yl)methanol;
(*R**)-(3,4-dichlorophenyl)((*S**)-5,5-dimethylpyrrolidin-2-yl)methanol;
(*R**)-(3,4-dichlorophenyl)((*R**)-5,5-dimethylpyrrolidin-2-yl)methanol;
(*R**)-(3-chlorophenyl)((*S**)-5,5-dimethylpyrrolidin-2-yl)methanol;
(*R**)-(3-chlorophenyl)((*R**)-5,5-dimethylpyrrolidin-2-yl)methanol;
*(R*)*-(3-chlorophenyl)(*(R*)*-6,6-dimethylpiperidin-2-yl)methanol;
*(R*)*-((*S**)-5,5-dimethylpyrrolidin-2-yl)(3-(trifluoromethyl)phenyl)methanol;
(*R**)*-*((*R**)-5,5-dimethylpyrrolidin-2-yl)(3-(trifluoromethyl)phenyl)methanol;
(*R**)-((*S**)-5,5-dimethylpyrrolidin-2-yl)(phenyl)methanol;
(*R**)*-*((*R**)-5,5-dimethylpyrrolidin-2-yl)(phenyl)methanol;
*(R*)-((5*)*-5,5-dimethylpyrrolidin-2-yl)(m-tolyl)methanol;
*(R*)*-((*R**)-5,5-dimethylpyrrolidin-2-yl)(m-tolyl)methanol;
(*R**)-(2,6-dichlorophenyl)((*S**)-5,5-dimethylpyrrolidin-2-yl)methanol;
*(R)*-(2,6-dichlorophenyl)*((R)*-5,5-dimethylpyrrolidin-2-yl)methanol;
(*R**)-(3,4-dichlorophenyl)((*S**)-6,6-dimethylpiperidin-2-yl)methanol;
*(R*)*-(3,4-dichlorophenyl)(*(R**)-6,6-dimethylpiperidin-2-yl)methanol;
(*R**)-(3-chlorophenyl)(*(S*)*-6,6-dimethylpiperidin-2-yl)methanol;
*(R*)-(2,4-dichlorophenyl)((S*)-5,5-dimethylpyrrolidin-2-yl)methanol;*
(*R**)-(2,4-dichlorophenyl)((*R**)-5,5-dimethylpyrrolidin-2-yl)methanol; and
3-((*R**)-((*R**)-5,5-dimethylpyrrolidin-2-yl)(hydroxy)methyl)benzonitrile.

2. The compound according to Claim 1, wherein the compound is a compound of formula (IA) 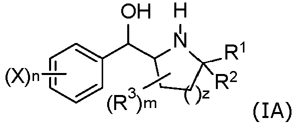 or a pharmaceutically acceptable salt thereof, wherein:
R¹, R², R³, X and n are as defined in Claim 1;
z represents 1 or 2; and
when z represents 1 then m represents 0 to 5, and when z represents 2 then m represents 0 to 7.

3. A compound as defined in any one of Claims 1 and 2, wherein either:
each R¹ and R² independently represents C₁₋₃ alkyl optionally substituted by one or more halo; or
R¹ and R² are linked together to form a 3-membered ring, which is optionally substituted by one or more groups independently selected from halo and C₁₋₆ alkyl optionally substituted by one or more halo.

4. A compound as defined in any one of the preceding claims, wherein each X independently represents halo, OH, CN, CF₃ or NH₂.

5. A compound as defined in any one of the preceding claims, wherein n represents 1, 2 or 3.

6. A compound as defined in any one of the preceding claims, wherein m represents 0.

7. A compound as defined in any one of the preceding claims, wherein the compound is selected from the group consisting of:
(A)
(*R*)-((*R*)-6,6-Dimethylpiperidin-2-yl)(3-fluorophenyl)methanol;
(*S*)-((*R*)-6,6-Dimethylpiperidin-2-yl)(3-fluorophenyl)methanol;
3-((*R)*-((*R*)-6,6-Dimethylpiperidin-2-yl)(hydroxy)methyl)phenol;
3-((*S*)-((R)-6,6-Dimethylpiperidin-2-yl)(hydroxy)methyl)phenol;
(*R*)-(2-Chlorophenyl)((*R*)-6,6-dimethylpiperidin-2-yl)methanol;
(*S*)-(2-Chlorophenyl)((*R*)-6,6-dimethylpiperidin-2-yl)methanol;
(*R*)-(2-Chlorophenyl)((*R*)-6,6-dimethylpiperidin-2-yl)methanol;
(*S*)-(2-Chlorophenyl)((*R*)-6,6-dimethylpiperidin-2-yl)methanol;
(*R*)-((R)-6,6-Dimethylpiperidin-2-yl)(2-fluorophenyl)methanol;
(*S*)-((R)-6,6-Dimethylpiperidin-2-yl)(2-fluorophenyl)methanol;
(*R*)-((R)-5,5-Dimethylpyrrolidin-2-yl)(3-fluorophenyl)methanol;
(*S*)-((R)-5,5-Dimethylpyrrolidin-2-yl)(3-fluorophenyl)methanol;
(*R*)-((S)-5,5-Dimethylpyrrolidin-2-yl)(3-fluorophenyl)methanol;
(*S*)-((*S*)-5,5-Dimethylpyrrolidin-2-yl)(3-fluorophenyl)methanol;
(*R*)-((*R*)-5,5-Oimethylpyrrolidin-2-yl)(hydroxy)methyl)phenol;
(*S*)-((*R*)*-*5,5-Dimethylpyrrolidin-2-yl)(hydroxy)methyl)phenol;
(*R*)-((*S*)-5,5-Dimethylpyrrolidin-2-yl)(hydroxy)methyl)phenol;
(*S*)-((*S*)-5,5-Dimethylpyrrolidin-2-yl)(hydroxy)methyl)phenol;
(*R*)-((*R*)-5,5-Dimethylpyrrolidin-2-yl)(2-fluorophenyl)methanol;
(*S*)-((*R*)-5,5-Dimethylpyrrolidin-2-y)(2-fluorophenyl)methanol;
(*R*)*-*((*R*)*-5,5-Dimethylpyrrolidin-2-yl)(4-fluorophenyl)methanol;*
(*S*)-((*R*)-5,5-Dimethylpyrrolidin-2-yl)(4-fluorophenyl)methanol;
(*R*)-(2-Chlorophenyl)((*R*)-5,5-dimethylpyrrolidin-2-yl)methanol;
(*S*)-(2-Chlorophenyl)((*R*)*-5*,5-dimethylpyrrolidin-2-yl)methanol;
(*R*)-(3-Chlorophenyl)((*R*)-5,5-dimethylpyrrolidin-2-yl)methanol;
(*S*)*-*(2-Chlorophenyl)((*R*)-5,5-dimethylpyrrolidin-2-yl)methanol;
(*R*)-((*R*)-5,5-Dipropylpyrrolidin-2-yl)(3-fluorophenyl)methanol;
(*S*)-((*R*)*-*5*,*5-Dipropylpyrrolidin-2-yl)(3-fluorophenyl)methanol;
(*R*)-(3-Fluorophenyl)((*R*)-4-azaspiro[2.4]heptan-5-yl)methanol; and
(*S*)-(3-Fluorophenyl)((*R*)-4-azaspiro[2.4]heptan-5-yl)methanol,
and pharmaceutically acceptable salts thereof; and
(B)
(*R*)-((*R*)-4,4-dimethylazetidin-2-yl)(3-fluorophenyl)methanol;
(*S*)-((*S*)-4,4-dimethylazetidin-2-yl)(2-fluorophenyl)methanol;
(*R*)-((*R*)-4,4-dimethylazetidin-2-yl)(2-fluorophenyl)methanol;
(*S*)-((*R*)*-*4,4-dimethylazetidin-2-yl)(2-fluorophenyl)methanol;
(*R*)-((*S*)-4,4-dimethylazetidin-2-yl)(2-fluorophenyl)methanol;
(*S*)-((*R*)-4,4-dimethylazetidin-2-yl)(2-fluorophenyl)methanol;
(*R*)-((*S*)-4,4-dimethylazetidin-2-yl)(3-fluorophenyl)methanol;
(*S*)-((*S*)-4,4-dimethylazetidin-2-yl)(3-fluorophenyl)methanol;
(*R)*-((S)-6,6-dimethylpiperidin-2-yl)(2-fluorophenyl)methanol;
(*S*)-((*S*)-6,6-dimethylpiperidin-2-yl)(2-fluorophenyl)methanol;
(*S*)-((*S*)-6,6-dimethylpiperidin-2-yl)(3-fluorophenyl) methanol;
(*R*)-((*S*)-6,6-dimethylpiperidin-2-yl)(3-fluorophenyl)methanol;
(*R*)*-*(3-chlorophenyl)((*R*)-*4*,4-dimethylazetidin-2-yl)methanol;
(*S*)-(3-chlorophenyl)((*R*)-4,4-dimethylazetidin-2-yl)methanol;
(*R*)-(3-chlorophenyl)((*S*)-4,4-dimethylazetidin-2-yl)methanol;
(*S*)-(3-chlorophenyl)((*S*)-4,4-dimethylazetidin-2-yl)methanol;
(*R*)-(3-amino-2-fluorophenyl)((*R*)-5,5-dimethylpyrrolidin-2-yl)methanol;
(*S*)-(3-amino-2-fluorophenyl)((*R*)-5,5-dimethylpyrrolidin-2-yl)methanol; and
(*S*)-(4-Amino-3,5-difluorophenyl)((*R*)-5,5-dimethylpyrrolidin-2-yl)methanol,
and pharmaceutically acceptable salts thereof.

8. A compound as defined in any one of Claims 1 to 7, but without the proviso, for use in medicine.

9. A pharmaceutical composition comprising a compound as defined in any one of Claims 1 to 7, and optionally one or more pharmaceutically acceptable adjuvant, diluent and/or carrier,
with the proviso that the compound of formula I is not a compound selected from the list consisting of:
(*S*)-((*S*)-5,5-dimethylpyrrolidin-2-yl)(4-(methylthio)phenyl)methanol;
(3,4-dichlorophenyl)(5,5-dimethylpyrrolidin-2-yl)methanol;
(5,5-dimethylpyrrolidin-2-yl)(*p*-tolyl)methanol;
(4-chlorophenyl)(5,5-dimethylpyrrolidin-2-yl)methanol;
3-((5,5-dimethylpyrrolidin-2-yl)(hydroxy)methyl)benzonitrile;
(5,5-dimethylpyrrolidin-2-yl)(m-tolyl)methanol;
(5,5-dimethylpyrrolidin-2-yl)(3-(trifluoromethyl)phenyl)methanol;
(5,5-dimethylpyrrolidin-2-yl)(phenyl)methanol;
(2,4-dichlorophenyl)(5,5-dimethylpyrrolidin-2-yl)methanol;
(2,6-dichlorophenyl)(5,5-dimethylpyrrolidin-2-yl)methanol;
(3,4-dichlorophenyl)(6,6-dimethylpiperidin-2-yl)methanol;
(3-chlorophenyl)(6,6-dimethylpiperidin-2-yl)methanol;
(2,4-dimethylphenyl)(5,5-dimethylpyrrolidin-2-yl)methanol;
(3-chlorophenyl)(5,5-dimethylpyrrolidin-2-yl)methanol;
(4-chlorophenyl)(6,6-dimethylpiperidin-2-yl)methanol;
(*R**)-(4-chlorophenyl)((*S**)-5,5-dimethylpyrrolidin-2-yl)methanol;
(*R**)-(4-chlorophenyl)((*R**)-5,5-dimethylpyrrolidin-2-yl)methanol;
(*R**)-(3,4-dichlorophenyl)((*S**)-5,5-dimethylpyrrolidin-2-yl)methanol;
(*R**)-(3,4-dichlorophenyl)((*R**)-5,5-dimethylpyrrolidin-2-yl)methanol;
(*R**)-(3-chlorophenyl)((*S**)-5,5-dimethylpyrrolidin-2-yl)methanol;
(*R**)-(3-chlorophenyl)((*R**)-5,5-dimethylpyrrolidin-2-yl)methanol;
(*R**)-(3-chlorophenyl)((*R**)-6,6-dimethylpiperidin-2-yl)methanol;
(*R**)-((*S**)*-*5,5-dimethylpyrrolidin-2-yl)(3-(trifluoromethyl)phenyl)methanol;
(*R**)-((*R**)-5,5-dimethylpyrrolidin-2-yl)(3-(trifluoromethyl)phenyl)methanol;
(*R**)-((*S**)-5,5-dimethylpyrrolidin-2-yl)(phenyl)methanol;
(*R**)-((*R**)-5,5-dimethylpyrrolidin-2-yl)(phenyl)methanol;
(*R**)*-*((*S**)*-5,5-dimethylpyrrolidin-2-yl)(m-tolyl)methanol;*
(*R**)-((*R**)-5,5-dimethylpyrrolidin-2-yl)(*m*-tolyl)methanol;
(*R**)-(2,6-dichlorophenyl)((*S**)-5,5-dimethylpyrrolidin-2-yl)methanol;
(*R*)*-*(2,6-dichlorophenyl)*((R)*-5,5-dimethylpyrrolidin-2-yl)methanol;
(*R**)-(3,4-dichlorophenyl)((*S**)-6,6-dimethylpiperidin-2-yl)methanol;
(*R**)-(3,4-dichlorophenyl)((*R**)-6,6-dimethylpiperidin-2-yl)methanol ;
(*R*-(3-chlorophenyl)((*S**)-6,6-dimethylpiperidin-2-yl)methanol;
(*R**)-(2,4-dichlorophenyl)((*S**)-5,5-dimethylpyrrolidin-2-yl)methanol;
(*R**)-(2,4-dichlorophenyl)((*R**)-5,5-dimethylpyrrolidin-2-yl)methanol; and
3-((*R**)-((*R**)-5,5-dimethylpyrrolidin-2-yl)(hydroxy)methyl)benzonitrile.

10. A compound as defined in any one of Claims 1 to 7, but without the proviso, for use in the treatment of hyperglycaemia or a disorder **characterized by** hyperglycaemia.

11. The compound for use according to Claim 10, wherein the hyperglycaemia or disorder **characterised by** hyperglycaemia is, or is **characterised by** the patient displaying, severe insulin resistance.

12. The compound for use according to any one of Claims 10 and 11, wherein the disorder **characterised by** hyperglycaemia is selected from the group consisting of Type 2 diabetes, Rabson-Mendenhall syndrome, Donohue's syndrome (leprechaunism), Type A and Type B syndromes of insulin resistance, the HAIR-AN (hyperandrogenism, insulin resistance, and acanthosis nigricans) syndromes, pseudoacromegaly, and lipodystrophy.

13. A combination product comprising:
(a) a compound as defined in any one of Claims 1 to 7 but without the proviso; and
(b) one or more other therapeutic agent that is useful in the treatment of hyperglycaemia or a disorder **characterised by** hyperglycaemia,
wherein each of components (a) and (b) is formulated in admixture, optionally with one or more pharmaceutically-acceptable adjuvant, diluent or carrier.

14. A kit-of-parts comprising:
(a) a pharmaceutical composition comprising a compound as defined in any one of Claims 1 to 7, and
(b) one or more other therapeutic agent that is useful in the treatment of hyperglycaemia or a disorder **characterised by** hyperglycaemia, optionally in admixture with one or more pharmaceutically-acceptable adjuvant, diluent or carrier,
which components (a) and (b) are each provide in a form that is suitable for administration in conjunction with the other.

15. A compound as defined in any one of Claims 1 to 7, but without the proviso, for use in the treatment of a non-alcoholic fatty liver disease.

16. A process for the preparation of a compound as defined in any one of Claims 1 to 7, comprising the step of:
(i) reaction of a compound of formula II 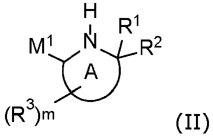
wherein ring A, R¹, R², R³ and m are as defined in any one of Claims 1 to 7, and wherein M¹ represents a suitable metal or metal halide, with a compound of formula III 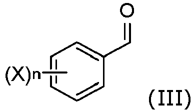
wherein n and X are as defined in any one of Claims 1 to 7;
(ii) reaction of a compound of formula IV 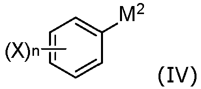
wherein n and X are as defined in any one of Claims 1 to 7, and wherein M² represents a suitable metal or metal halide, with a compound of formula V 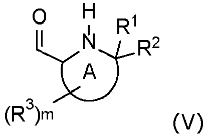
wherein ring A, R¹, R², R³ and m are as defined in any one of Claims 1 to 7;
(iii) for compounds wherein at least one X is present and represents -OH, deprotection of a compound of formula VI 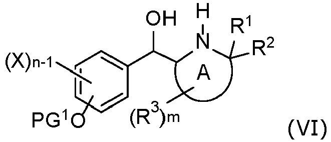 wherein ring A, R¹, R², R³, n and m are as defined in any one of Claims 1 to 7, and PG¹ represents a suitable protecting group;
(iv) for compounds wherein at least one X is present and represents NH₂, deprotection of a compound of formula VII 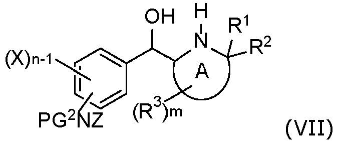 wherein ring A, R¹, R², R³, n and m are as defined in any one of Claims 1 to 7, and Z represents H or PG³, wherein PG² and PG³ each represents a suitable protecting group;
(v) for compounds wherein at least one X is present and represents NH₂, reduction of a compound of formula VIII 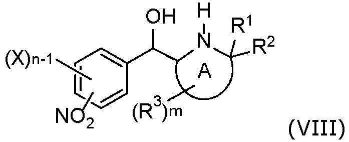 wherein ring A, R¹, R², R³, n, and m are as defined in any one of Claims 1 to 7;
(vi) deprotection of a compound of formula IX 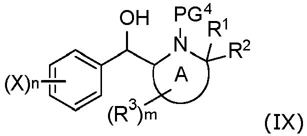 wherein ring A, X, R¹, R², R³, n and m are as defined in any one of Claims 1 to 7, and PG⁴ represents a suitable protecting group; or
(vii) reduction of a compound of formula XI 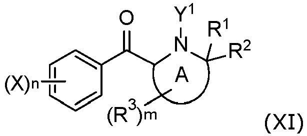 wherein ring A, X, R¹, R², R³, n and m are as defined in any one of Claims 1 to 7 and Y¹ represents H or PG³, wherein PG⁵ is a suitable protecting group, in the presence of a suitable catalyst and in the presence of a suitable solvent.

## Patentansprüche

1. Verbindung der Formel I 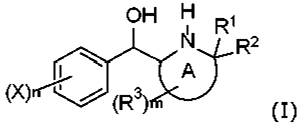 oder ein pharmazeutisch verträgliches Salz davon, wobei:
Ring A ein 4- bis 8-gliedriges Heterocycloalkyl darstellt;
jeder R¹ und R² unabhängig voneinander C₁₋₆-Alkyl, C₂₋₆-Alkenyl oder C₂₋₆-Alkinyl darstellt, jeweils gegebenenfalls substituiert durch ein oder mehrere Halogene;
oder alternativ R¹ und R² miteinander verbunden sein können, um einen 3-gliedrigen Ring zu bilden, der gegebenenfalls durch eine oder mehrere Gruppen substituiert ist, unabhängig voneinander ausgewählt aus Halogen und C₁₋₆-Alkyl, C₂₋₆-Alkenyl oder C₂₋₆-Alkinyl, jeweils gegebenenfalls substituiert durch ein oder mehrere Halogen;
jeder R³ unabhängig voneinander ein Halogen oder C₁₋₆-Alkyl, C₂₋₆-Alkenyl oder C₂₋₆-Alkinyl darstellt, wobei diese jeweils gegebenenfalls durch ein oder mehrere Halogen substituiert sind;
jedes X unabhängig voneinander Halogen, R^{a}, -CN, -N₃, -N(R^{b})R^{c}, -NO₂, - ONO₂, -OR^{d}, -S(O)_{P}R^{e} oder -S(O)_{q}N(R^{f})R^{g} darstellt;
R^{a} ein C₁₋₆-Alkyl, C₂₋₆-Alkenyl oder C₂₋₆-Alkinyl darstellt, die jeweils gegebenenfalls durch eine oder mehrere Gruppen substituiert sind, die unabhängig voneinander aus G¹ ausgewählt sind;
jedes R^{b}, R^{c}, R^{d}, R^{e}, R^{f} und R^{g} unabhängig voneinander für H oder C₁₋₆-Alkyl, C₂₋₆-Alkenyl oder C₂₋₆-Alkinyl darstellt, jeweils gegebenenfalls substituiert durch eine oder mehrere Gruppen, unabhängig voneinander ausgewählt aus G²;
oder alternativ jeder von R^{b} und R^{c} und/oder R^{f} und R^{g} miteinander verbunden sein können, um zusammen mit dem Stickstoffatom, an das sie gebunden sind, einen 4- bis 6-gliedrigen Ring zu bilden, wobei der Ring gegebenenfalls ein weiteres Heteroatom enthält und wobei der Ring gegebenenfalls durch eine oder mehrere Gruppen substituiert ist, die unabhängig voneinander ausgewählt sind aus Halogen, C₁₋₃-Alkyl, C₂₋₃-Alkenyl oder C₂₋₃-Alkinyl, jeweils gegebenenfalls substituiert durch ein oder mehrere Halogene, und =O;
G¹ und G² Halogen, -CN, -N(R^{a1})R^{b1}, -OR^{c1}, -S(O)_{P}R^{d1}, -S(O)_{q}N(R^{e1})R^{f1} oder =O darstellen;
jedes R^{a1}, R^{b1}, R^{c1}, R^{d1}, R^{e1} und R^{f1} unabhängig voneinander H oder C₁₋₆-Alkyl, C₂₋₆-Alkenyl oder C₂₋₆-Alkinyl darstellt, jeweils gegebenenfalls substituiert durch ein oder mehrere Halogen;
oder alternativ jeder von R^{a1} und R^{b1} und/oder R^{e1} und R^{f1} miteinander verbunden sein können, um zusammen mit dem Stickstoffatom, an das sie gebunden sind, einen 4- bis 6-gliedrigen Ring zu bilden, wobei der Ring gegebenenfalls ein weiteres Heteroatom enthält und wobei der Ring gegebenenfalls durch eine oder mehrere Gruppen substituiert ist, die unabhängig voneinander ausgewählt sind aus Halogen, C₁₋₃-Alkyl, C₂₋₃-Alkenyl oder C₂₋₃-Alkinyl, jeweils gegebenenfalls substituiert durch ein oder mehrere Halogene, und =O;
n 0 bis 5 darstellt;
jedes p unabhängig voneinander 0, 1 oder 2 darstellt;
jedes q unabhängig voneinander 1 oder 2 darstellt;
m 0 bis 11 darstellt, wie angemessen,
wobei Alkyl-, Alkenyl- und Alkinylgruppen geradkettig oder, wenn eine ausreichende Anzahl von Kohlenstoffatomen vorhanden ist, verzweigtkettig und/oder cyclisch oder teilcyclisch sein können,
mit der Maßgabe, dass die Verbindung der Formel I keine Verbindung ist, ausgewählt aus der Liste zusammengesetzt aus:
(*S*)-((*S*)-5,5-Dimethylpyrrolidin-2-yl)(4-(methylthio)phenyl)methanol;
(3,4-Dichlorphenyl)(5,5-dimethylpyrrolidin-2-yl)methanol;
(5,5-Dimethylpyrrolidin-2-yl)(p-tolyl)methanol;
(4-Chlorphenyl)(5,5-dimethylpyrrolidin-2-yl)methanol;
3-((5,5-Dimethylpyrrolidin-2-yl)(hydroxy)methyl)benzonitril;
(5,5-Dimethylpyrrolidin-2-yl)(m-tolyl)methanol;
(5,5-Dimethylpyrrolidin-2-yl)(3-(trifluormethyl)phenyl)methanol;
(5,5-Dimethylpyrrolidin-2-yl)(phenyl)methanol;
(2,4-Dichlorphenyl)(5,5-dimethylpyrrolidin-2-yl)methanol;
(2,6-Dichlorphenyl)(5,5-dimethylpyrrolidin-2-yl)methanol;
(3,4-Dichlorphenyl)(6,6-dimethylpiperidin-2-yl)methanol;
(3-Chlorphenyl)(6,6-dimethylpiperidin-2-yl)methanol;
(2,4-Dimethylphenyl)(5,5-dimethylpyrrolidin-2-yl)methanol;
(3-Chlorphenyl)(5,5-dimethylpyrrolidin-2-yl)methanol;
(4-Chlorphenyl)(6,6-dimethylpiperidin-2-yl)methanol;
(*R**)-(4-Chlorphenyl)((*S**)-5,5-dimethylpyrrolidin-2-yl)methanol;
(*R**)-(4-Chlorphenyl)((*R**)-5,5-dimethylpyrrolidin-2-yl)methanol;
(*R**)-(3,4-Dichlorphenyl)((*S**)-5,5-dimethylpyrrolidin-2-yl)methanol;
(*R**)-(3,4-Dichlorphenyl)((*R**)-5,5-dimethylpyrrolidin-2-yl)methanol;
(*R**)-(3-Chlorphenyl)((*S**)-5,5-dimethylpyrrolidin-2-yl)methanol;
(*R**)-(3-Chlorphenyl)((*R**)-5,5-dimethylpyrrolidin-2-yl)methanol;
(*R**)-(3-Chlorphenyl)((*R**)-6,6-dimethylpiperidin-2-yl)methanol;
(*R**)-((*S**)-5,5-Dimethylpyrrolidin-2-yl)(3-(trifluormethyl)phenyl)methanol;
(*R**)-((*R**)-5,5-Dimethylpyrrolidin-2-yl)(3-(trifluormethyl)phenyl)methanol;
(*R**)-((*S**)-5,5-Dimethylpyrrolidin-2-yl)(phenyl)methanol;
(*R**)-((*R**)-5,5-Dimethylpyrrolidin-2-yl)(phenyl)methanol;
(*R**)-((*S**)-5,5-Dimethylpyrrolidin-2-yl)(m-tolyl)methanol;
(*R**)-((*R**)-5,5-Dimethylpyrrolidin-2-yl)(m-tolyl)methanol;
(*R**)-(2,6-Dichlorphenyl)((*S**)-5,5-dimethylpyrrolidin-2-yl)methanol;
(*R*)-(2,6-Dichlorphenyl)((*R*)-5,5-dimethylpyrrolidin-2-yl)methanol;
(*R**)-(3,4-Dichlorphenyl)((*S**)-6,6-dimethylpiperidin-2-yl)methanol;
(*R**)-(3,4-Dichlorphenyl)((*R**)-6,6-dimethylpiperidin-2-yl)methanol;
(*R**)-(3-chlorphenyl)((*S**)-6,6-dimethylpiperidin-2-yl)methanol;
(*R**)-(2,4-Dichlorphenyl)((*S**)-5,5-dimethylpyrrolidin-2-yl)methanol;
(*R**)-(2,4-Dichlorphenyl)((*R**)-5,5-dimethylpyrrolidin-2-yl)methanol; und 3-((*R**)-((*R**)-5,5-Dimethylpyrrolidin-2-yl)(hydroxy)methyl)benzonitril.

2. Verbindung nach Anspruch 1, wobei die Verbindung eine Verbindung der Formel (IA) ist 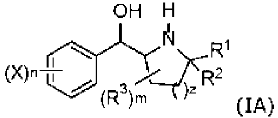 oder ein pharmazeutisch verträgliches Salz davon, wobei:
R¹, R², R³, X und n wie in Anspruch 1 definiert sind;
z 1 oder 2 darstellt; und
wenn z 1 darstellt, dann stellt m 0 bis 5 dar, und wenn z 2 darstellt, dann stellt m 0 bis 7 dar.

3. Verbindung nach einem der Ansprüche 1 und 2, wobei entweder:
R¹ und R² jeweils unabhängig voneinander ein C₁₋₃-Alkyl darstellen, der gegebenenfalls durch ein oder mehrere Halogene substituiert ist; oder
R¹ und R² miteinander verbunden sind, um einen 3-gliedrigen Ring zu bilden, der gegebenenfalls durch eine oder mehrere Gruppen substituiert ist, die unabhängig voneinander aus Halogen und C₁₋₆-Alkyl, das gegebenenfalls durch ein oder mehrere Halogenatome substituiert ist, ausgewählt sind.

4. Verbindung nach einem der vorstehenden Ansprüche, wobei jedes X unabhängig voneinander Halogen, OH, CN, CF₃ oder NH₂ darstellt.

5. Verbindung nach einem der vorstehenden Ansprüche, wobei n 1, 2 oder 3 darstellt.

6. Verbindung nach einem der vorstehenden Ansprüche, wobei m 0 darstellt.

7. Verbindung nach einem der vorstehenden Ansprüche, wobei die Verbindung aus der Gruppe ausgewählt ist, bestehend aus:
(A)
(*R*)-((*R*)-6,6-Dimethylpiperidin-2-yl)(3-fluorphenyl)methanol;
(*S*)-((*R*)-6,6-Dimethylpiperidin-2-yl)(3-fluorphenyl)methanol;
3-((*R*)-((*R*)-6,6-Dimethylpiperidin-2-yl)(hydroxy)methyl)phenol;
3-((*S*)-((*R*)-6,6-Dimethylpiperidin-2-yl)(hydroxy)methyl)phenol;
(*R*)-(2-Chlorphenyl)((*R*)-6,6-dimethylpiperidin-2-yl)methanol;
(*S*)-(2-Chlorphenyl)((*R*)-6,6-dimethylpiperidin-2-yl)methanol;
(*R*)-(2-Chlorphenyl)((*R*)-6,6-dimethylpiperidin-2-yl)methanol;
(*S*)-(2-Chlorphenyl)((*R*)-6,6-dimethylpiperidin-2-yl)methanol;
(*R*)-((*R*)-6,6-Dimethylpiperidin-2-yl)(2-fluorphenyl)methanol;
(*S*)-((*R*)-6,6-Dimethylpiperidin-2-yl)(2-fluorphenyl)methanol;
(*R*)-((*R*)-5,5-Dimethylpyrrolidin-2-yl)(3-fluorphenyl)methanol;
(*S*)-((*R*)-5,5-Dimethylpyrrolidin-2-yl)(3-fluorphenyl)methanol;
(*R*)-((*S*)-5,5-Dimethylpyrrolidin-2-yl)(3-fluorphenyl)methanol;
(*S*)-((*S*)-5,5-Dimethylpyrrolidin-2-yl)(3-fluorphenyl)methanol;
(*R*)-((*R*)-5,5-Dimethylpyrrolidin-2-yl)(hydroxy)methyl)phenol;
(*S*)-((*R*)-5,5-Dimethylpyrrolidin-2-yl)(hydroxy)methyl)phenol;
(*R*)-((*S*)-5,5-Dimethylpyrrolidin-2-yl)(hydroxy)methyl)phenol;
(*S*)-((*S*)-5,5-Dimethylpyrrolidin-2-yl)(hydroxy)methyl)phenol;
(*R*)-((*R*)-5,5-Dimethylpyrrolidin-2-yl)(2-fluorphenyl)methanol;
(*S*)-((*R*)-5,5-Dimethylpyrrolidin-2-yl)(2-fluorphenyl)methanol;
(*R*)-((*R*)-5,5-Dimethylpyrrolidin-2-yl)(4-fluorphenyl)methanol;
(*S*)-((*R*)-5,5-Dimethylpyrrolidin-2-yl)(4-fluorphenyl)methanol;
(*R*)-(2-Chlorphenyl)((R)-5,5-dimethylpyrrolidin-2-yl)methanol;
(*S*)-(2-Chlorphenyl)((R)-5,5-dimethylpyrrolidin-2-yl)methanol;
(*R*)-(3-Chlorphenyl)((R)-5,5-dimethylpyrrolidin-2-yl)methanol;
(*S*)-(2-Chlorphenyl)((R)-5,5-dimethylpyrrolidin-2-yl)methanol;
(*R*)-((*R*)-5,5-Dipropylpyrrolidin-2-yl)(3-fluorphenyl)methanol;
(*S*)-((*R*)-5,5-Dipropylpyrrolidin-2-yl)(3-Fluorphenyl)methanol;
(*R*)-(3-Fluorphenyl)((R)-4-azaspiro[2.4]heptan-5-yl)methanol; und
(*S*)-(3-Fluorphenyl)((R)-4-azaspiro[2.4]heptan-5-yl)methanol,
und pharmazeutisch verträglichen Salzen davon. und
(B)
(*R*)-((*R*)-4,4-Dimethylazetidin-2-yl)(3-fluorphenyl)methanol;
(*S*)-((*S*)-4,4-Dimethylazetidin-2-yl)(2-fluorphenyl)methanol;
(*R*)-((*R*)-4,4-Dimethylazetidin-2-yl)(2-fluorphenyl)methanol;
(*S*)-((*R*)-4,4-Dimethylazetidin-2-yl)(2-fluorphenyl)methanol;
(*R*)-((*S*)-4,4-Dimethylazetidin-2-yl)(2-fluorphenyl)methanol;
(*S*)-((*R*)-4,4-Dimethylazetidin-2-yl)(2-fluorphenyl)methanol;
(*R*)-((*S*)-4,4-Dimethylazetidin-2-yl)(3-fluorphenyl)methanol;
(*S*)-((*S*)-4,4-Dimethylazetidin-2-yl)(3-fluorphenyl)methanol;
(*R*)-((*S*)-6,6-Dimethylpiperidin-2-yl)(2-fluorphenyl)methanol;
(*S*)-((*S*)-6,6-Dimethylpiperidin-2-yl)(2-fluorphenyl)methanol;
(*S*)-((*S*)-6,6-Dimethylpiperidin-2-yl)(3-fluorphenyl)methanol;
(*R*)-((*S*)-6,6-Dimethylpiperidin-2-yl)(3-fluorphenyl)methanol;
(*R*)-(3-Chlorphenyl)((*R*)-4,4-dimethylazetidin-2-yl)methanol;
(*S*)-(3-Chlorphenyl)((*R*)-4,4-dimethylazetidin-2-yl)methanol;
(*R*)-(3-Chlorphenyl)((*S*)-4,4-dimethylazetidin-2-yl)methanol;
(*S*)-(3-Chlorphenyl)((*S*)-4,4-dimethylazetidin-2-yl)methanol;
(*R*)-(3-Amino-2-fluorphenyl)((*R*)-5,5-dimethylpyrrolidin-2-yl)methanol;
(*S*)-(3-Amino-2-fluorphenyl)((*R*)-5,5-dimethylpyrrolidin-2-yl)methanol; und
(*S*)-(4-Amino-3,5-difluorphenyl)((*R*)-5,5-dimethylpyrrolidin-2-yl)methanol,
und pharmazeutisch verträglichen Salzen davon.

8. Verbindung nach einem der Ansprüche 1 bis 7, jedoch ohne den Vorbehalt, zur Verwendung in der Medizin.

9. Pharmazeutische Zusammensetzung, umfassend eine Verbindung nach einem der Ansprüche 1 bis 7 sowie gegebenenfalls ein oder mehrere pharmazeutisch verträgliche Hilfsstoffe, Verdünnungsmittel und/oder Trägerstoffe,
mit der Maßgabe, dass die Verbindung der Formel I keine Verbindung ist, ausgewählt aus der Liste zusammengesetzt aus:
(*S*)-((*S*)-5,5-Dimethylpyrrolidin-2-yl)(4-(methylthio)phenyl)methanol;
(3,4-Dichlorphenyl)(5,5-dimethylpyrrolidin-2-yl)methanol;
(5,5-Dimethylpyrrolidin-2-yl)(p-tolyl)methanol;
(4-Chlorphenyl)(5,5-dimethylpyrrolidin-2-yl)methanol;
3-((5,5-Dimethylpyrrolidin-2-yl)(hydroxy)methyl)benzonitril;
(5,5-Dimethylpyrrolidin-2-yl)(m-tolyl)methanol;
(5,5-Dimethylpyrrolidin-2-yl)(3-(trifluormethyl)phenyl)methanol;
(5,5-Dimethylpyrrolidin-2-yl)(phenyl)methanol;
(2,4-Dichlorphenyl)(5,5-dimethylpyrrolidin-2-yl)methanol;
(2,6-Dichlorphenyl)(5,5-dimethylpyrrolidin-2-yl)methanol;
(3,4-Dichlorphenyl)(6,6-dimethylpiperidin-2-yl)methanol;
(3-Chlorphenyl)(6,6-dimethylpiperidin-2-yl)methanol;
(2,4-Dimethylphenyl)(5,5-dimethylpyrrolidin-2-yl)methanol;
(3-Chlorphenyl)(5,5-dimethylpyrrolidin-2-yl)methanol;
(4-Chlorphenyl)(6,6-dimethylpiperidin-2-yl)methanol;
(*R**)-(4-Chlorphenyl)((*S**)-5,5-dimethylpyrrolidin-2-yl)methanol;
(*R**)-(4-Chlorphenyl)((*R**)-5,5-dimethylpyrrolidin-2-yl)methanol;
(*R**)-(3,4-Dichlorphenyl)((*S**)-5,5-dimethylpyrrolidin-2-yl)methanol;
(*R**)-(3,4-Dichlorphenyl)((*R**)-5,5-dimethylpyrrolidin-2-yl)methanol;
(*R**)-(3-Chlorphenyl)((*S**)-5,5-dimethylpyrrolidin-2-yl)methanol;
(*R**)-(3-Chlorphenyl)((*R**)-5,5-dimethylpyrrolidin-2-yl)methanol;
(*R**)-(3-Chlorphenyl)((*R**)-6,6-dimethylpiperidin-2-yl)methanol;
(*R**)-((*S**)-5,5-Dimethylpyrrolidin-2-yl)(3-(trifluormethyl)phenyl)methanol;
(*R**)-((*R**)-5,5-Dimethylpyrrolidin-2-yl)(3-(trifluormethyl)phenyl)methanol;
(*R**)-((*S**)-5,5-Dimethylpyrrolidin-2-yl)(phenyl)methanol;
(*R**)-((*R**)-5,5-Dimethylpyrrolidin-2-yl)(phenyl)methanol;
(*R**)-((*S**)-5,5-Dimethylpyrrolidin-2-yl)(m-tolyl)methanol;
(*R**)-((*R**)-5,5-Dimethylpyrrolidin-2-yl)(m-tolyl)methanol;
(*R**)-(2,6-Dichlorphenyl)((*S**)-5,5-dimethylpyrrolidin-2-yl)methanol;
(*R*)-(2,6-Dichlorphenyl)((*R*)-5,5-dimethylpyrrolidin-2-yl)methanol;
(*R**)-(3,4-Dichlorphenyl)((*S**)-6,6-dimethylpiperidin-2-yl)methanol;
(*R**)-(3,4-Dichlorphenyl)((*R**)-6,6-dimethylpiperidin-2-yl)methanol;
(*R**)-(3-chlorphenyl)((*S**)-6,6-dimethylpiperidin-2-yl)methanol;
(*R**)-(2,4-Dichlorphenyl)((*S**)-5,5-dimethylpyrrolidin-2-yl)methanol;
(*R**)-(2,4-Dichlorphenyl)((*R**)-5,5-dimethylpyrrolidin-2-yl)methanol; und
3-((*R**)-((*R**)-5,5-dimethylpyrrolidin-2-yl)(hydroxy)methyl)benzonitril.

10. Verbindung nach einem der Ansprüche 1 bis 7, jedoch ohne den Vorbehalt, zur Verwendung bei der Behandlung von Hyperglykämie oder einer Störung, **gekennzeichnet durch** Hyperglykämie.

11. Verbindung zur Verwendung nach Anspruch 10, wobei die Hyperglykämie oder die Störung, **gekennzeichnet durch** Hyperglykämie, eine schwere Insulinresistenz darstellt oder **gekennzeichnet ist dadurch**, dass der Patient eine schwere Insulinresistenz aufweist.

12. Verbindung zur Verwendung nach einem der Ansprüche 10 und 11, wobei die Störung, **gekennzeichnet durch** Hyperglykämie, ausgewählt ist aus der Gruppe bestehend aus Typ-2-Diabetes, Rabson-Mendenhall-Syndrom, Donohue-Syndrom (Leprechaunismus), Insulinresistenz-Syndrom Typ A und Typ B, den HAIR-AN-Syndromen (Hyperandrogenismus, Insulinresistenz und Acanthosis nigricans), Pseudoakromegalie und Lipodystrophie.

13. Kombinationsprodukt, umfassend:
(a) eine Verbindung nach einem der Ansprüche 1 bis 7, jedoch ohne Vorbehalt; und
(b) ein oder mehrere weitere therapeutische Mittel, die bei der Behandlung von Hyperglykämie oder einer Störung, **gekennzeichnet durch** Hyperglykämie, von Nutzen sind,
wobei die Komponenten (a) und (b) jeweils in einer Mischung, gegebenenfalls zusammen mit einem oder mehreren pharmazeutisch verträglichen Hilfsstoffen, Verdünnungsmitteln oder Trägerstoffen, formuliert sind.

14. Teilesatz, umfassend:
(a) eine pharmazeutische Zusammensetzung, die eine Verbindung nach einem der Ansprüche 1 bis 7 umfasst, und
(b) ein oder mehrere weitere therapeutische Mittel, die bei der Behandlung von Hyperglykämie oder einer Störung, **gekennzeichnet durch** Hyperglykämie, von Nutzen sind, gegebenenfalls in Mischung mit einem oder mehreren pharmazeutisch verträglichen Hilfsstoffen, Verdünnungsmitteln oder Trägern,
wobei die Komponenten (a) und (b) jeweils in einer Form bereitgestellt sind, die für eine Verabreichung zusammen mit der anderen geeignet ist.

15. Verbindung nach einem einem der Ansprüche 1 bis 7, jedoch ohne die Maßgabe, zur Verwendung bei der Behandlung einer nicht-alkoholischen Fettlebererkrankung.

16. Prozess zur Herstellung einer Verbindung nach einem der Ansprüche 1 bis 7, umfassend den folgenden Schritt:
(i) Reagieren einer Verbindung der Formel II 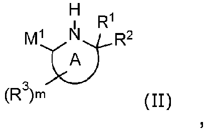
wobei Ring A, R¹, R², R³ und m wie in einem der Ansprüche 1 bis 7 definiert sind, und wobei M¹ ein geeignetes Metall oder Metallhalogenid darstellt, mit einer Verbindung der Formel III 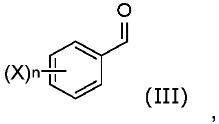
wobei n und X wie in einem der Ansprüche 1 bis 7 definiert sind;
(ii) Reagieren einer Verbindung der Formel IV 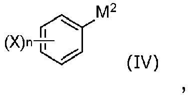
wobei n und X wie in einem der Ansprüche 1 bis 7 definiert sind, und wobei M² ein geeignetes Metall oder Metallhalogenid darstellt, mit einer Verbindung der Formel V 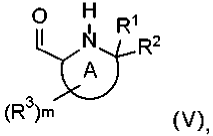
wobei Ring A, R¹, R², R³ und m wie in einem der Ansprüche 1 bis 7 definiert sind;
(iii) für Verbindungen, wobei mindestens ein X vorhanden ist und -OH darstellt, Entschützung einer Verbindung der Formel VI 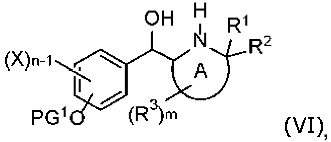 wobei Ring A, R¹, R², R³, n und m wie in einem der Ansprüche 1 bis 7 definiert sind, und PG¹ eine geeignete Schutzgruppe darstellt;
(iv) für Verbindungen, wobei mindestens ein X vorhanden ist NH₂ darstellt, Entschützung einer Verbindung der Formel VII 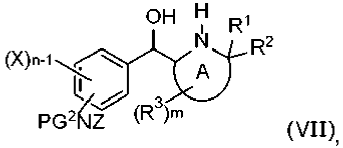 wobei Ring A, R¹, R², R³, n und m wie in einem der Ansprüche 1 bis 7 definiert sind und Z H oder PG³ darstellt, wobei PG² und PG³ jeweils eine geeignete Schutzgruppe darstellen;
(v) für Verbindungen, wobei mindestens ein X vorhanden ist und NH₂ darstellt, Reduktion einer Verbindung der Formel VIII 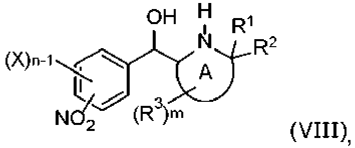 wobei Ring A,R¹, R², R³, n und m wie in einem der Ansprüche 1 bis 7 definiert sind;
(vi) Entschützung einer Verbindung der Formel IX 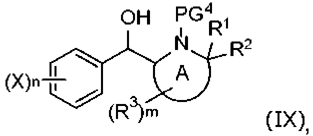 wobei Ring A, X, R¹, R², R³, n und m wie in einem der Ansprüche 1 bis 7 definiert sind, und PG⁴ eine geeignete Schutzgruppe darstellt; oder
(vii) Reduktion einer Verbindung der Formel XI 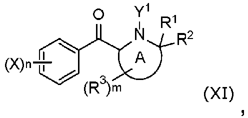 wobei Ring A, X, R¹, R², R³, n und m wie in einem der Ansprüche 1 bis 7 definiert sind und Y¹ H oder PG⁵ darstellt, wobei PG⁵ eine geeignete Schutzgruppe ist, in Gegenwart eines geeigneten Katalysators und in Gegenwart eines geeigneten Lösungsmittels.

## Revendications

1. Composé de formule I 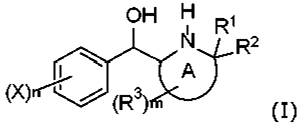 ou sel pharmaceutiquement acceptable de celui-ci, où :
le cycle A représente un hétérocycloalkyle de 4 à 8 chaînons ;
chaque R¹ et R² représente indépendamment un alkyle en C₁₋₆, un alcényle en C₂₋₆ ou un alcynyle en C₂₋₆, chacun éventuellement substitué par un ou plusieurs halogènes ;
ou alternativement, R¹ et R² peuvent être liés ensemble pour former un cycle à 3 chaînons, lequel est éventuellement substitué par un ou plusieurs groupes indépendamment choisis parmi halogène et alkyle en C₁₋₆, alcényle en C₂₋₆ ou alcynyle en C₂₋₆, chacun éventuellement substitué par un ou plusieurs halogènes ;
chaque R³ représente indépendamment un halogène ou un alkyle en C₁₋₆, un alcényle en C₂₋₆ ou un alcynyle en C₂₋₆, chacun éventuellement substitué par un ou plusieurs halogènes ;
chaque X représente indépendamment un halogène, R^{a}, -CN, -N₃, - N(R^{b})R^{c}, -NO₂, -ONO₂, -OR^{d}, -S(O)ₚR^{e} ou -S(O)_{q}N(R^{f})R^{g} ;
R^{a} représente un alkyle en C₁₋₆, un alcényle en C₂₋₆ ou un alcynyle en C₂₋₆, chacun éventuellement substitué par un ou plusieurs groupes indépendamment choisis parmi G¹ ;
chaque R^{b}, R^{c}, R^{d}, R^{e}, R^{f} et R^{g} représente indépendamment H ou un alkyle en C₁₋₆, un alcényle en C₂₋₆ ou un alcynyle en C₂₋₆, chacun éventuellement substitué par un ou plusieurs groupes indépendamment choisis parmi G² ;
ou alternativement, l'un quelconque parmi R^{b} et R^{c} et/ou R^{f} et R^{g} peuvent être liés ensemble pour former, conjointement avec l'atome d'azote auquel ils sont attachés, un cycle de 4 à 6 chaînons, lequel cycle contient éventuellement un hétéroatome supplémentaire et lequel cycle est éventuellement substitué par un ou plusieurs groupes indépendamment choisis parmi halogène, alkyle en C₁₋₃, alcényle en C₂₋₃ ou alcynyle en C₂₋₃, chacun éventuellement substitué par un ou plusieurs halogènes, et =O ;
G¹ et G² représentent un halogène, -CN, -N(R^{a1})R^{b1}, -OR^{c1}, -S(O)ₚR^{d1}, - S(O)_{q}N(R^{e1})R^{f1} ou =O ;
chaque R^{a1}, R^{b1}, R^{c1}, R^{d1}, R^{e1} et R^{f1} représente indépendamment H ou un alkyle en C₁₋₆, un alcényle en C₂₋₆ ou un alcynyle en C₂₋₆, chacun éventuellement substitué par un ou plusieurs halogènes ;
ou alternativement, l'un quelconque parmi R^{a1} et R^{b1} et/ou R^{e1} et R^{f1} peuvent être liés ensemble pour former, conjointement avec l'atome d'azote auquel ils sont attachés, un cycle de 4 à 6 chaînons, lequel cycle contient éventuellement un hétéroatome supplémentaire et lequel cycle est éventuellement substitué par un ou plusieurs groupes indépendamment choisis parmi halogène, alkyle en C₁₋₃, alcényle en C₂₋₃ ou alcynyle en C₂₋₃, chacun éventuellement substitué par un ou plusieurs halogènes, et =O ;
n représente 0 à 5 ;
chaque p représente indépendamment 0, 1 ou 2 ;
chaque q représente indépendamment 1 ou 2 ;
m représente 0 à 11, selon le cas,
dans lequel les groupes alkyle, alcényle et alcynyle peuvent être à chaîne droite ou, en présence d'un nombre suffisant d'atomes de carbone, être à chaîne ramifiée et/ou cycliques ou partiellement cycliques,
à condition que le composé de formule I ne soit pas un composé choisi dans la liste constituée de :
(*S*)-((*S*)-5,5-diméthylpyrrolidin-2-yl)(4-(méthylthio)phényl)méthanol ;
(3,4-dichlorophényl)(5,5-diméthylpyrrolidin-2-yl)méthanol ;
(5,5-diméthylpyrrolidin-2-yl)(*p*-tolyl)méthanol ;
(4-chlorophényl)(5,5-diméthylpyrrolidin-2-yl)méthanol ;
3-((5,5-diméthylpyrrolidin-2-yl)(hydroxy)méthyl)benzonitrile ;
(5,5-diméthylpyrrolidin-2-yl)(*m*-tolyl)méthanol ;
(5,5-diméthylpyrrolidin-2-yl)(3-(trifluorométhyl)phényl)méthanol ;
(5,5-diméthylpyrrolidin-2-yl)(phényl)méthanol ;
(2,4-dichlorophényl)(5,5-diméthylpyrrolidin-2-yl)méthanol ;
(2,6-dichlorophényl)(5,5-diméthylpyrrolidin-2-yl)méthanol ;
(3,4-dichlorophényl)(6,6-diméthylpipéridin-2-yl)méthanol ;
(3-chlorophényl)(6,6-diméthylpipéridin-2-yl)méthanol ;
(2,4-diméthylphényl)(5,5-diméthylpyrrolidin-2-yl)méthanol ;
(3-chlorophényl)(5,5-diméthylpyrrolidin-2-yl)méthanol ;
(4-chlorophényl)(6,6-diméthylpipéridin-2-yl)méthanol ;
(*R**)-(4-chlorophényl)((*S**)-5,5-diméthylpyrrolidin-2-yl)méthanol ;
(*R**)-(4-chlorophényl)((*R**)-5,5-diméthylpyrrolidin-2-yl)méthanol ;
(*R**)-(3,4-dichlorophényl)((*S**)-5,5-diméthylpyrrolidin-2-yl)méthanol ;
(*R**)-(3,4-dichlorophényl)((*R**)-5,5-diméthylpyrrolidin-2-yl)méthanol ;
(*R**)-(3-chlorophényl)((*S**)-5,5-diméthylpyrrolidin-2-yl)méthanol ;
(*R**)-(3-chlorophényl)((*R**)-5,5-diméthylpyrrolidin-2-yl)méthanol ;
(*R**)-(3-chlorophényl)((*R**)-6,6-diméthylpipéridin-2-yl)méthanol ;
(*R**)-((*S**)-5,5-diméthylpyrrolidin-2-yl)(3-(trifluorométhyl)phényl)méthanol ;
(*R**)-((*R**)-5,5-diméthylpyrrolidin-2-yl)(3-(trifluorométhyl)phényl)méthanol ;
(*R**)-((*S**)-5,5-diméthylpyrrolidin-2-yl)(phényl)méthanol ;
(*R**)-((*R**)-5,5-diméthylpyrrolidin-2-yl)(phényl)méthanol ;
(*R**)-((*S**)-5,5-diméthylpyrrolidin-2-yl)(*m*-tolyl)méthanol ;
(*R**)-((*R**)-5,5-diméthylpyrrolidin-2-yl)(*m*-tolyl)méthanol ;
(*R**)-(2,6-dichlorophényl)((*S**)-5,5-diméthylpyrrolidin-2-yl)méthanol ;
(*R*)-(2,6-dichlorophényl)((*R*)-5,5-diméthylpyrrolidin-2-yl)méthanol ;
(*R**)-(3,4-dichlorophényl)((*S**)-6,6-diméthylpipéridin-2-yl)méthanol ;
(*R**)-(3,4-dichlorophényl)((*R**)-6,6-diméthylpipéridin-2-yl)méthanol ;
(*R**)-(3-chlorophényl)((*S**)-6,6-diméthylpipéridin-2-yl)méthanol ;
(*R**)-(2,4-dichlorophényl)((*S**)-5,5-diméthylpyrrolidin-2-yl)méthanol ;
(*R**)-(2,4-dichlorophényl)((*R**)-5,5-diméthylpyrrolidin-2-yl)méthanol ; et 3-((*R**)-((*R**)-5,5-diméthylpyrrolidin-2-yl)(hydroxy)méthyl)benzonitrile.

2. Composé selon la revendication 1, dans lequel le composé est un composé de formule (IA) 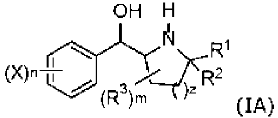 ou un sel pharmaceutiquement acceptable de celui-ci, où :
R¹, R², R³, X et n sont tels que définis dans la revendication 1 ;
z représente 1 ou 2 ; et
lorsque z représente 1, alors m représente 0 à 5 et, lorsque z représente 2, alors m représente 0 à 7.

3. Composé tel que défini selon l'une quelconque des revendications 1 et 2, dans lequel soit :
chaque R¹ et R² représente indépendamment un alkyle en C₁₋₃, éventuellement substitué par un ou plusieurs halogènes ; soit
R¹ et R² sont liés ensemble pour former un cycle à 3 chaînons, lequel est éventuellement substitué par un ou plusieurs groupes indépendamment choisis parmi halogène et alkyle en C₁₋₆, éventuellement substitués par un ou plusieurs halogènes.

4. Composé tel que défini selon l'une quelconque des revendications précédentes, dans lequel chaque X représente indépendamment un halogène, OH, CN, CF₃ ou NH₂.

5. Composé tel que défini selon l'une quelconque des revendications précédentes, dans lequel n représente 1, 2 ou 3.

6. Composé tel que défini selon l'une quelconque des revendications précédentes, dans lequel m représente 0.

7. Composé tel que défini selon l'une quelconque des revendications précédentes, dans lequel le composé est choisi dans le groupe constitué de :
(A)
(*R*)-((*R*)-6,6-diméthylpipéridin-2-yl)(3-fluorophényl)méthanol ;
(*S*)-((*R*)-6,6-diméthylpipéridin-2-yl)(3-fluorophényl)méthanol ;
3-((*R*)-((*R*)-6,6-diméthylpipéridin-2-yl)(hydroxy)méthyl)phénol ;
3-((*S*)-((*R*)-6,6-diméthylpipéridin-2-yl)(hydroxy)méthyl)phénol ;
(*R*)-(2-chlorophényl)((*R*)-6,6-diméthylpipéridin-2-yl)méthanol ;
(*S*)-(2-chlorophényl)((*R*)-6,6-diméthylpipéridin-2-yl)méthanol ;
(*R*)-(2-chlorophényl)((*R*)-6,6-diméthylpipéridin-2-yl)méthanol ;
(*S*)-(2-chlorophényl)((*R*)-6,6-diméthylpipéridin-2-yl)méthanol ;
(*R*)-((*R*)-6,6-diméthylpipéridin-2-yl)(2-fluorophényl)méthanol ;
(*S*)-((*R*)-6,6-diméthylpipéridin-2-yl)(2-fluorophényl)méthanol ;
(*R*)-((*R*)-5,5-diméthylpyrrolidin-2-yl)(3-fluorophényl)méthanol ;
(*S*)-((*R*)-5,5-diméthylpyrrolidin-2-yl)(3-fluorophényl)méthanol ;
(*R*)-((*S*)-5,5-diméthylpyrrolidin-2-yl)(3-fluorophényl)méthanol ;
(*S*)-((*S*)-5,5-diméthylpyrrolidin-2-yl)(3-fluorophényl)méthanol ;
(*R*)-((*R*)-5,5-diméthylpyrrolidin-2-yl)(hydroxy)méthyl)phénol ;
(*S*)-((*R*)-5,5-diméthylpyrrolidin-2-yl)(hydroxy)méthyl)phénol ;
(*R*)-((*S*)-5,5-diméthylpyrrolidin-2-yl)(hydroxy)méthyl)phénol ;
(*S*)-((*S*)-5,5-diméthylpyrrolidin-2-yl)(hydroxy)méthyl)phénol ;
(*R*)-((*R*)-5,5-diméthylpyrrolidin-2-yl)(2-fluorophényl)méthanol ;
(*S*)-((*R*)-5,5-diméthylpyrrolidin-2-yl)(2-fluorophényl)méthanol ;
(*R*)-((*R*)-5,5-diméthylpyrrolidin-2-yl)(4-fluorophényl)méthanol ;
(*S*)-((*R*)-5,5-diméthylpyrrolidin-2-yl)(4-fluorophényl)méthanol ;
(*R*)-(2-chlorophényl)((*R*)-5,5-diméthylpyrrolidin-2-yl)méthanol ;
(*S*)-(2-chlorophényl)((*R*)-5,5-diméthylpyrrolidin-2-yl)méthanol ;
(*R*)-(3-chlorophényl)((*R*)-5,5-diméthylpyrrolidin-2-yl)méthanol ;
(*S*)-(2-chlorophényl)((*R*)-5,5-diméthylpyrrolidin-2-yl)méthanol ;
(*R*)-((*R*)-5,5-dipropylpyrrolidin-2-yl)(3-fluorophényl)méthanol ;
(*S*)-((*R*)-5,5-dipropylpyrrolidin-2-yl)(3-fluorophényl)méthanol ;
(*R*)-(3-fluorophényl)((*R*)-4-azaspiro[2.4]heptan-5-yl)méthanol ; et
(*S*)-(3-fluorophényl)((*R*)-4-azaspiro[2.4]heptan-5-yl)méthanol,
et sels pharmaceutiquement acceptables de ceux-ci ; et
(B)
(*R*)-((*R*)-4,4-diméthylazétidin-2-yl)(3-fluorophényl)méthanol ;
(*S*)-((*S*)-4,4-diméthylazétidin-2-yl)(2-fluorophényl)méthanol ;
(*R*)-((*R*)-4,4-diméthylazétidin-2-yl)(2-fluorophényl)méthanol ;
(*S*)-((*R*)-4,4-diméthylazétidin-2-yl)(2-fluorophényl)méthanol ;
(*R*)-((*S*)-4,4-diméthylazétidin-2-yl)(2-fluorophényl)méthanol ;
(*S*)-((*R*)-4,4-diméthylazétidin-2-yl)(2-fluorophényl)méthanol ;
(*R*)-((*S*)-4,4-diméthylazétidin-2-yl)(3-fluorophényl)méthanol ;
(*S*)-((*S*)-4,4-diméthylazétidin-2-yl)(3-fluorophényl)méthanol ;
(*R*)-((*S*)-6,6-diméthylpipéridin-2-yl)(2-fluorophényl)méthanol ;
(*S*)-((*S*)-6,6-diméthylpipéridin-2-yl)(2-fluorophényl)méthanol ;
(*S*)-((*S*)-6,6-diméthylpipéridin-2-yl)(3-fluorophényl)méthanol ;
(*R*)-((*S*)-6,6-diméthylpipéridin-2-yl)(3-fluorophényl)méthanol ;
(*R*)-(3-chlorophényl)((*R*)-4,4-diméthylazétidin-2-yl)méthanol ;
(*S*)-(3-chlorophényl)((*R*)-4,4-diméthylazétidin-2-yl)méthanol ;
(*R*)-(3-chlorophényl)((*S*)-4,4-diméthylazétidin-2-yl)méthanol ;
(*S*)-(3-chlorophényl)((*S*)-4,4-diméthylazétidin-2-yl)méthanol ;
(*R*)-(3-amino-2-fluorophényl)((*R*)-5,5-diméthylpyrrolidin-2-yl)méthanol ;
(*S*)-(3-amino-2-fluorophényl)((*R*)-5,5-diméthylpyrrolidin-2-yl)méthanol ; et
(*S*)-(4-amino-3,5-difluorophényl)((*R*)-5,5-diméthylpyrrolidin-2-yl)méthanol,
et sels pharmaceutiquement acceptables de ceux-ci.

8. Composé tel que défini selon l'une quelconque des revendications 1 à 7, mais sans la condition, destiné à être utilisé en médecine.

9. Composition pharmaceutique comprenant un composé tel que défini selon l'une quelconque des revendications 1 à 7, et éventuellement un ou plusieurs adjuvants, diluants et/ou supports pharmaceutiquement acceptables,
à condition que le composé de formule I ne soit pas un composé choisi dans la liste constituée de :
(*S*)-((*S*)-5,5-diméthylpyrrolidin-2-yl)(4-(méthylthio)phényl)méthanol ;
(3,4-dichlorophényl)(5,5-diméthylpyrrolidin-2-yl)méthanol ;
(5,5-diméthylpyrrolidin-2-yl)(*p*-tolyl)méthanol ;
(4-chlorophényl)(5,5-diméthylpyrrolidin-2-yl)méthanol ;
3-((5,5-diméthylpyrrolidin-2-yl)(hydroxy)méthyl)benzonitrile ;
(5,5-diméthylpyrrolidin-2-yl)(*m*-tolyl)méthanol ;
(5,5-diméthylpyrrolidin-2-yl)(3-(trifluorométhyl)phényl)méthanol ;
(5,5-diméthylpyrrolidin-2-yl)(phényl)méthanol ;
(2,4-dichlorophényl)(5,5-diméthylpyrrolidin-2-yl)méthanol ;
(2,6-dichlorophényl)(5,5-diméthylpyrrolidin-2-yl)méthanol ;
(3,4-dichlorophényl)(6,6-diméthylpipéridin-2-yl)méthanol ;
(3-chlorophényl)(6,6-diméthylpipéridin-2-yl)méthanol ;
(2,4-diméthylphényl)(5,5-diméthylpyrrolidin-2-yl)méthanol ;
(3-chlorophényl)(5,5-diméthylpyrrolidin-2-yl)méthanol ;
(4-chlorophényl)(6,6-diméthylpipéridin-2-yl)méthanol ;
(*R**)-(4-chlorophényl)((*S**)-5,5-diméthylpyrrolidin-2-yl)méthanol ;
(*R**)-(4-chlorophényl)((*R**)-5,5-diméthylpyrrolidin-2-yl)méthanol ;
(*R**)-(3,4-dichlorophényl)((*S**)-5,5-diméthylpyrrolidin-2-yl)méthanol ;
(*R**)-(3,4-dichlorophényl)((*R**)-5,5-diméthylpyrrolidin-2-yl)méthanol ;
(*R**)-(3-chlorophényl)((*S**)-5,5-diméthylpyrrolidin-2-yl)méthanol ;
(*R**)-(3-chlorophényl)((*R**)-5,5-diméthylpyrrolidin-2-yl)méthanol ;
(*R**)-(3-chlorophényl)((*R**)-6,6-diméthylpipéridin-2-yl)méthanol ;
(*R**)-((*S**)-5,5-diméthylpyrrolidin-2-yl)(3-(trifluorométhyl)phényl)méthanol ;
(*R**)-((*R**)-5,5-diméthylpyrrolidin-2-yl)(3-(trifluorométhyl)phényl)méthanol ;
(*R**)-((*S**)-5,5-diméthylpyrrolidin-2-yl)(phényl)méthanol ;
(*R**)-((*R**)-5,5-diméthylpyrrolidin-2-yl)(phényl)méthanol ;
(*R**)-((*S**)-5,5-diméthylpyrrolidin-2-yl)(*m*-tolyl)méthanol ;
(*R**)-((*R**)-5,5-diméthylpyrrolidin-2-yl)(*m*-tolyl)méthanol ;
(*R**)-(2,6-dichlorophényl)((*S**)-5,5-diméthylpyrrolidin-2-yl)méthanol ;
(*R*)-(2,6-dichlorophényl)((*R*)-5,5-diméthylpyrrolidin-2-yl)méthanol ;
(*R**)-(3,4-dichlorophényl)((*S**)-6,6-diméthylpipéridin-2-yl)méthanol ;
(*R**)-(3,4-dichlorophényl)((*R**)-6,6-diméthylpipéridin-2-yl)méthanol ;
(*R**)-(3-chlorophényl)((*S**)-6,6-diméthylpipéridin-2-yl)méthanol ;
(*R**)-(2,4-dichlorophényl)((*S**)-5,5-diméthylpyrrolidin-2-yl)méthanol ;
(*R**)-(2,4-dichlorophényl)((*R**)-5,5-diméthylpyrrolidin-2-yl)méthanol ; et
3-((*R**)-((*R**)-5,5-diméthylpyrrolidin-2-yl)(hydroxy)méthyl)benzonitrile.

10. Composé tel que défini selon l'une quelconque des revendications 1 à 7, mais sans la condition, destiné à être utilisé pour le traitement de l'hyperglycémie ou d'un trouble **caractérisé par** une hyperglycémie.

11. Composé utilisable selon la revendication 10, dans lequel l'hyperglycémie ou le trouble **caractérisé par** une hyperglycémie est, ou est **caractérisé par le fait que** le patient présente, une résistance à l'insuline sévère.

12. Composé utilisable selon l'une quelconque des revendications 10 et 11, dans lequel le trouble **caractérisé par** une hyperglycémie est choisi dans le groupe constitué du diabète de type 2, syndrome de Rabson-Mendenhall, syndrome de Donohue (lepréchaunisme), syndromes de résistance à l'insuline de type A et de type B, syndromes HAIR-AN (hyperandrogénie, résistance à l'insuline et *acanthosis nigricans*), pseudo-acromégalie et lipodystrophie.

13. Produit de combinaison comprenant :
(a) un composé tel que défini selon l'une quelconque des revendications 1 à 7, mais sans la condition ; et
(b) un ou plusieurs autres agents thérapeutiques utiles dans le traitement de l'hyperglycémie ou d'un trouble **caractérisé par** une hyperglycémie,
dans lequel chacun des composants (a) et (b) est formulé en mélange, éventuellement avec un ou plusieurs adjuvants, diluants ou supports pharmaceutiquement acceptables.

14. Kit de composants comprenant :
(a) une composition pharmaceutique comprenant un composé tel que défini selon l'une quelconque des revendications 1 à 7, et
(b) un ou plusieurs autres agents thérapeutiques utiles dans le traitement de l'hyperglycémie ou d'un trouble **caractérisé par** une hyperglycémie, éventuellement en mélange avec un ou plusieurs adjuvants, diluants ou supports pharmaceutiquement acceptables,
lesquels composants (a) et (b) sont chacun fournis sous une forme appropriée pour une administration conjointement avec l'autre.

15. Composé tel que défini selon l'une quelconque des revendications 1 à 7, mais sans la condition, destiné à être utilisé pour le traitement d'une stéatose hépatique non alcoolique.

16. Procédé pour la préparation d'un composé tel que défini selon l'une quelconque des revendications 1 à 7, comprenant l'étape consistant à :
(i) mettre en réaction un composé de formule Il 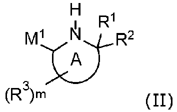
où le cycle A, R¹, R², R³ et m sont tels que définis selon l'une quelconque des revendications 1 à 7, et où M¹ représente un métal ou halogénure de métal approprié, avec un composé de formule III 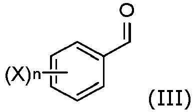
où n et X sont tels que définis selon l'une quelconque des revendications 1 à 7 ;
(ii) mettre en réaction un composé de formule IV 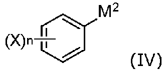
où n et X sont tels que définis selon l'une quelconque des revendications 1 à 7, et où M² représente un métal ou halogénure de métal approprié, avec un composé de formule V 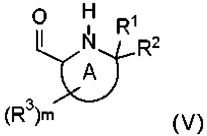
où le cycle A, R¹, R², R³ et m sont tels que définis selon l'une quelconque des revendications 1 à 7 ;
(iii) pour les composés dans lesquels au moins un X est présent et représente -OH, lever la protection d'un composé de formule VI 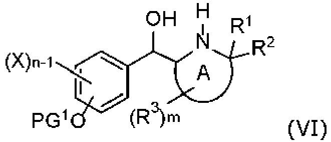 où le cycle A, R¹, R², R³, n et m sont tels que définis selon l'une quelconque des revendications 1 à 7, et PG¹ représente un groupe protecteur approprié ;
(iv) pour les composés dans lesquels au moins un X est présent et représente NH₂, lever la protection d'un composé de formule VII 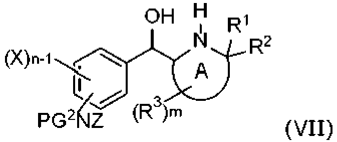 où le cycle A, R¹, R², R³, n et m sont tels que définis selon l'une quelconque des revendications 1 à 7, et Z représente H ou PG³, où PG² et PG³ représentent chacun un groupe protecteur approprié ;
(v) pour les composés dans lesquels au moins un X est présent et représente NH₂, réduire un composé de formule VIII 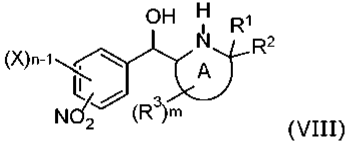 où le cycle A, R¹, R², R³, n et m sont tels que définis selon l'une quelconque des revendications 1 à 7 ;
(vi) lever la protection d'un composé de formule IX 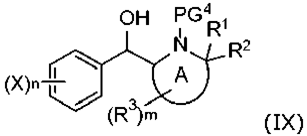 où le cycle A, X, R¹, R², R³, n et m sont tels que définis selon l'une quelconque des revendications 1 à 7, et PG⁴ représente un groupe protecteur approprié ; ou
(vii) réduire un composé de formule XI 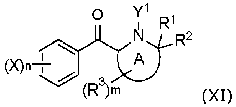 où le cycle A, X, R¹, R², R³, n et m sont tels que définis selon l'une quelconque des revendications 1 à 7 et Y¹ représente H ou PG⁵, où PG⁵ est un groupe protecteur approprié, en présence d'un catalyseur approprié et en présence d'un solvant approprié.
